(19) Europäisches Patentamt
European Patent Office
Office européen des brevets

(11) EP 4 190 776 A1

(12) EUROPEAN PATENT APPLICATION
published in accordance with Art. 153(4) EPC

(43) Date of publication:
**07.06.2023 Bulletin 2023/23**

(21) Application number: **21849912.7**

(22) Date of filing: **27.07.2021**

(51) International Patent Classification (IPC):
***C07D 403/14*** *(2006.01)* ***A61K 31/517*** *(2006.01)*
***A61P 5/00*** *(2006.01)* ***A61P 3/00*** *(2006.01)*
***A61P 13/00*** *(2006.01)* ***A61P 1/16*** *(2006.01)*
***A61P 9/00*** *(2006.01)* ***A61P 25/00*** *(2006.01)*
***A61P 25/04*** *(2006.01)* ***A61P 35/00*** *(2006.01)*
***A61P 29/00*** *(2006.01)*

(52) Cooperative Patent Classification (CPC):
**A61K 31/517; A61P 1/00; A61P 1/16; A61P 3/00; A61P 5/00; A61P 9/00; A61P 13/00; A61P 25/00; A61P 25/04; A61P 29/00; A61P 35/00; A61P 37/02; C07D 403/14**

(86) International application number:
**PCT/CN2021/108515**

(87) International publication number:
**WO 2022/022476 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**28.07.2020 CN 202010735232**
**28.07.2020 CN 202010734940**
**28.07.2020 CN 202010734975**
**28.07.2020 CN 202010735496**
**28.07.2020 CN 202010735081**

(71) Applicants:
- **Shandong Xuanzhu Pharma Co., Ltd.**
**National High-Tech Development Zone Jinan Shandong 250101 (CN)**
- **Xuanzhu Biopharmaceutical Co., Ltd.**
**Shijiazhuang, Hebei 050035 (CN)**

(72) Inventors:
- **WENG, Zhentao**
**Jinan, Shandong 250101 (CN)**
- **WANG, Feng**
**Jinan, Shandong 250101 (CN)**

(74) Representative: **ABG Intellectual Property Law, S.L.**
**Avenida de Burgos, 16D**
**Edificio Euromor**
**28036 Madrid (ES)**

(54) **SALT AND CRYSTAL FORM OF KETOHEXOKINASE INHIBITOR AND USE THEREOF**

(57) Disclosed are a crystal form and a pharmaceutically acceptable salt of a ketohexokinase inhibitor, a preparation method therefor, and a pharmaceutical composition and use thereof, which specifically relate to a crystal form of a compound of formula (1), a pharmaceutically acceptable salt of the compound of formula (1), and a crystal form of the pharmaceutically acceptable saltserving as the KHK inhibitor, a preparation method therefor, a pharmaceutical composition containing the crystal form, the pharmaceutically acceptable salt, and the crystal form of the pharmaceutically acceptable salt, and use of the crystal form and the pharmaceutically acceptable salt of the compound of formula (1), and the crystal form of the pharmaceutically acceptable salt in the preparation of a medicament for treatment and/or prevention of a KHK-mediated disease or related disease.



Processed by Luminess, 75001 PARIS (FR)

(Cont. next page)

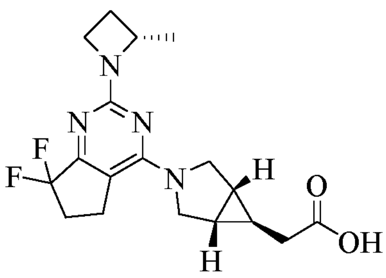

（1）

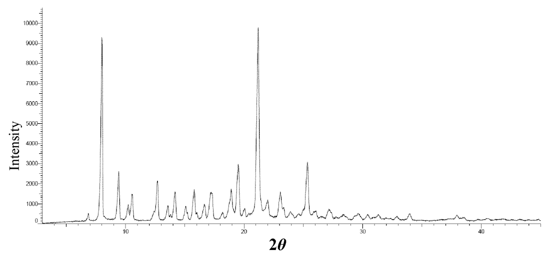


FIG. 1

## Description

### TECHNICAL FIELD

The present invention relates to a crystal form and a pharmaceutically acceptable salt of a ketohexokinase inhibitor, and preparation methods thereof, a pharmaceutical composition and use thereof in the preparation of a medicament for the treatment and/or prevention of a KHK-mediated disease or related disease.

### BACKGROUND

NAFLD/NASH is a liver manifestation of metabolic syndrome. Dietary and lifestyle changes leading to the prevalence of obesity and metabolic syndrome in western and many Asian countries, thus leading to a significant increase in the incidence of NAFLD (nonalcoholic fatty liver disease), which has become one of the public health problems of considerable concern. Nonalcoholic steatohepatitis (NASH) is a result of further development of simple fatty liver, which is pathologically characterized by lipidosis, inflammatory cell infiltration, hepatic tissue necrosis and fibrotic lesions, with further lesions into more severe cirrhosis and hepatocellular carcinoma (HCC). NAFLD not only affects the hepatobiliary system of patients, but is also closely related to insulin resistance, dyslipidemia, atherosclerosis, fat embolism, hematological system diseases, etc. (Friedman SL et al, Nat Med, 2018, 24: 908-22). Since all components of metabolic syndrome are associated with liver fat content, patients with metabolic syndrome should be assessed for risk of NAFLD. Patients with Type 2 Diabetes Mellitus are also more prevalent with insulin resistance, obesity, dyslipidemia, and liver enzymology abnormalities, and the prevalence of NAFLD is also higher in people at risk of Type 2 Diabetes Mellitus.

The increasing addition of sugar (typically sucrose and high fructose corn syrup) to beverages and processed foods has led to an increase in the amount of fructose in modern diets. High fructose intake has been shown to cause a number of undesirable metabolic effects, which have a specific role in the development of obesity and metabolic syndrome, such as weight gain, hyperlipidemia, hypertension and insulin resistance. ((a) Elliott SS, Keim NL, Stem JS, Teff K, Havel PJ. Fructose, weight gain, and the insulin resistance syn-drome. (b) Bray GA. Soft drink consumption and obesity: it is all about fructose. Current opinion in lipidology. 2010; 21(1): 51-7. (c) The American journal of clinical nutrition. 2002; 76(5): 911-22. and cardiovascular disease. The American journal of clinical nutrition. 2007; 86(4): 899-906.). Fructose promotes the occurrence and development of NAFLD, and aggravates the development and deterioration of NAFLD (Shi Hongbin et al, Study on association between fructose and nonalcoholic fatty liver disease, Medical Recapitulate 2017 23 (9), 1685-1689). Meanwhile, high fructose intake increases the risk of NASH and advanced hepatic fibrosis (The 2016 EASL-EASD-EASO Clinical Practice Guidelines for the Management of Non Alcoholic Fatty Liver Disease). Unlike glucose, fructose metabolism is not regulated by negative feedback. Fructose is preferentially metabolized relative to other carbohydrates, and the metabolism of fructose produces various reactive and signaling metabolites that contribute to the progression of metabolic diseases. In the absence of KHK, fructose consumption-induced weight gain and insulin resistance are blocked (George Marek, 1 Varinderpal Pannu, 1 Prashanth Shanmugham, 1 Brianna Pancione, 1 Dominic Mascia, 1 Sean Crosson, 1 Takuji Ishimoto, 2 and Yuri Y. Sautin1; Adiponectin Resistance and Proinflammatory Changes in the Visceral Adipose Tissue Induced by Fructose Consumption via Ketohexokinase-Dependent Pathway; Diabetes 2015; 64:508-518). Reducing the intake of sugar/HFCS (high fructose corn syrup) and/or blocking the production of uric acid may help to reduce downstream complications of NAFLD, cirrhosis and chronic liver diseases thereof (Thomas Jensen et al, Fructose and Sugar: A Major Mediator of Nonalcoholic Fatty Liver Disease, J Hepatol. 2018 May; 68(5): 1063-1075). Meanwhile, human genetic mutagenesis results in basic fructosyl diabetes, which is a rare harmless abnormality characterized by the presence of fructose in urine after ingestion of fructose-containing food. The high prevalence rate of T2D, obesity, NAFLD/NASH and related metabolic diseases such as cardiovascular disease and stroke has led to an increased need for both prophylactic health care and therapeutic intervention.

Ketohexokinase (also called fructokinase) is a basic enzyme of fructose metabolism. KHK enzyme in liver phosphorylates fructose C1 site with the assistance of ATP (adenosine triphosphate) to produce fructose-1-phosphate (F1P), which enters normal metabolic pathway; and meanwhile, uric acid is generated in the downstream of the ATP. Human ketohexokinase (hKHK) expressed by two alternative mRNA spliceosomes encodes two distinct regioisomerases KHK-A and KHK-C. KHK-C has lower Km value, higher Kcat, and catalytic efficiency higher than 405 times, indicating that KHK-C has significantly higher affinity and ability for fructose phosphorylation than that of KHK-A. Although KHK-A is widely expressed and KHK-C is distributed in liver, kidney and intestine, KHK-C is a main metabolic site of fructose in vivo.

In a human body, glucose is converted to fructose through a polyol pathway via an intermediate sorbitol, producing endogenous fructose (Mingule A, et al, Endogenous fructose production and metabolism in the liver contributes to the development of metabolic syndrome, Nat Commun. 2013; 4: 2434.), and the activity of this pathway increases with hyperglycemia. Studies have shown that mice with KHK knock-out are protected from glucose-induced weight gain, insulin resistance and steatosis, suggesting that endogenously produced fructose may contribute to insulin resistance

and steatosis in hyperglycemic conditions (Lanaspa, M.A et al, Nature Comm.4, 2434, 2013). Fructose is the only common carbohydrate that produces uric acid during its metabolism, and meanwhile, fructose also stimulates the synthesis of uric acid from amino acid precursors. Therefore, inhibition of KHK is presumed to be beneficial in a number of diseases in which alterations in either or both of endogenous or ingested fructose are involved.

Hepatic fructokinase deficiency is a basis of fructosuria. In contrast to this benign condition, the absence of aldolase B (next enzyme in the metabolic pathway of fructose through KHK) leads to an accumulation of F1P in fructose intake and possibly leads to a lethal depletion of cellular ATP (hereditary fructose intolerance). An enzyme responsible for the decomposition of F1P immediately downstream of the KHK step in the fructose metabolic pathway is Aldolase (ALDOB), the absence of which leads to hereditary fructose intolerance (HFI). About 1 out of 20,000 people suffer from a rare disorder. This mutation leads to F1P accumulation and increased uric acid formation following ATP depletion, which are combined to cause hypoglycemia, hyperuricemia, and lactic acidosis, as well as other metabolic disorders. HFI blocks the downstream metabolism of fructose, resulting in acute symptoms such as emesis, severe hypoglycemia, diarrhea, and abdominal pain, which in turn leads to long-term growth defects, liver and kidney damage, and potential death (Ali M et al, J. Med. Genet. May1998: 35 (5); 353-365). Patients often experience annual survival before diagnosis and the only treatment modality is to avoid fructose in diet. Glucose in vivo is converted to endogenous fructose through the polyol pathway and metabolized in vivo, which also presents a challenge to this treatment modality. The presence of fructose in most food products presents a challenge to dietary. In addition to physical symptoms, many patients face emotional and social isolation due to their unusual diet, and need to strictly abide by dietary restrictions simultaneously (HFI-INFO Discussion Board, http: //hfiinfo.proboards.com. Accessed on December 14, 2015). In addition, infusions containing fructose, sorbitol or invert sugar may also be life threatening to the patients. There is a high unmet clinical need for this disease.

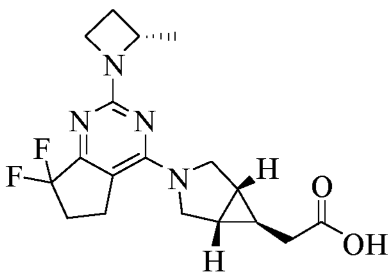
formula (I)

The study of crystal form plays an important role in medicament research and development. Different crystal forms of the same medicament have significant differences in solubility, stability, bioavailability and other aspects. In order to better control the quality of medicament and meet the requirements of preparation, production and transportation, crystal forms of the compound of formula (1) are studied in order to find out crystal forms with good properties.

**SUMMARY**

The present invention relates to a crystal form A, a crystal form B, a crystal form C, a crystal form D and a pharmaceutically acceptable salt of a ketohexokinase inhibitor 2-((1*R*,5*S*,6*R*)-3-(7,7-difluoro-2-((*S*)-2-methylazetidin-1-yl)-6,7-dihydro-5*H*-cyclopenta [*d*]p-yrimidin-4-yl)-3-azabicyclo[3.1.0]hexan-6-yl)acetic acid, and a crystal form of the pharmaceutically acceptable salt. The present invention also relates to preparation methods of the crystal form A, the crystal form B, the crystal form C, the crystal form D, the pharmaceutically acceptable salt and the crystal form of the pharmaceutically acceptable salt; pharmaceutical compositions containing the crystal form A, the crystal form B, the crystal form C, the crystal form D, the pharmaceutically acceptable salt and the crystal form of the pharmaceutically acceptable salt; and use of the crystal form A, the crystal form B, the crystal form C, the crystal form D and the pharmaceutically acceptable salt of these compounds and the crystal form of the pharmaceutically acceptable salt in preparation of a medicament for treatment and/or prevention of a KHK-mediated disease or related disease.

The present invention provides a crystal form A of a compound of formula (1) having characteristic peaks at 2$\theta$ positions of 8.0±0.2°, 9.4±0.2°, 10.5±0.2°, 12.7±0.2°, 19.5±0.2°, and 21.2±0.2° in an X-ray powder diffraction pattern by using Cu-K$\alpha$ radiation;

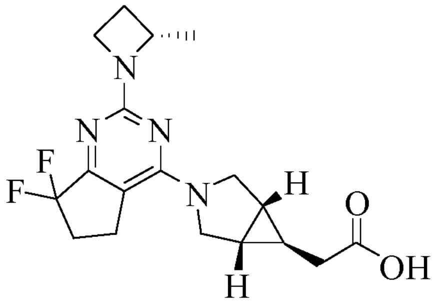 formula (1).

In some embodiments, the crystal form A of the compound of formula (1), on the basis of containing the characteristic peaks above, also has characteristic peaks at 2$\theta$ positions of 10.2±0.2°, 13.6±0.2°, 14.2±0.2°, 15.8±0.2°, 17.2±0.2°, and 25.3±0.2° in an X-ray powder diffraction pattern by using Cu-K$\alpha$ radiation.

In some embodiments, the crystal form A of the compound of formula (1), on the basis of containing the characteristic peaks above, also has characteristic peaks at 2$\theta$ positions of 6.9±0.2°, 15.1±0.2°, 16.6±0.2°, 18.9±0.2°, 22.0±0.2°, and 23.1±0.2° in an X-ray powder diffraction pattern by using Cu-K$\alpha$ radiation.

In some embodiments, the crystal form A of the compound of formula (1) has an X-ray powder diffraction pattern at 2$\theta$ (°) by using Cu-K$\alpha$ radiation substantially as shown in FIG. 1.

In some embodiments, the crystal form A of the compound of formula (1) has an endothermic peak in the range of about 167°C to 173°C in a differential scanning calorimetry thermogram; in some embodiments, the crystal form A of the compound of formula (1) has an endothermic peak in the range of 170°C±2°C in a differential scanning calorimetry thermogram; and in some embodiments, the crystal form A of the compound of formula (1) has a differential scanning calorimetry thermogram substantially as shown in FIG. 2.

In some embodiments, the crystal form A of the compound of formula (1) has no weight loss below 200°C as determined by thermogravimetric analysis; and in some embodiments, the crystal form A of the compound of formula (1) has a thermogravimetric analysis chart substantially as shown in FIG. 3.

The present invention provides a crystal form B of the compound of formula (1) having characteristic peaks at 2$\theta$ positions of 6.4±0.2°, 10.0±0.2°, 12.7±0.2°, 15.5±0.2°, 17.6±0.2°, and 22.0±0.2° in an X-ray powder diffraction pattern by using Cu-K$\alpha$ radiation.

In some embodiments, the crystal form B of the compound of formula (1), on the basis of containing the characteristic peaks above, also has characteristic peaks at 2$\theta$ positions of 8.3±0.2°, 16.5±0.2°, 17.7±0.2°, and 20.6±0.2° in an X-ray powder diffraction pattern by using Cu-K$\alpha$ radiation.

In some embodiments, the crystal form B of the compound of formula (1) has an X-ray powder diffraction pattern at 2$\theta$ position (°) by using Cu-K$\alpha$ radiation substantially as shown in FIG. 4.

In some embodiments, the crystal form B of the compound of formula (1) has an endothermic peak in the range of about 167°C to 173°C in a differential scanning calorimetry thermogram; in some embodiments, the crystal form B of the compound of formula (1) has an endothermic peak in the range of 169°C±2°C in a differential scanning calorimetry thermogram; and in some embodiments, the crystal form B of the compound of formula (1) has a differential scanning calorimetry thermogram substantially as shown in FIG. 5.

In some embodiments, the crystal form B of the compound of formula (1) has no weight loss below 200°C as determined by thermogravimetric analysis; and in some embodiments, the crystal form B of the compound of formula (1) has a thermogravimetric analysis chart substantially as shown in FIG. 6.

The present invention provides a crystal form C of a compound of formula (1) having characteristic peaks at 2$\theta$ positions of 8.5±0.2°, 9.9±0.2°, 10.7±0.2°, and 18.5±0.2° in an X-ray powder diffraction pattern by using Cu-K$\alpha$ radiation.

In some embodiments, the crystal form C of the compound of formula (1), on the basis of containing the characteristic peaks above, also has characteristic peaks at 2$\theta$ positions of 13.0±0.2°, 13.4±0.2°, 13.8±0.2°, and 16.8±0.2° in an X-ray powder diffraction pattern by using Cu-K$\alpha$ radiation.

In some embodiments, the crystal form C of the compound of formula (1), on the basis of containing the characteristic peaks above, also has characteristic peaks at 2$\theta$ positions of 19.2±0.2°, 20.7±0.2°, 21.1±0.2°, and 22.1±0.2° in an X-ray powder diffraction pattern by using Cu-K$\alpha$ radiation.

In some embodiments, the crystal form C of the compound of formula (1) has an X-ray powder diffraction pattern at 2$\theta$ position (°) by using Cu-K$\alpha$ radiation substantially as shown in FIG. 7.

In some embodiments, the crystal form C of the compound of formula (1) has an endothermic peak in the range of about 170°C to 175°C in a differential scanning calorimetry thermogram; in some embodiments, the crystal form C of the compound of formula (1) has an endothermic peak in the range of 172°C±2°C in a differential scanning calorimetry

thermogram; and in some embodiments, the crystal form C of the compound of formula (1) has a differential scanning calorimetry thermogram substantially as shown in FIG. 8.

In some embodiments, the crystal form C of the compound of formula (1) has no weight loss below 200°C as determined by thermogravimetric analysis; and in some embodiments, the crystal form C of the compound of formula (1) has a thermogravimetric analysis chart substantially as shown in FIG. 9.

The present invention provides a crystal form D of a compound of formula (1) having characteristic peaks at $2\theta$ positions of 6.4±0.2°, 10.0±0.2°, 12.7±0.2°, 17.7±0.2°, 18.6±0.2°, and 25.5±0.2° in an X-ray powder diffraction pattern by using Cu-Kα radiation.

In some embodiments, the crystal form D of the compound of formula (1), on the basis of containing the characteristic peaks above, also has characteristic peaks at $2\theta$ positions of 14.9±0.2°, 15.6±0.2°, and 20.6±0.2° in an X-ray powder diffraction pattern by using Cu-Kα radiation.

In some embodiments, the crystal form D of the compound of formula (1), on the basis of containing the characteristic peaks above, also has characteristic peaks at $2\theta$ positions of 8.3±0.2°, 16.5±0.2°, 19.1±0.2°, and 20.2±0.2° in an X-ray powder diffraction pattern by using Cu-Kα radiation.

In some embodiments, the crystal form D of the compound of formula (1) has an X-ray powder diffraction pattern at $2\theta$ position (°) by using Cu-Kα radiation substantially as shown in FIG. 10.

In some embodiments, the crystal form D of the compound of formula (1) has an endothermic peak in the range of about 165°C to 170°C in a differential scanning calorimetry thermogram; in some embodiments, the crystal form D of the compound of formula (1) has an endothermic peak in the range of 167°C±2°C in a differential scanning calorimetry thermogram; and in some embodiments, the crystal form D of the compound of formula (1) has a differential scanning calorimetry thermogram substantially as shown in FIG. 11.

In some embodiments, the crystal form D of the compound of formula (1) has no weight loss below 200°C as determined by thermogravimetric analysis; and in some embodiments, the crystal form D of the compound of formula (1) has a thermogravimetric analysis chart substantially as shown in FIG. 12.

The present invention further provides a pharmaceutically acceptable salt of the compound of formula (1), the pharmaceutically acceptable salt is selected from a base addition salt formed by the compound of formula (1) with an inorganic base or an organic base.

In some embodiments, the base addition salt is selected from a potassium salt, a sodium salt, a lithium salt, a magnesium salt or a calcium salt.

In some embodiments, the inorganic base is selected from potassium carbonate, sodium carbonate, magnesium carbonate, calcium carbonate, sodium bicarbonate, potassium bicarbonate, magnesium bicarbonate, calcium bicarbonate, potassium hydroxide, sodium hydroxide, lithium hydroxide, magnesium hydroxide, or calcium hydroxide.

In some embodiments, the organic base is selected from sodium methylate, potassium ethylate, potassium acetate, potassium tert-butoxide, or sodium tert-butoxide.

In some embodiments, a crystal form I of the potassium salt has characteristic peaks at $2\theta$ positions of 4.8±0.2°, 5.9±0.2°, 7.1±0.2°, 8.3±0.2°, 11.8±0.2°, and 15.5±0.2° in an X-ray powder diffraction pattern by using Cu-Kα radiation.

In some embodiments, the crystal form I of the potassium salt of the compound of formula (1), on the basis of containing the characteristic peaks above, also has characteristic peaks at $2\theta$ positions of 14.6±0.2°, 16.6±0.2°, 17.9±0.2°, 18.8±0.2°, 19.6±0.2°, and 21.5±0.2° in an X-ray powder diffraction pattern by using Cu-Kα radiation.

In some embodiments, the crystal form I of the potassium salt of the compound of formula (1), on the basis of containing the characteristic peaks above, also has characteristic peaks at $2\theta$ positions of 13.0±0.2°, 14.2±0.2°, 23.3±0.2°, and 25.0±0.2° in an X-ray powder diffraction pattern by using Cu-Kα radiation.

In some embodiments, the crystal form I of the potassium salt of the compound of formula (1) has an X-ray powder diffraction pattern at $2\theta$ position (°) by using Cu-Kα radiation substantially as shown in FIG. 13.

In some embodiments, the crystal form I of the potassium salt of the compound of formula (1) has an endothermic peak in the range of about 174°C to 180°C in a differential scanning calorimetry thermogram; in some embodiments, the crystal form I of the potassium salt of the compound of formula (1) has an endothermic peak in the range of 176°C±2°C in a differential scanning calorimetry thermogram; and in some embodiments, the crystal form I of the potassium salt of the compound of formula (1) has a differential scanning calorimetry thermogram substantially as shown in FIG. 14.

In some embodiments, the crystal form I of the potassium salt of the compound of formula (1) has weight loss below 100°C as determined by thermogravimetric analysis; and in some embodiments, the crystal form I of the potassium salt of the compound of formula (1) has a thermogravimetric analysis chart substantially as shown in FIG. 15.

In some embodiments, a crystal form II of the potassium salt has characteristic peaks at $2\theta$ positions of 4.7±0.2°, 6.5±0.2°, 7.9±0.2°, 10.3±0.2°, 11.0±0.2°, and 12.1±0.2° in an X-ray powder diffraction pattern by using Cu-Kα radiation.

In some embodiments, the crystal form II of the potassium salt of the compound of formula (1), on the basis of containing the characteristic peaks above, also has characteristic peaks at $2\theta$ positions of 5.1±0.2°, 13.0±0.2°, 20.1±0.2°, 21.4±0.2°, 22.4±0.2°, and 24.1±0.2° in an X-ray powder diffraction pattern by using Cu-Kα radiation.

In some embodiments, the crystal form II of the potassium salt of the compound of formula (1), on the basis of containing the characteristic peaks above, also has characteristic peaks at 2$\theta$ positions of 8.3±0.2°, 17.6±0.2°, 19.5±0.2°, 20.6±0.2°, 21.1±0.2°, and 24.5±0.2° in an X-ray powder diffraction pattern by using Cu-K$\alpha$ radiation.

In some embodiments, the crystal form II of the potassium salt of the compound of formula (1) has an X-ray powder diffraction pattern at 2$\theta$ position (°) by using Cu-K$\alpha$ radiation substantially as shown in FIG. 16.

In some embodiments, the crystal form II of the potassium salt of the compound of formula (1) has an endothermic peak in the range of about 155°C to 165°C in a differential scanning calorimetry thermogram. In some embodiments, the crystal form II of the potassium salt of the compound of formula (1) has an endothermic peak in the range of 160°C±2°C in a differential scanning calorimetry thermogram; and in some embodiments, the crystal form II of the potassium salt of the compound of formula (1) has a differential scanning calorimetry thermogram substantially as shown in FIG. 17A.

In some embodiments, the crystal form II of the potassium salt of the compound of formula (1) has weight loss below 200°C as determined by thermogravimetric analysis; and in some embodiments, the crystal form II of the potassium salt of the compound of formula (1) has a thermogravimetric analysis chart substantially as shown in FIG. 17B.

In some embodiments, a crystal form of the magnesium salt has characteristic peaks at 2$\theta$ positions of 18.8±0.2°, 23.8±0.2°, 26.3±0.2°, and 29.5±0.2° in an X-ray powder diffraction pattern by using Cu-K$\alpha$ radiation.

In some embodiments, the crystal form of the magnesium salt of the compound of formula (1), on the basis of containing the characteristic peaks above, also has characteristic peaks at 2$\theta$ positions of 23.0±0.2°, 25.9±0.2°, 31.5±0.2°, 32.0±0.2°, 38.1±0.2°, and 39.0±0.2° in an X-ray powder diffraction pattern by using Cu-K$\alpha$ radiation.

In some embodiments, the crystal form of the magnesium salt of the compound of formula (1) has an X-ray powder diffraction pattern at 2$\theta$ position (°) by using Cu-K$\alpha$ radiation substantially as shown in FIG. 18.

In some embodiments, the crystal form of the magnesium salt of the compound of formula (1) has an endothermic peak in the range of about 165°C to 180°C in a differential scanning calorimetry thermogram; in some embodiments, the crystal form of the magnesium salt of the compound of formula (1) has an endothermic peak in the range of 170°C±2°C in a differential scanning calorimetry thermogram; and in some embodiments, the crystal form of the magnesium salt of the compound of formula (1) has a differential scanning calorimetry thermogram substantially as shown in FIG. 19.

In some embodiments, a crystal form I of the calcium salt has characteristic peaks at 2$\theta$ positions of 15.8±0.2°, 16.1±0.2°, 26.3±0.2°, 28.0±0.2°, 30.2±0.2°, and 31.2±0.2° in an X-ray powder diffraction pattern by using Cu-K$\alpha$ radiation.

In some embodiments, the crystal form I of the calcium salt of the compound of formula (1) has an X-ray powder diffraction pattern at 2$\theta$ position (°) by using Cu-K$\alpha$ radiation substantially as shown in FIG. 20.

In some embodiments, a crystal form II of the calcium salt has characteristic peaks at 2$\theta$ positions of 3.9±0.2°, 6.0±0.2°, 8.1±0.2°, 11.1±0.2°, 16.0±0.2°, 17.4±0.2°, and 19.0±0.2° in an X-ray powder diffraction pattern by using Cu-K$\alpha$ radiation.

In some embodiments, the crystal form II of the calcium salt of the compound of formula (1) has an X-ray powder diffraction pattern at 2$\theta$ position (°) by using Cu-K$\alpha$ radiation substantially as shown in FIG. 21.

In some embodiments, the crystal form II of the calcium salt of the compound of formula (1) has a differential scanning calorimetry thermogram substantially as shown in FIG. 22.

In some embodiments, the crystal form II of the calcium salt of the compound of formula (1) has weight loss below 250°C as determined by thermogravimetric analysis; and in some embodiments, the crystal form II of the calcium salt of the compound of formula (1) has a thermogravimetric analysis chart substantially as shown in FIG. 23.

In some embodiments, a crystal form I of the sodium salt has characteristic peaks at 2$\theta$ positions of 3.9±0.2°, 4.5±0.2°, 7.8±0.2°, 9.1±0.2°, 11.8±0.2°, and 12.7±0.2° in an X-ray powder diffraction pattern by using Cu-K$\alpha$ radiation.

In some embodiments, the crystal form I of the sodium salt of the compound of formula (1), on the basis of containing the characteristic peaks above, also has characteristic peaks at 2$\theta$ positions of 8.5±0.2°, 10.5±0.2°, 14.2±0.2°, 16.8±0.2°, and 17.9±0.2° in an X-ray powder diffraction pattern by using Cu-K$\alpha$ radiation.

In some embodiments, the crystal form I of the sodium salt of the compound of formula (1), on the basis of containing the characteristic peaks above, also has characteristic peaks at 2$\theta$ positions of 18.6±0.2°, 20.4±0.2°, 30.5±0.2°, and 34.7±0.2° in an X-ray powder diffraction pattern by using Cu-K$\alpha$ radiation.

In some embodiments, the crystal form I of the sodium salt of the compound of formula (1) has an X-ray powder diffraction pattern at 2$\theta$ position (°) by using Cu-K$\alpha$ radiation substantially as shown in FIG. 24.

In some embodiments, the crystal form I of the sodium salt of the compound of formula (1) has a differential scanning calorimetry thermogram substantially as shown in FIG. 25.

In some embodiments, the crystal form I of the sodium salt of the compound of formula (1) has weight loss below 200°C as determined by thermogravimetric analysis.

In some embodiments, the crystal form I of the sodium salt of the compound of formula (1) has a thermogravimetric analysis chart substantially as shown in FIG. 26.

The present invention further provides a preparation method for the crystal form A of the compound of formula (1).

The compound of formula (1) is mixed with an organic solvent I (as solvent), heated to a first temperature,

cooled to be below 35°C, filtered, and dried to obtain the crystal form A of the compound of formula (1).

In some embodiments, the preparation method for the crystal form A of the compound of formula (1) further includes the step of dropwise adding another solvent after cooling the temperature to be below 35°C, filtering, and drying the mixture to obtain the crystal form A of the compound.

In some embodiments, the another solvent is an ether solvent selected from ether, isopropyl ether, methyl tert-butyl ether, tetrahydrofuran, and 1,4-dioxane.

In some embodiments, the first temperature is selected from 40°C-95°C, such as 50°C-70°C, such as 50°C-80°C, such as 50°C-90°C, such as 60°C-70°C, such as 60°C-80°C, such as 60°C-90°C, such as 70°C-80°C, such as 70°C-90°C, such as 65°C-95°C, such as 65°C-90°C, such as 65°C-75°C, and such as 85°C-95°C. In some embodiments, the first temperature refers to a temperature when the solution is heated to clear.

In some embodiments, the organic solvent I is a first ester solvent.

In some embodiments, the first ester solvent is a fatty ester solvent.

In some embodiments, the fatty ester solvent is selected from one or a combination of two or more solvents of methyl formate, ethyl formate, propyl formate, isopropyl formate, methyl acetate, ethyl acetate, n-propyl acetate, isopropyl acetate, methyl propionate, ethyl propionate, propyl propionate, isopropyl propionate, butyl acetate, and isobutyl acetate.

In some embodiments, the fatty ester solvent is selected from one or a combination of two or more solvents of ethyl formate, methyl acetate, ethyl acetate, isopropyl acetate, n-propyl acetate, butyl acetate, and isobutyl acetate.

The "combination of two or more solvents" refers to the solvent formed by mixing the above-mentioned organic solvent I in a specific proportion, which includes, but is not limited to the following specific examples: ethyl acetate/methyl formate, ethyl acetate/ethyl formate, ethyl acetate/propyl formate, ethyl acetate/methyl acetate, ethyl acetate/n-propyl acetate, ethyl acetate/isopropyl acetate, ethyl acetate/isobutyl acetate, ethyl formate/methyl acetate, ethyl formate/n-propyl acetate, ethyl formate /isopropyl acetate, methyl acetate/n-propyl acetate, methyl acetate/isopropyl acetate, and the like.

In some embodiments, a weight ratio of the compound of formula (1) to the organic solvent I is 1: 6 to 1: 12, such as 1: 7 to 1: 12, such as 1: 8 to 1: 12, such as 1: 9 to 1: 12, such as 1: 10 to 1: 12, such as 1: 11 to 1: 12, such as 1: 7 to 1: 11, such as 1: 8 to 1: 11, such as 1: 9 to 1: 11, such as 1: 10 to 1: 11, such as 1: 7 to 1: 10, such as 1: 8 to 1: 10, such as 1: 8 to 1: 9, and such as 1: 8, 1: 9, 1: 10, 1: 11, 1: 12, and the like.

In some embodiments, the "below 35°C" refers to 5°C-35°C, such as 10°C-35°C, such as 10°C-30°C, and such as 10°C-25°C.

In some embodiments, the preparation method for the crystal form A of the compound of formula (1) may be expressed as:

mixing the compound of formula (1) with a first ester solvent, heating the mixture to 60°C-90°C, cooling the temperature to be 10°C-35°C, filtering and drying the mixture to obtain the crystal form A of the compound.

In some embodiments, the preparation method for the crystal form A of the compound of formula (1) may be expressed as:

mixing the compound of formula (1) with a first ester solvent, heating the mixture to 60°C-90°C, cooling the temperature to be 10°C-35°C, adding isopropyl ether into the mixture, stirring, filtering and drying the mixture to obtain the crystal form A of the compound.

The present invention further provides a preparation method for the crystal form B of the compound of formula (1).

The compound of formula (1) is mixed with an organic solvent II (as solvent), heated to a second temperature, cooled to be 10°C-30°C, filtered, and dried to obtain the crystal form B of the compound of formula (1).

In some embodiments, the organic solvent II is a first alcohol solvent.

In some embodiments, the first alcohol solvent is selected from one or a combination of two or more solvents of a fatty alcohol solvent, an alicyclic alcohol solvent, and an aromatic alcohol solvent.

In some embodiments, the fatty alcohol solvent is selected from ethanol, propanol, isopropanol, n-butanol, isobutanol, tert-butanol, sec-butanol, n-pentanol, n-hexanol, ethylene glycol, propylene glycol, or glycerol.

In some embodiments, the fatty alcohol solvent is selected from ethanol, n-propanol, isopropanol, isobutanol, and n-pentanol.

In some embodiments, the alicyclic alcohol solvent is selected from cyclopentanol, cyclopentanemethanol, cyclohexanol, cyclohexanemethanol, or cyclohexyl ethanol.

In some embodiments, the aromatic alcohol solvent is selected from phemethylol, phenyl ethanol, or phenylpropanol.

The "combination of two or more solvents" refers to the solvent formed by mixing the above-mentioned organic solvent II in a specific proportion, which includes, but is not limited to the following specific examples: ethanol/propanol, ethanol/isopropanol, ethanol/n-butanol, ethanol/isobutanol, ethanol/tert-butanol, ethanol/n-pentanol, ethanol/cyclopentanol, ethanol/phemethylol, ethanol/tert-butanol, and the like.

In some embodiments, a weight ratio of the compound of formula (1) to the organic solvent II is 1: 7 to 1: 13, such as 1: 8 to 1: 13, such as 1: 9 to 1: 13, such as 1: 10 to 1: 13, such as 1: 11 to 1: 13, such as 1: 12 to 1: 13, such

as 1: 7 to 1: 12, such as 1: 8 to 1: 12, such as 1: 9 to 1: 12, such as 1: 10 to 1: 12, such as 1: 11 to 1: 12, such as 1: 7 to 1: 11, such as 1: 8 to 1: 11, such as 1: 9 to 1: 11, such as 1: 10 to 1: 11, such as 1: 7 to 1: 10, such as 1: 8 to 1: 10, such as 1: 8 to 1: 9, and such as 1: 8, 1: 9, 1: 10, 1: 11, 1: 12, and the like.

In some embodiments, the second temperature is selected from 40°C-80°C, such as 50°C-60°C, such as 50°C-70°C, such as 50°C-80°C, such as 60°C-70°C, such as 60°C-80°C, such as 70°C-80°C, such as 55°C-65°C, and such as 60°C-65°C. In some embodiments, the second temperature refers to a temperature when the solution is heated to clear.

In some embodiments, the cooling refers to cooling by air bath stirring, natural cooling, and the like.

In some embodiments, the temperature of the air bath refers to 20°C-30°C, such as 25°C-30°C, such as 26°C-30°C, such as 27°C-30°C, such as 28°C-30°C, and such as 29°C-30°C.

The present invention further provides a preparation method for the crystal form C of the compound of formula (1).

The compound of formula (1) is mixed with an organic solvent III (as solvent), heated to a third temperature, cooled for 10h-25h, filtered and dried, and then transferred to an oven of 100°C-140°C for 1-5h to obtain the crystal form C of the compound of formula (1).

In some embodiments, the organic solvent III refers to methanol.

In some embodiments, a weight ratio of the compound of formula (1) to the organic solvent III is 1: 2 to 1: 8, such as 1: 3 to 1: 8, such as 1: 4 to 1: 8, such as 1: 5 to 1: 8, such as 1: 6 to 1: 8, such as 1: 7 to 1: 8, such as 1: 2 to 1: 7, such as 1: 3 to 1: 7, such as 1: 4 to 1: 7, such as 1: 5 to 1: 7, such as 1: 6 to 1: 7, such as 1: 2 to 1: 6, such as 1: 3 to 1: 6, such as 1: 4 to 1: 6, such as 1: 5 to 1: 6, such as 1: 2 to 1: 5, such as 1: 3 to 1: 5, such as 1: 4 to 1: 5, such as 1: 2 to 1: 4, such as 1: 3 to 1: 4, such as 1: 2 to 1: 3, and such as 1: 4, 1: 5, 1: 6, 1: 7, and the like.

In some embodiments, the third temperature is selected from 40°C-80°C, such as 50°C-60°C, such as 50°C-70°C, such as 50°C-80°C, such as 60°C-70°C, such as 60°C-80°C, such as 70°C-80°C, such as 50°C-65°C, such as 55°C-60°C, and such as 60°C-65°C. In some embodiments, the third temperature refers to a temperature when the solution is heated to clear.

In some embodiments, the preparation method for the crystal form C may be expressed as:
mixing the compound of formula (1) with methanol, heating the mixture to 50°C-65°C, cooling for 15h-20h, filtering, drying, and then transferring the mixture to an oven of 120°C-130°C for 1-3h to obtain the crystal form C of the compound of formula (1).

The present invention further provides a preparation method for the crystal form D of the compound of formula (1).

The compound of formula (1) is mixed with an organic solvent IV (as solvent), added with water after dissolving, the mixture is filtered to obtain a solid, an organic solvent V is added, the mixture is heated and refluxed for 10-25h, preferably 15-25h; and then the mixture is filtered and dried to obtain the crystal form D of the compound of formula (1).

In some embodiments, the organic solvent IV is an organic solvent that is at least slightly soluble in water, such as an organic solvent that is mutually soluble with water.

In some embodiments, the organic solvent IV is a first alcohol solvent.

In some embodiments, the first alcohol solvent is selected from one or a combination of two or more solvents of a fatty alcohol solvent, an alicyclic alcohol solvent, and an aromatic alcohol solvent.

In some embodiments, the fatty alcohol solvent is selected from ethanol, propanol, isopropanol, n-butanol, isobutanol, tert-butanol, sec-butanol, n-pentanol, n-hexanol, ethylene glycol, propylene glycol, or glycerol.

In some embodiments, the fatty alcohol solvent is selected from ethanol, n-propanol, isopropanol, isobutanol, and n-pentanol.

In some embodiments, the alicyclic alcohol solvent is selected from cyclopentanol, cyclopentanemethanol, cyclohexanol, cyclohexanemethanol, or cyclohexyl ethanol.

In some embodiments, the aromatic alcohol solvent is selected from phemethylol, phenyl ethanol, or phenylpropanol.

In some embodiments, a volume ratio of the organic solvent IV to the water is selected from 1: 6 to 6: 1, such as 1: 5 to 5: 1, such as 1: 4 to 4: 1, such as 1: 3 to 3: 1, such as 1: 2 to 2: 1, such as 4: 5 to 5: 4, such as 1: 2, such as 1: 1, such as 3: 2, such as 3: 4, such as 3: 5, such as 4: 3, such as 4: 5, such as 2: 3, such as 5: 3, such as 5: 4, such as 5: 6, such as 5: 7, such as 5: 8, such as 5: 9, such as 6: 5, such as 6: 7, such as 6: 10, such as 6: 11, such as 7: 4, such as 7: 5, such as 7: 6, such as 7: 8, such as 7: 9, such as 7: 10, such as 7: 11, such as 7: 12, such as 7: 13, such as 8: 5, such as 8: 7, such as 8: 9, such as 8: 11, such as 8: 13, such as 8: 14, such as 8: 15, such as 9: 5, such as 9: 7, such as 9: 8, such as 9: 10, such as 9: 11, such as 9: 13, such as 9: 14, such as 9: 16, and such as 9: 17.

In some embodiments, a weight ratio of the compound of formula (1) to the organic solvent IV is 1: 7 to 1: 13, such as 1: 8 to 1: 13, such as 1: 9 to 1: 13, such as 1: 10 to 1: 13, such as 1: 11 to 1: 13, such as 1: 12 to 1: 13, such as 1: 8 to 1: 12, such as 1: 9 to 1: 12, such as 1: 10 to 1: 12, such as 1: 11 to 1: 12, such as 1: 8 to 1: 11, such as 1: 9 to 1: 11, such as 1: 10 to 1: 11, such as 1: 8 to 1: 10, such as 1: 9 to 1: 10, such as 1: 8 to 1: 9, such as 1: 5, such as 1: 6, such as 1: 7, such as 1: 8, such as 1: 9, such as 1: 10, such as 1: 11, such as 1: 12, such as 1: 13, such as 1: 14, and such as 1: 15.

In some embodiments, the organic solvent V is an ether solvent selected from ether, isopropyl ether, methyl

tert-butyl ether, tetrahydrofuran, and 1,4-dioxane. In some embodiments, the filtering described in this preparation methods for the crystal form A, the crystal form B, the crystal form C or the crystal form D may be performed by suction filtration.

In some embodiments, the process of preparing the crystal form A, the crystal form B, the crystal form C or the crystal form D further includes the step of stirring. In some embodiments, the stirring is mechanical stirring or manual stirring. In some embodiments, the stirring is mechanical stirring.

In some embodiments, the drying described in this preparation methods for the crystal form A, the crystal form B, the crystal form C or the crystal form D may be carried out by vacuum drying, oven drying, natural airing or ventilation drying, and a drying temperature does not exceed 60°C, such as 30°C-55°C, and such as 35°C-50°C.

In some embodiments, the method further includes the step of adding crystal seeds of the crystal form A, the crystal form B, the crystal form C or the crystal form D in the cooling process, so as to facilitate the formation and precipitation of various crystal forms, wherein the crystal seeds of various crystal forms are prepared by the method of preparing various crystal forms without adding crystal seeds in the application document.

The present invention further provides a preparation method for the pharmaceutically acceptable salt of the compound of formula (1):
reacting the compound of formula (1) with an inorganic base in a reaction solvent A.

In some embodiments, the inorganic base is selected from potassium carbonate, sodium carbonate, magnesium carbonate, calcium carbonate, potassium bicarbonate, sodium bicarbonate, magnesium bicarbonate, calcium bicarbonate, potassium hydroxide, sodium hydroxide, lithium hydroxide, magnesium hydroxide, or calcium hydroxide.

In some embodiments, the pharmaceutically acceptable salt is selected from a potassium salt, a sodium salt, a lithium salt, a magnesium salt or a calcium salt of the compound of formula (1).

In some embodiments, the reaction solvent A is selected from one or a combination of two or more solvents of a second alcohol solvent, a ketone solvent, a nitrile solvent, or a second ester solvent.

In some embodiments, the second alcohol solvent is selected from one or more of a fatty alcohol solvent, an alicyclic alcohol solvent, and an aromatic alcohol solvent. Preferably, the fatty alcohol solvent is selected from methanol, ethanol, propanol, isopropanol, n-butanol, isobutanol, tert-butanol, sec-butanol, n-pentanol, n-hexanol, ethylene glycol, propylene glycol, or glycerol; more preferably, the fatty alcohol solvent is selected from methanol, ethanol, n-propanol, isopropanol, isobutanol, or n-pentanol; preferably, the alicyclic alcohol solvent is selected from cyclopentanol, cyclopentanemethanol, cyclohexanol, cyclohexanemethanol, or cyclohexylethanol; and preferably, the aromatic alcohol solvent is selected from phemethylol, phenylethanol, or phenylpropanol.

In some embodiments, the ketone solvent is selected from acetone.

In some embodiments, the nitrile solvent is selected from acetonitrile.

In some embodiments, the second ester solvent is a fatty ester solvent; preferably, the fatty ester solvent is selected from one or a combination of two or more solvents of methyl formate, ethyl formate, propyl formate, isopropyl formate, methyl acetate, ethyl acetate, n-propyl acetate, isopropyl acetate, methyl propionate, ethyl propionate, propyl propionate, isopropyl propionate, butyl acetate, and isobutyl acetate; and further preferably, the fatty ester solvent is selected from one or a combination of two or more solvents of ethyl formate, methyl acetate, ethyl acetate, isopropyl acetate, n-propyl acetate, butyl acetate, and isobutyl acetate.

The present invention also provides a preparation method for the crystal form of the potassium salt of the compound of formula (1):
reacting the compound of formula (1) with the potassium salt in a reaction solvent B.

In some embodiments, the potassium salt is selected from potassium carbonate, potassium bicarbonate, or potassium hydroxide.

In some embodiments, the reaction solvent B is selected from a third alcohol solvent.

In some embodiments, the third alcohol solvent is selected from one or a combination of two or more solvents of a fatty alcohol solvent, an alicyclic alcohol solvent, and an aromatic alcohol solvent.

In some embodiments, the fatty alcohol solvent is selected from one or a combination of two or more solvents of methanol, ethanol, propanol, isopropanol, n-butanol, isobutanol, tert-butanol, sec-butanol, n-pentanol, n-hexanol, ethylene glycol, propylene glycol, or glycerol.

In some embodiments, the fatty alcohol solvent is selected from one or a combination of two or more solvents of methanol, ethanol, n-propanol, isopropanol, isobutanol, and n-pentanol.

In some embodiments, the alicyclic alcohol solvent is selected from cyclopentanol, cyclopentanemethanol, cyclohexanol, cyclohexanemethanol, or cyclohexyl ethanol.

In some embodiments, the aromatic alcohol solvent is selected from phemethylol, phenyl ethanol, or phenylpropanol.

In order to analyze the obtained crystals, an X-ray diffraction crystal analysis method is usually adopted.

When the crystal form of the present invention is measured by using X-ray powder diffraction, sometimes there is a slight measurement error for measured peaks due to a measuring instrument or measuring conditions, and the crystallization of a spectral peak within the error range is also included in the crystals of the present invention. Therefore,

this error should be taken into account when determining a crystal structure. Therefore, the applicant considered that the error range was within ±0.2° when determining the $2\theta$ position. In addition, main peaks of X-ray powder diffraction (XRPD) of different samples with specific crystal forms are the same, but secondary peaks may be changed.

The present invention further provides a pharmaceutical composition, which contains the crystal form A, the crystal form B, the crystal form C, and the crystal form D, or the pharmaceutically acceptable salt of the compound of formula (1), the crystal form of the pharmaceutically acceptable salt, as well as one or more second therapeutic active agents. Optionally, the pharmaceutical composition also contains one or more pharmaceutically acceptable carriers and/or diluents.

The present invention further provides a pharmaceutical formulation, which contains the crystal form A, the crystal form B, the crystal form C, the crystal form D, or the pharmaceutically acceptable salt of the compound of formula (1) and the crystal form of the pharmaceutically acceptable salt, as well as one or more pharmaceutically acceptable carriers and/or diluents. The pharmaceutical formulation is any clinically or pharmaceutically acceptable dosage form.

In some embodiments of the present invention, the above pharmaceutical formulation is administered orally, parenterally, rectally or intrapulmonally to a patient or a subject in need of such treatment. When used for oral administration, the pharmaceutical formulation may be made into a conventional solid preparation, such as tablet, capsule, pill, granule, and the like. The pharmaceutical formulation may also be made into oral liquid formulation, such as oral solution, oral suspension, syrup, and the like. When the pharmaceutical formulation is made into an oral formulation, appropriate filler, adhesive, disintegrant, lubricant, and the like, may be added. When being used for parenteral administration, the pharmaceutical formulation may be made into an injection, including an injection solution, a sterile powder for injection and a concentrated solution for injection. When the pharmaceutical formulation is made into an injection, the pharmaceutical formulation may be produced by using a conventional method in the existing pharmaceutical field. When preparing the injection, it is not necessary to add additive, or appropriate additive may be added according to the property of the medicament. When being used for rectal administration, the pharmaceutical formulation may be made into suppository, and the like. When being used for pulmonary administration, the pharmaceutical formulation may be made into inhalant or spray, and the like.

The present invention further provides a method for treating and/or preventing a KHK-mediated disease and related disease, which includes the step of administering an effective amount of a crystal form A, a crystal form B, a crystal form C, a crystal form D, or a pharmaceutically acceptable salt of 2-((1*R*,5*S*,6*R*)-3-(7,7-difluoro-2-((*S*)-2-methylazetidin-1-yl)-6,7-dihydro-5*H*-cyclopenta [*d*]pyrimidin-4-yl)-3-azabicyclo[3.1.0]hexan-6-yl)acetic acid and a crystal form of the pharmaceutically acceptable salt to a patient in need of such treatment.

In one embodiment, the KHK-mediated disease and related diseases are selected from endocrine disorders, urinary diseases, metabolic diseases, nonalcoholic steatohepatitis, cirrhosis, fatty liver, hepatitis, liver failure, hereditary fructose intolerance, nonalcoholic fatty liver disease, hepatobiliary diseases, fibrotic diseases, cardiovascular and cerebrovascular diseases, inflammatory-immune diseases, central nervous system diseases, gastrointestinal diseases, and hyperproliferative diseases such as cancer.

The present invention further provides use of the crystal form A, the crystal form B, the crystal form C, the crystal form D, or the pharmaceutically acceptable salt of the compound of formula (1) and the crystal form of the pharmaceutically acceptable salt in preparation of a medicament for treatment and/or prevention of a KHK-mediated disease or related disease.

In one embodiment, the KHK-mediated disease and related disease are selected from endocrine disorders, urinary diseases, metabolic diseases, nonalcoholic steatohepatitis, cirrhosis, fatty liver, hepatitis, liver failure, hereditary fructose intolerance, nonalcoholic fatty liver disease, hepatobiliary diseases, fibrotic diseases, cardiovascular and cerebrovascular diseases, inflammatory-immune diseases, central nervous system diseases, gastrointestinal diseases, and hyperproliferative diseases such as cancer.

The present invention further provides use of the crystal form A, the crystal form B, the crystal form C, the crystal form D, or the pharmaceutically acceptable salt of the compound of formula (1) and the crystal form of the pharmaceutically acceptable salt for treatment and/or prevention of a KHK-mediated disease and related disease.

In one embodiment, the KHK-mediated disease and related disease are selected from endocrine disorders, urinary diseases, metabolic diseases, nonalcoholic steatohepatitis, cirrhosis, fatty liver, hepatitis, liver failure, hereditary fructose intolerance, nonalcoholic fatty liver diseases, hepatobiliary diseases, fibrotic diseases, cardiovascular and cerebrovascular diseases, inflammatory-immune diseases, central nervous system diseases, gastrointestinal diseases, and hyperproliferative diseases such as cancer.

The present invention further provides a composition of the crystal form A, the crystal form B, the crystal form C, the crystal form D, the pharmaceutically acceptable salt of the compound of formula (1) and the crystal form of the pharmaceutically acceptable salt with one or more other medicaments, wherein these medicaments may be administered simultaneously or successively with the crystal form A, the crystal form B, the crystal form C, the crystal form D, the pharmaceutically acceptable salt of the compound of formula (1) and the crystal form of the pharmaceutically acceptable salt for treating and/or preventing a KHK-mediated disease and related disease.

In one embodiment, the KHK-mediated disease and related disease are selected from endocrine disorders, urinary diseases, metabolic diseases, nonalcoholic steatohepatitis, cirrhosis, fatty liver, hepatitis, liver failure, hereditary fructose intolerance, nonalcoholic fatty liver diseases, hepatobiliary diseases, fibrotic diseases, cardiovascular and cerebrovascular diseases, inflammatory-immune diseases, central nervous system diseases, gastrointestinal diseases, and hyperproliferative diseases such as cancer.

Unless otherwise specified in the present invention, all scientific and technical terms used herein have the meanings commonly understood by those skilled in the art. Meanwhile, in order to better understand the present invention, definitions and explanations of some terms are provided below.

As used herein, the "effective dose" refers to a dose sufficient to achieve a desired therapeutic or preventive effect, for example, a dose to achieve relief of symptoms related to diseases to be treated.

As used herein, the "treatment" refers to the alleviation or elimination of a targeted disease state or symptom. If a subject receives a therapeutic dose of the crystal form or the pharmaceutical composition thereof according to the method described herein, and one or more indications and symptoms of the subject show an observable and/or detectable decrease or improvement, the subject is successfully "treated". It should also be understood that the treatment of the disease state or symptom includes not only complete treatment, but also includes incomplete treatment, but achieves some biological or medical related results.

The main advantages of the crystal form A, the crystal form B, the crystal form C, the crystal form D, and the pharmaceutically acceptable salt of the compound of formula (1) and the crystal form of the pharmaceutically acceptable salt include that:

(1) the preparation methods for the crystal form A, the crystal form B, the crystal form C, the crystal form D, and the pharmaceutically acceptable salt of the compound 2-((1*R*,5*S*,6*R*)-3-(7,7-difluoro-2-((*S*)-2-methylazetidin-1-yl)-6,7-dihydro-5*H*-cyclopenta [*d*] pyrimidin-4-yl)-3-azabicyclo[3.1.0]hexan-6-yl)acetic acid and the crystal form of the pharmaceutically acceptable salt provided by the present invention are simple and convenient to operate, and suitable for industrial production;

(2) the crystal form A, the crystal form B, the crystal form C, the crystal form D, the pharmaceutically acceptable salt, and the crystal form of the pharmaceutically acceptable salt provided by the present invention have good properties, which are convenient for detection, formulation, transportation and storage;

(3) the crystal form A, the crystal form B, the crystal form C, the crystal form D, the pharmaceutically acceptable salt, and the crystal form of the pharmaceutically acceptable salt provided by the present invention have high purity, little residual solvent, high solubility, good stability and easy quality control;

(4) the crystal form A, the crystal form B, the crystal form C, the crystal form D, the pharmaceutically acceptable salt, and the crystal form of the pharmaceutically acceptable salt provided by the present invention have excellent bioavailability; and

(5) the crystal form A, the crystal form B, the crystal form C, the crystal form D, the pharmaceutically acceptable salt, and the crystal form of the pharmaceutically acceptable salt provided by the present invention have good inhibitory effects on KHK kinase, and may be used for treating and/or preventing the KHK-mediated disease and related disease.

## BRIEF DESCRIPTION OF THE DRAWINGS

FIG. 1 shows an X-ray powder diffraction pattern of a crystal form A of a compound of formula (1), wherein an ordinate indicates diffraction intensity and an abscissa indicates a diffraction angle ($2\theta$).

FIG. 2 shows a differential scanning calorimetry (DSC) thermogram of the crystal form A of the compound of formula (1), wherein an ordinate indicates a heat flow in a unit of (W/g), and an abscissa indicates a temperature in a unit of (°C).

FIG. 3 shows a thermogravimetric analysis (TG) curve and a derivative thermogravimetric analysis (DTG) curve of the crystal form A of the compound of formula (1), wherein an abscissa indicates a temperature (°C), an ordinate in the left indicates a weight (%), and an ordinate in the right indicates a relationship between a weight loss rate (%) and the temperature.

FIG. 4 shows an X-ray powder diffraction pattern of a crystal form B of a compound of formula (1), wherein an ordinate indicates diffraction intensity and an abscissa indicates a diffraction angle ($2\theta$).

FIG. 5 shows a differential scanning calorimetry (DSC) thermogram of the crystal form B of the compound of formula (1), wherein an ordinate indicates a heat flow in a unit of (W/g), and an abscissa indicates a temperature in a unit of (°C).

FIG. 6 shows a thermogravimetric analysis (TG) curve and a derivative thermogravimetric analysis (DTG) curve of the crystal form B of the compound of formula (1), wherein an abscissa indicates a temperature (°C), an ordinate in the left indicates a weight (%), and an ordinate in the right indicates a relationship between a weight loss rate (%)

and the temperature.

FIG. 7 shows an X-ray powder diffraction pattern of a crystal form C of a compound of formula (1), wherein an ordinate indicates diffraction intensity and an abscissa indicates a diffraction angle ($2\theta$).

FIG. 8 shows a differential scanning calorimetry (DSC) thermogram of the crystal form C of the compound of formula (1), wherein an ordinate indicates a heat flow in a unit of (W/g), and an abscissa indicates a temperature in a unit of (°C).

FIG. 9 shows a thermogravimetric analysis (TG) curve and a derivative thermogravimetric analysis (DTG) curve of the crystal form C of the compound of formula (1), wherein an abscissa indicates a temperature (°C), an ordinate in the left indicates a weight (%), and an ordinate in the right indicates a relationship between a weight loss rate (%) and the temperature.

FIG. 10 shows an X-ray powder diffraction pattern of a crystal form D of a compound of formula (1), wherein an ordinate indicates diffraction intensity and an abscissa indicates a diffraction angle ($2\theta$).

FIG. 11 shows a differential scanning calorimetry (DSC) thermogram of the crystal form D of the compound of formula (1), wherein an ordinate indicates a heat flow in a unit of (W/g), and an abscissa indicates a temperature in a unit of (°C).

FIG. 12 shows a thermogravimetric analysis (TG) curve and a derivative thermogravimetric analysis (DTG) curve of the crystal form D of the compound of formula (1), wherein an abscissa indicates a temperature (°C), an ordinate in the left indicates a weight (%), and an ordinate in the right indicates a relationship between a weight loss rate (%) and the temperature.

FIG. 13 shows an X-ray powder diffraction pattern of a crystal form I of a potassium salt of the compound of formula (1), wherein an ordinate indicates diffraction intensity and an abscissa indicates a diffraction angle ($2\theta$).

FIG. 14 shows a differential scanning calorimetry (DSC) thermogram of the crystal form I of the potassium salt of the compound of formula (1), wherein an ordinate indicates a heat flow in a unit of (W/g), and an abscissa indicates a temperature in a unit of (°C).

FIG. 15 shows a thermogravimetric analysis (TG) curve and a derivative thermogravimetric analysis (DTG) curve of the crystal form I of the potassium salt of the compound of formula (1), wherein an abscissa indicates a temperature (°C), an ordinate in the left indicates a weight (%), and an ordinate in the right indicates a relationship between a weight loss rate (%) and the temperature.

FIG. 16 shows an X-ray powder diffraction pattern of a crystal form II of the potassium salt of the compound of formula (1), wherein an ordinate indicates diffraction intensity and an abscissa indicates a diffraction angle ($2\theta$).

FIG. 17A shows a differential scanning calorimetry (DSC) thermogram of the crystal form II of the potassium salt of the compound of formula (1), wherein an ordinate indicates a heat flow in a unit of (W/g), and an abscissa indicates a temperature in a unit of (°C).

FIG. 17B shows a thermogravimetric analysis (TG) curve of the crystal form II of the potassium salt of the compound of formula (1), wherein an abscissa indicates a temperature (°C), and ordinate indicates a weight (%).

FIG. 18 shows an X-ray powder diffraction pattern of a crystal form of a magnesium salt of the compound of formula (1), wherein an ordinate indicates diffraction intensity and an abscissa indicates a diffraction angle ($2\theta$).

FIG. 19 shows a differential scanning calorimetry (DSC) thermogram of the crystal form of the magnesium salt of the compound of formula (1), wherein an ordinate indicates a heat flow in a unit of (W/g), and an abscissa indicates a temperature in a unit of (°C).

FIG. 20 shows an X-ray powder diffraction pattern of a crystal form I of a calcium salt of the compound of formula (1), wherein an ordinate indicates diffraction intensity and an abscissa indicates a diffraction angle ($2\theta$).

FIG. 21 shows an X-ray powder diffraction pattern of a crystal form II of the calcium salt of the compound of formula (1), wherein an ordinate indicates diffraction intensity and an abscissa indicates a diffraction angle ($2\theta$).

FIG. 22 shows a differential scanning calorimetry (DSC) thermogram of the crystal form II of the calcium salt of the compound of formula (1), wherein an ordinate indicates a heat flow in a unit of (W/g), and an abscissa indicates a temperature in a unit of (°C).

FIG. 23 shows a thermogravimetric analysis (TG) curve of the crystal form II of the calcium salt of the compound of formula (1), wherein an abscissa indicates a temperature (°C), and ordinate indicates a weight (%).

FIG. 24 shows an X-ray powder diffraction pattern of a crystal form I of a sodium salt of the compound of formula (1), wherein an ordinate indicates diffraction intensity and an abscissa indicates a diffraction angle ($2\theta$).

FIG. 25 shows a differential scanning calorimetry (DSC) thermogram of the crystal form I of the sodium salt of the compound of formula (1), wherein an ordinate indicates a heat flow in a unit of (W/g), and an abscissa indicates a temperature in a unit of (°C).

FIG. 26 shows a thermogravimetric analysis (TG) curve of the crystal form I of the sodium salt of the compound of formula (1), wherein an abscissa indicates a temperature (°C), and ordinate indicates a weight (%).

## DETAILED DESCRIPTION OF THE EMBODIMENTS

The following further describes the present invention in detail with reference to specific embodiments. It shall not be construed as a limitation on the protection scope of the present invention. All technologies implemented based on the foregoing content of the present invention shall fall within the intended protection scope of the present invention.

The following abbreviations represent the following definitions:

DMSO: dimethyl sulfoxide; PE: petroleum ether; EA: ethyl acetate; DAST: diethylamine sulfur trifluoride; NMP: N-methyl pyrrolidone; DBN: 1,5-diazabicyclo [4.3.0] non-5-ene; THF: tetrahydrofuran; DIEA: N, N-diisopropylethylamine; IPAc: isopropyl acetate.

### Preparation Example

### (1) Preparation of 1-ethyl 2,4-dimethyl 1-oxobutan-1,2,4-tricarboxylate

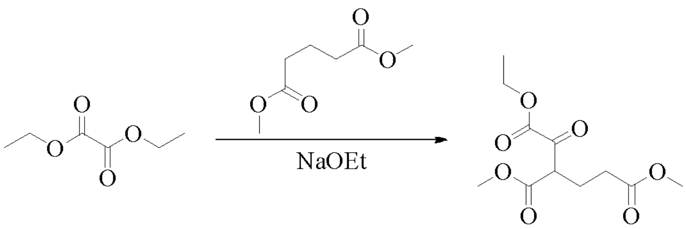


Sodium metal (4.3 g, 186.9 mmol) was added to ethanol (200 mL), completely dissolved, spin-dried and suspended in tetrahydrofuran (200 mL). Diethyl oxalate (27.4 g, 187.5 mmol) was slowly added to the solution, then added dimethyl glutarate (30.0 g, 187.3 mmol), and reacted at 25°C for 16 hours. The reaction was detected by TLC (Rf=0.5, PE: EA=1: 1), spin-dried, then added with water (300 mL) and methyl tert-butyl ether (200 mL), and subjected to liquid separation. An aqueous phase was adjusted to the pH of 1, and extracted with ethyl acetate (3×200 ml). Organic phases were combined, dried with anhydrous sodium sulfate, filtered and spin-dried, and the residue was directly used for next step.

### (2) Preparation of 2- oxohexanedioic acid

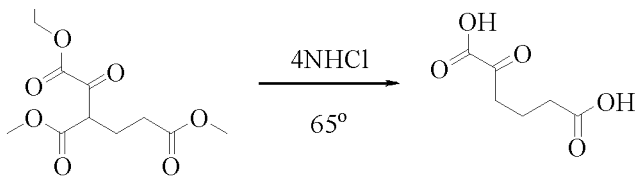


1-ethyl 2,4-dimethyl 1-oxobutane-1,2,4-tricarboxylate (crude product of the previous step) was added into 4N hydrochloric acid (230 mL, 920 mmol), heated to 65°C for 6 hours, and spin-dried, to obtain 21.0 g of compounds, with a two-step yield of 70.0%.

### (3) Preparation of 6-methoxy-5,6-dioxohexanoic acid

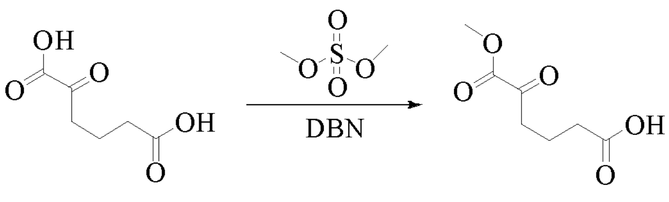

At 0°C, 2-oxohexanedioic acid (10.0 g, 62.5 mmol) was added to acetone (100 mL) solution containing DBN (9.3 g,74.9 mmol), and dropwise added with dimethyl sulfate (7.9 g, 62.6 mmol), and dried at 25°C for 16 hours. Water (50 mL) was added, adjusted the pH to be 2-3, and then the solution was extracted with ethyl acetate (3×100 ml). Organic phases were combined, dried with anhydrous sodium sulfate, filtered and spin-dried, and the residue was directly used for next step.

**(4) Preparation of dimethyl 2-oxohexanedioate**

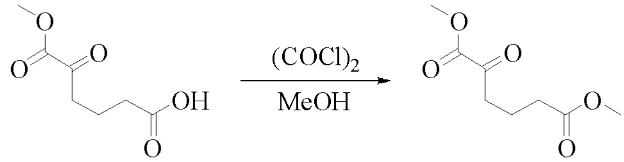


6-methoxy-5,6-dioxohexanoic acid (crude product of the previous step) was dissolved in dichloromethane (100 mL), added with 2 drops of N,N-dimethylformamide, at 0°C, added with oxalyl chloride (15.9 g, 125.2 mmol), and spin-dried at 25°C for 16 hours. At 0°C, methanol (40 mL) was added to the residue, and spin-dried at 25°C for 2 hours. The residue was subjected to column chromatography (PE: EA=5: 1) to obtain a product (7.9 g, with a two-step yield of 66.9%).

**(5) Preparation of dimethyl 2,2-difluorohexanedioate**

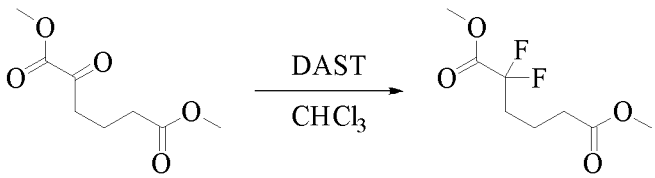


Dimethyl 2-oxohexanedioate (4.8 g, 25.5 mmol) was dissolved in chloroform (50 mL) and then added into DAST, reacted at 25°C for 48 hours, added with water for quenching, and then extracted with ethyl acetate (3×100 ml). Organic phases were combined, concentrated and subjected to column chromatography (ethyl acetate: petroleum ether=1: 3) to obtain a product (2.0 g, 37.0%).

**(6) Preparation of methyl 3,3-difluoro-2-oxocyclopentane-1-carboxylate**

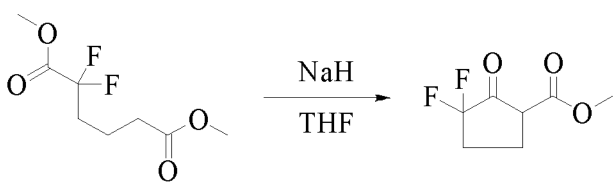


Sodium hydride (60%) (0.57 g, 14.2 mmol) was dissolved in THF (40 mL), added with THF solution (10 mL) containing dimethyl 2,2-difluorohexanedioate (2.0 g, 9.5 mmol), reacted at 20°C for 16 hours, added with water for quenching, and the pH was adjusted to be 5-6. The solution was extracted with ethyl acetate (3×100 ml). Organic phases were combined, dried with anhydrous sodium sulfate, filtered, and concentrated. The residue was directly used for next step (1.6 g of product, with a yield of 94.1%).

**(7) Preparation** of **7,7-difluoro-2-(methylthio)-6,7-dihydro-5*H*-cyclopenta[*d*]pyrimidine-4-ol**

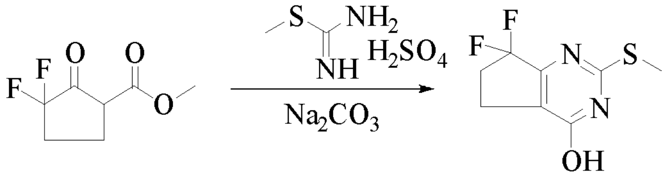


Methyl 3,3-difluoro-2-oxocyclopentane-1-carboxylate (2.4 g, 13.5 mmol) was dissolved in water (40 mL), added with methylisothiourea sulfate (3.8 g, 20.2 mmol) and sodium carbonate (2.85 g, 26.9 mmol), and reacted at 25°C for 16 hours. The pH was adjusted to be 2 with 1N hydrochloric acid, and then the solution was extracted with ethyl acetate (3×100 ml). Organic phases were combined, dried with anhydrous sodium sulfate, filtered and concentrated to obtain 2.4 g of crude products directly used for next reaction.

**(8) Preparation** of **4-chloro-7,7-difluoro-2-(methylthio)-6,7-dihydro-5*H*-cyclopenta[*d*]pyrimidine**

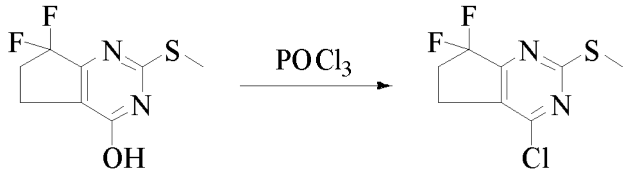


7,7-difluoro-2-(methylthio)-6,7-dihydro-5*H*-cyclopenta[*d*]pyrimidin-4-ol (2.4 g crude product) was dissolved in phosphorus oxychloride (5 mL)/1,2-dichloroethane (10 mL), reacted at 110°C for 16 hours, and then concentrated. The pH was adjusted to be 7-8 with saturated sodium bicarbonate, and then the solution was extracted with ethyl acetate (3×80 ml). Organic phases were combined, concentrated, and subjected to column chromatography (ethyl acetate: petroleum ether=1: 10) to obtain 1.4 g of compounds, with a two-step yield of 43.8%.

**(9) Preparation of methyl 2-((1*R*,5*S*,6s)-3-(7,7-difluoro-2-(methylthio)-6,7-dihydro-5*H*-cyclopenta[*d*]pyrimidi n-4-yl)-3-azabicyclo[3.1.0]hexan-6-yl) acetate**

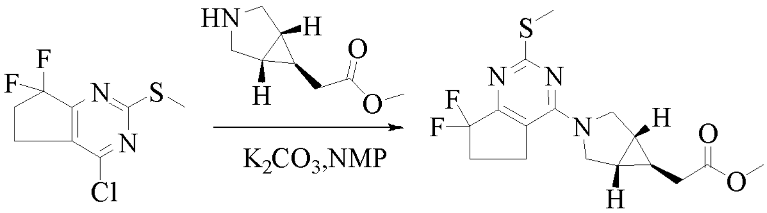


4-chloro-7,7-difluoro-2-(methylthio)-6,7-dihydro-5H-cyclopenta[d]pyrimidine (200 mg, 0.84 mmol) was dissolved in NMP (5 mL), added with methyl 2-((1*R*,5*S*,6*s*)-3-azabicyclo[3.1.0] hexan-6-yl) acetate (160 mg, 1.03 mmol) and potassium carbonate (260 mg, 1.88 mmol), reacted at 90°C for 16 hours, added with water, and extracted with ethyl acetate (3×50 ml). Organic phases were combined and concentrated. Crude products were directly used for next step.

**(10)Preparation of methyl 2-((1*R*,5*S*,6s)-3-(7,7-difluoro-2-(methylsulfonyl)-6,7-dihydro-5*H*-cyclopenta[*d*]pyri midin-4-yl)-3-azabicyclo[3.1.0]hexan-6-yl)acetate**

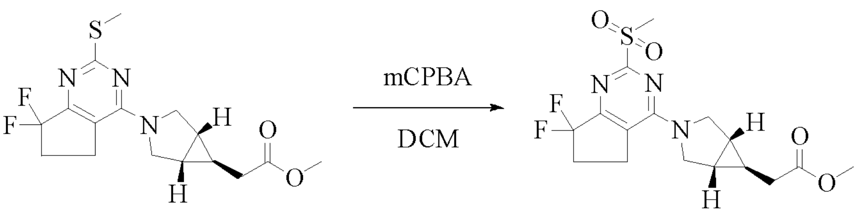


Methyl 2-((1*R*,5*S*,6s)-3-(7,7-difluoro-2-(methylthio)-6,7-dihydro-5*H*-cyclopenta[*d*]pyrimidin-4 -yl)-3-azabicyclo[3.1.0]hexan-6-yl) acetate (crude product of the previous step) was dissolved in dichloromethane (10 mL), added with m-chloroperoxybenzoic acid (80%) (400 mg, 1.85 mmol), and reacted at 20°C for 3 hours. The solution was added with

saturated sodium carbonate solution, and subjected to liquid separation. An aqueous phase was extracted with dichloromethane (2×30 ml). Organic phases were combined, spin-dried, and subjected to column chromatography (ethyl acetate: petroleum ether=3: 1) to obtain 160 mg of compounds, with a two-step yield of 48.6%.

**(11)Preparation of methyl 2-((1*R*,5*S*,6*R*)-3-(7,7-difluoro-2-((*S*)-2-methylazetidin-1-yl)-6,7-dihydro-5*H*-cyclope nta[*d*]pyrimidine-4-yl)-3-azabicyclo[3.1.0]hexan-6-yl)acetate**

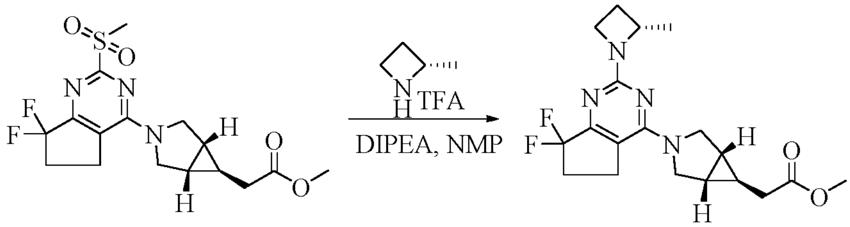


Methyl 2-((1*R*,5*S*,6s)-3-(7,7-difluoro-2-(methylsulfonyl)-6,7-dihydro-5*H*-cyclopenta[*d*]pyrimid in-4-yl)-3-azabicyclo[3.1.0]hexan-6-yl)acetate (100 mg, 0.26 mmol) was dissolved in NMP (3 mL), added with DIEA (0.5 ml) and (S)-2-methylazetidine trifluoroacetate salt (107 mg, 0.58 mmol), reacted in microwave at 160°C for 2 hours, added with water, and extracted with ethyl acetate (2×30 ml). Organic phases were combined, spin-dried, and subjected to column chromatography (ethyl acetate: petroleum ether=1: 1) to obtain 60 mg of compounds, with a yield of 61.4%.

(12) **Preparation of 2-((1*R*,5*S*,6*R*)-3-(7,7-difluoro-2-((*S*)-2-methylazetidin-1-yl)-6,7-dihydro-5*H*-cyclopenta[*d*]pyrimidin-4-yl)-3-azabicyclo[3.1.0]hexan-6-yl)a cetic acid (preparation of compound of formula (1))**

Method I:

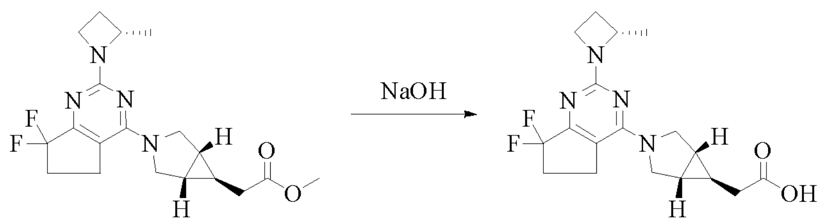


Methyl 2-((1*R*,5*S*,6*R*)-3-(7,7-difluoro-2-((*S*)-2-methylazetidine-1-yl)-6,7-dihydro-5*H*-cyclopent a[*d*]pyrimidin-4-yl)-3-azabicyclo[3.1.0]hexan-6-yl)acetate (60 mg, 0.16 mmol) was dissolved in MeOH/THF/$H_2O$ (3/3/3 mL), added with NaOH (26 mg, 0.65 mmol), and reacted at 25°C for 3 hours. The pH was adjusted to be 5 with 1N hydrochloric acid, concentrated, and then the solution was subjected to column chromatography (ACN/$H_2O$=40: 60) to obtain compound of formula (1) (38 mg, 65.8%).

Molecular formula: $C_{18}H_{22}F_2N_4O_2$, molecular weight: 364.4, and LC-MS (M/e): 365.0(M+H$^+$)

$^1$HNMR (400MHz, MeOD): $\delta$: 4.38-4.43 (m, 1H), 3.98-4.05 (m, 3H), 3.84-3.91 (m, 1H), 3.61-3.68 (m, 2H), 3.04-3.09 (m, 2H), 2.31-2.48 (m, 3H), 2.30 (d, *J*=7.2 Hz, 2H), 1.90-1.96 (m, 1H), 1.56 (s, 2H), 1.48 (d, *J*=6.4 Hz, 3H), 0.85-0.89 (m, 1H).

Method II:

Water (150 ml), lithium hydroxide monohydrate (33.3 g, 0.792 mol) and methanol (1.35 L) were added into a 2L three-necked flask, stirred and diluted, and then added with 2-((1R,5S,6R)-3-(7,7-difluoro-2-((S)-2-methylazetidin-1-yl)-6,7-dihydro-5H-cyclopenta [d]pyrimidin-4-yl)-3-azabicyclo[3.1.0]hexan-6-yl)methyl acetate (150 g, 0.396mol), stirred and dispersed, heated to 35°C, and reacted for 2-3 hours. The solvent was removed by rotary evaporation and concentration, and the residue was dissolved by adding water (1,500 ml) and stirring, and then extracted by adding dichloromethane (200 ml×2). The residual solvent was removed by rotary evaporation of an obtained water phase at 40°C, and the pH was adjusted to be 4.4-5.0 with 2N hydrochloric acid to precipitate a large number of solids, which were

filtered, rinsed with water (2 L) and aired to obtain 141.3 g of solids, with a yield of 97.8%.
$^1$HNMR (400MHz, DMSO): $\delta$: 12.08 (s, br, 1H), 4.24-4.29 (m, 1H), 3.75-3.88 (m, 4H), 3.59 (s, 2H), 2.99-3.02 (m, 2H), 2.20-2.38 (m, 3H), 2.21 (d, *J*=7.2 Hz, 2H), 1.86-1.88 (m, 1H), 1.52 (s, 2H), 1.40 (d, *J*=6.0 Hz, 3H), 0.74-0.77(m, 1H).

**Example 1: Preparation of crystal form A**

Method I:

20.8 g of the solids in the method II of step (12) of the above preparation example was added into a 500 ml three-necked round-bottom flask, added with 210 ml of ethyl acetate, and stirred. Then, the system was heated to a material liquid temperature of 70°C, and clarified. Solids were completely dissolved, and oil bath heating was closed. The system was naturally cooled to 10-25°C, filtered and aired to obtain 12.6 g of solids, with a yield of 60.6%.

Method II:

10.0 g of the solids in the method II of step (12) of the above preparation example was added into a 250 ml three-necked round-bottom flask, added with 100 ml of isopropyl acetate, and stirred. Then, the system was heated to a material liquid temperature of 89°C, refluxed and clarified. Solids were completely dissolved, and the system was slowly cooled to be below 35°C. Oil bath heating was stopped. 50 ml of isopropyl ether was dropwise added at 20-30°C, then the system was continuously stirred for 16 hours, filtered when T=11°C, and dried at 50°C to obtain 8.12 g of solids.

**Example 2: Preparation of crystal form B**

22.6 g of the solids in the method II of step (12) of the above preparation example was added into a 500 ml three-necked round-bottom flask, added with 230 ml of ethanol, stirred and dispersed, and then heated. The system was clarified when T=63°C, and solids were dissolved. The system was transferred to an air bath of 27.6°C, stirred, cooled and crystallized. A large amount of white solid was precipitated when T=29°C, and the system was continuously stirred for 2.5 hours and filtered. 12.2 g of solids was obtained by airing, with a yield of 54.0%.

**Example 3: Preparation of crystal form C**

15.0 g of the solids in the method II of step (12) of the above preparation example was added into a 250ml three-necked flask, added with 75 ml of methanol, transferred to an oil bath of 60°C, and then heated and dissolved. The system was dissolved when T=55°C, the heating was stopped to slowly cool for 18 hours, and then the system was filtered. Materials were aired in a ventilated place for 2 hours, transferred to a 125°C oven for crystallization for 2 hours, and taken out after 2 hours to obtain 10.7 g of solids, with a yield of 71.3%.

**Example 4: Preparation of crystal form D**

According to the method II of step (12) of the above preparation example, 200.07 g of 2-((1R,5S,6R)-3-(7,7-difluoro-2-((S)-2-methylazetidin-1-yl)-6,7-dihydro-5H-cyclopenta [d]pyrimidin-4-yl)-3-azabicyclo[3.1.0]hexan-6-yl)methyl acetate was added to obtain a wet product, added with ethanol (1,800 ml), then added with water (3,600 ml) after dissolution. Solids were precipitated, filtered and aired to obtain solids (184.3 g). 5 g of the dry solids was added into a 100ml single-necked flask, added with 50 ml of isopropyl ether, and then heated and refluxed for 19 hours. 4.69 g of solids was obtained by filtering and airing, with a yield of 93.8%.

**Example 5: Preparation of potassium salt of compound of formula (1)**

Method I:

0.46 g (8.2 mmol) of potassium hydroxide was added into a 50ml single-necked round-bottom flask, and added with 15 ml of ethanol to dissolve solids by stirring. After the system was clarified, 3.0 g (8.2 mmol) of the compound of formula (1) was added, the system was instantly clarified, and solids were gradually precipitated, stirred for 16 hours, filtered and air to obtain 0.48 g of solids, which were identified as a crystal form I of the potassium salt by XRD.

Method II:

Potassium bicarbonate (0.27 g) or potassium carbonate (0.19 g) was respectively placed into a container, and

added with 10 ml of ethanol. The mixture was stirred at a temperature of 25°C and a stirring rate of 500 rpm. 1.0 g of the compound of formula (1) was added into the mixture, and then subjected to suction filtration after 6 hours. The obtained product was placed in a vacuum drying oven of 50°C, dried for 1 hour, and detected as a crystal form II of the potassium salt by XRD.

Method III:

3.02 g of the compound of formula (1) was placed in a container, added with 0.6656 g of potassium carbonate and 60 mL of ethanol, stirred at a temperature of 25°C, for 6 hours, and then subjected to suction filtration after 6 hours. The obtained product was dried for one day at normal temperature, and was detected as a crystal form II of the potassium salt by XRD.

**Example 6: Preparation of sodium salt of compound of formula (1)**

Method I:

0.33 g (8.2 mmol) of sodium hydroxide was added into a 50ml single-necked round-bottom flask, and added with 15 ml of ethanol to dissolve solids by stirring. After the system was clarified, 3.0 g (8.2 mmol) of the compound of formula (1) was added, the system was instantly clarified, and solids were gradually precipitated in 15 minutes. The system became viscous and could not be stirred. After adding 15 ml of ethanol, the system was stirred for 16 hours, then filtered and aired to obtain 1.25 g of solids.

Method II:

0.23 g of sodium bicarbonate was placed in a container, and added with 15 ml of acetone/acetonitrile/butyl acetate. The mixture was stirred at a temperature of 25°C and a stirring rate of 500 rpm. 0.5 g of the compound of formula (1) was added into the mixture, and then subjected to suction filtration after 12 hours. The obtained product was placed in a vacuum drying oven of 50°C and dried for 1 hour.

Method III:

4.03 g of the compound of formula (1) was placed in a container, added with 0.916 g of sodium bicarbonate and then added with 36 mL of isobutanol, stirred at a temperature of 25°C, and then filtered after 7 hours. The obtained product was dried for one day in a 50°C oven to obtain a sodium salt, which was detected as a crystal form I of the sodium salt by XRD.

**Example 7: Preparation of lithium salt of compound of formula (1)**

0.57 g (13.7 mmol) of lithium hydroxide monohydrate was added into a 100ml single-necked flask, added with 50 ml of ethanol, stirred and dissolved. After 25 minutes, solid particles were dissolved, and the ethanol system was pale white. 5.0 g (13.7 mmol) of the compound of formula (1) was added into a single-necked flask, and the system was milky and turbid, and then quickly became clear at a speed visible to naked eye. After stirring for 2.5 hours, the system became viscous and could not be stirred. The viscosity of the system was not obviously improved when the system was refluxed in an oil bath of 80°C. The system could be stirred normally by adding 50 ml of ethanol and replacing a 250 ml single-necked flask. After refluxing and stirring for 1 hour, the heating was stopped, and the system was stirred for 1 hour and filtered. 2.95 g of solids was obtained by airing.

**Example 8: Preparation of magnesium salt of compound of formula (1)**

Method I:

5.0 g (13.7 mmol) of the compound of formula (1) was added into a 250 ml single-necked flask, added with 50 ml of ethanol and transferred to an oil bath of 60°C.The material liquid system was clarified immediately, and added with 0.40 g (6.9 mmol) of magnesium hydroxide to keep the temperature and stir. The system was milky and turbid. After stirring for 2 hours, the turbidity of the system was reduced, but was not clarified yet. The heating was stopped, and the system was continuously stirred for 19 hours, filtered, and then blow-dried at 60°C for 2 hours to obtain 0.73 g of products.

Method II:

0.12 g of magnesium carbonate was placed in a container, added with 6 ml of ethanol, and then added with 2 ml of formic acid. The mixture was stirred at a temperature of 25°C and a stirring rate of 500 rpm. 1.0 g of the compound of formula (1) was added into the mixture, and then subjected to suction filtration after 5 hours. The obtained product was placed in a vacuum drying oven of 50°C, dried for 1 hour, and detected as a crystal form of the magnesium salt by XRD.

**Example 9: Preparation of calcium salt of compound of formula (1)**

Method I:

5.0 g (13.7 mmol) of the compound of formula (1) was added into a 250 ml single-necked flask, added with 50 ml of ethanol and transferred to an oil bath of 60°C. The material liquid system was clarified immediately, and added with 0.51 g (6.9 mmol) of calcium hydroxide to keep the temperature and stir. The system was milky and turbid. After stirring for 2 hours, the turbidity of the system remained unchanged. The heating was stopped, and the system was continuously stirred for 19 hours, filtered, and then blow-dried at 60°C for 2 hours to obtain 3.78 g of products.

Method II:

0.14 g of calcium carbonate was placed in a container, added with 6 ml of ethanol, and then added with 2 ml of formic acid. The mixture was stirred at a temperature of 25°C and a stirring rate of 500 rpm. 1.0 g of the compound of formula (1) was added into the mixture, and then subjected to suction filtration after 5 hours. The obtained product was placed in a vacuum drying oven of 50°C, dried for 1 hour, and detected as a crystal form I of the calcium salt by XRD.

Method III:

2.0 g of the compound of formula (1) was placed in a crystallizer, added with 30 ml of ethanol, and mixed evenly. 0.2 g of calcium hydroxide was placed in a container, added with 15 ml of water, and mixed evenly. Then, the suspension was poured into the crystallizer. The mixture was stirred at a temperature of 25°C. The temperature was cooled to 10°C in 4 hours, and then the mixture was subjected to suction filtration. The obtained product was placed under normal temperature, dried for 1 day, and detected as a crystal form II of the calcium salt by XRD.

**X-ray powder diffraction determination, differential scanning calorimetry determination and thermogravimetric analysis were carried out on the crystal forms prepared by the methods listed in Example 1, Example 2, Example 3, Example 4 and the method I of Example 5.**

**X-ray powder diffraction determination**

The crystal structures of the present invention were not limited to the crystal structures that provide X-ray powder diffraction patterns exactly the same as the X-ray powder diffraction patterns drawn in the drawings disclosed in the application, and any crystal structure that is basically the same as those disclosed in the drawings is included in the scope of the present invention.

Conditions for X-ray powder diffraction determination:

X-ray reflection parameters: Cu, K$\alpha$; entrance slit: 0.6 mm; divergence slit: 8 mm; and scan mode: continuous; and scan range: 3.0-45.0°; sampling step: 0.02°; scanning time per step: 0.3 s; and detector angle: 2.0°.

A crystal form A had characteristic peaks at 2$\theta$ positions (°) of 8.0±0.2°, 9.4±0.2°, 10.5±0.2°, 12.7±0.2°, 19.5±0.2°, and 21.2±0.2° in an X-ray powder diffraction pattern by using Cu-K$\alpha$ radiation; also had characteristic peaks at positions of 10.2±0.2°, 13.6±0.2°, 14.2±0.2°, 15.8±0.2°, 17.2±0.2°, and 25.3±0.2°; and also had characteristic peaks at positions of 6.9±0.2°, 15.1±0.2°, 16.6±0.2°, 18.9±0.2°, 22.0±0.2°, and 23.1±0.2°.

The X-ray powder diffraction pattern of the crystal form A of the compound of formula (1) was shown in FIG. 1. The crystal form had peaks at the following diffraction angle 2$\theta$ (°) of 6.9±0.2°, 8.0±0.2°, 9.4±0.2°, 10.2±0.2°, 10.5±0.2°, 12.7±0.2°, 13.6±0.2°, 14.2±0.2°, 15.1±0.2°, 15.8±0.2°, 16.6±0.2°, 17.2±0.2°, 18.9±0.2°, 19.5±0.2°, 21.2±0.2°, 22.0±0.2°, 23.1±0.2°, and 25.3±0.2°.

A crystal form B had characteristic peaks at 2$\theta$ (°) positions of 6.4±0.2°, 10.0±0.2°, 12.7±0.2°, 15.5±0.2°, 17.6±0.2°, and 22.0±0.2° in an X-ray powder diffraction pattern by using Cu-K$\alpha$ radiation; and also had characteristic peaks at positions of 8.3±0.2°, 16.5±0.2°, 17.7±0.2°, and 20.6±0.2°.

The X-ray powder diffraction pattern of the crystal form B of the compound of formula (1) was shown in FIG.

4. The crystal form had peaks at the following diffraction angle 2$\theta$ (°) of 6.4±0.2°, 8.3±0.2°, 10.0±0.2°, 12.7±0.2°, 15.5±0.2°, 16.5±0.2°, 17.6±0.2°, 17.7±0.2°, 20.6±0.2°, and 22.0±0.2°.

A crystal form C had characteristic peaks at 2$\theta$ positions (°) of 8.5±0.2°, 9.9±0.2°, 10.7±0.2°, and 18.5±0.2° in an X-ray powder diffraction pattern by using Cu-K$\alpha$ radiation; also had characteristic peaks at positions of 13.0±0.2°, 13.4±0.2°, 13.8±0.2°, and 16.8±0.2°; and also had characteristic peaks at positions of 19.2±0.2°, 20.7±0.2°, 21.1±0.2°, and 22.1±0.2°.

The X-ray powder diffraction pattern of the crystal form C of the compound of formula (1) was shown in FIG. 7. The crystal form had peaks at the following diffraction angle 2$\theta$ (°) of 8.5±0.2°, 9.9±0.2°, 10.7±0.2°, 13.0±0.2°, 13.4±0.2°, 13.8±0.2°, 16.8±0.2°, 18.5±0.2°, 19.2±0.2°, 20.7±0.2°, 21.1±0.2°, and 22.1±0.2°.

A crystal form D had characteristic peaks at 2$\theta$ positions (°) of 6.4±0.2°, 10.0±0.2°, 12.7±0.2°, 17.7±0.2°, 18.6±0.2°, and 25.5±0.2° in an X-ray powder diffraction pattern by using Cu-K$\alpha$ radiation; also had characteristic peaks at positions of 14.9±0.2°, 15.6±0.2°, and 20.6±0.2°; and also had characteristic peaks at positions of 8.3±0.2°, 16.5±0.2°, 19.1±0.2°, and 20.2±0.2°.

The X-ray powder diffraction pattern of the crystal form D of the compound of formula (1) was shown in FIG. 10. The crystal form had peaks at the following diffraction angle 2$\theta$ (°) of 6.4±0.2°, 8.3±0.2°, 10.0±0.2°, 12.7±0.2°, 14.9±0.2°, 15.6±0.2°, 16.5±0.2°, 17.7±0.2°, 18.6±0.2°, 19.1±0.2°, 20.2±0.2°, 20.6±0.2°, and 25.5±0.2°.

The crystal form I of the potassium salt had characteristic peaks at 2$\theta$ positions (°) of 4.8±0.2°, 5.9±0.2°, 7.1±0.2°, 8.3±0.2°, 11.8±0.2°, and 15.5±0.2° in an X-ray powder diffraction pattern by using Cu-K$\alpha$ radiation; also had characteristic peaks at positions of 14.6±0.2°, 16.6±0.2°, 17.9±0.2°, 18.8±0.2°, 19.6±0.2°, and 21.5±0.2°; and also had characteristic peaks at positions of 13.0±0.2°, 14.2±0.2°, 23.3±0.2°, and 25.0±0.2°.

The X-ray powder diffraction pattern of the crystal form I of the potassium salt of the compound of formula (1) was shown in FIG. 13. The crystal form had peaks at the following diffraction angle 2$\theta$(°) of 4.8±0.2°, 5.9±0.2°, 7.1±0.2°, 8.3±0.2°, 11.8±0.2°, 13.0±0.2°, 14.2±0.2°, 14.6±0.2°, 15.5±0.2°, 16.6±0.2°, 17.9±0.2°, 18.8±0.2°, 19.6±0.2°, 21.5±0.2°, 23.3±0.2°, and 25.0±0.2°.

**Differential scanning calorimetry**

Solid-state thermal properties of the crystal form A, the crystal form B, the crystal form C, the crystal form D and the crystal form I of the potassium salt of the compound of formula (1) were studied by differential scanning calorimetry (DSC).

Determination conditions: purging with nitrogen at 50 ml/min, collecting data at a heating rate of 10°C/min between 25°C and 230°C, and plotting with an endothermic peak downward.

The crystal form A of the compound of formula (1) had an endothermic peak in the range of 167°C to 173°C, and the differential scanning calorimetry thermogram thereof was basically as shown in FIG. 2.

The crystal form B of the compound of formula (1) had an endothermic peak in the range of 167°C to 173°C, and the differential scanning calorimetry thermogram thereof was basically as shown in FIG. 5.

The crystal form C of the compound of formula (1) had an endothermic peak in the range of 170°C to 175°C, and the differential scanning calorimetry thermogram thereof was basically as shown in FIG. 8.

The crystal form D of the compound of formula (1) had an endothermic peak in the range of 165°C to 170°C, and the differential scanning calorimetry thermogram thereof was basically as shown in FIG. 11.

The crystal form I of the potassium salt of the compound of formula (1) had an endothermic peak in the range of 174°C to 180°C, and the differential scanning calorimetry thermogram thereof was basically as shown in FIG. 14.

In DSC determination, according to the measurement parameters and heating rate, the actually measured starting temperature and maximum temperature had some variability.

**Thermogravimetric analysis**

Test conditions: purging with nitrogen at 60 ml/min, and collecting data at a heating rate of 10°C/min between room temperature and 400°C.

The crystal form A of the compound of formula (1) had no weight loss below 200°C, and a TG curve thereof was displayed in FIG. 3.

The crystal form B of the compound of formula (1) had no weight loss below 200°C, and a TG curve thereof was displayed in FIG. 6.

The crystal form C of the compound of formula (1) had no weight loss below 200°C, and a TG curve thereof was displayed in FIG. 9.

The crystal form D of the compound of formula (1) had no weight loss below 200°C, and a TG curve thereof was displayed in FIG. 12.

The crystal form I of the potassium salt of the compound of formula (1) had weight loss below 100°C, which is

adsorbed water, and a TG curve thereof was displayed in FIG. 15.

**X-ray powder diffraction determination, differential scanning calorimetry determination and thermogravimetric analysis were carried out on the crystal form II of the potassium salt prepared by the methods listed in the method II and the method III of Example 5.**

**X-ray powder diffraction determination**

Test conditions: D/max - 2500 diffractometer (Rigaku, Japan);

$2\theta$ ranging from 2° to 40°, and a scanning speed of 8°/min; and
a voltage of 40 KV, and a current of 100 mA.

The crystal form II of the potassium salt had characteristic peaks at $2\theta$ positions (°) of 4.7±0.2°, 6.5±0.2°, 7.9±0.2°, 10.3±0.2°, 11.0±0.2°, and 12.1±0.2° in an X-ray powder diffraction pattern by using Cu-K$\alpha$ radiation; also had characteristic peaks at positions of 5.1±0.2°, 13.0±0.2°, 20.1±0.2°, 21.4±0.2°, 22.4±0.2°, and 24.1±0.2°; and also had characteristic peaks at positions of 8.3±0.2°, 17.6±0.2°, 19.5±0.2°, 20.6±0.2°, 21.1±0.2°, and 24.5±0.2°.

The X-ray powder diffraction pattern of the crystal form II of the potassium salt of the compound of formula (1) was shown in FIG. 16. The crystal form had peaks at the following diffraction angle $2\theta$(°) of 4.7±0.2°, 5.1±0.2°, 6.5±0.2°, 7.9±0.2°, 8.3±0.2°, 10.3±0.2°, 11.0±0.2°, 12.1±0.2°, 13.0±0.2°, 17.6±0.2°, 19.5±0.2°, 20.1±0.2°, 20.6±0.2°, 21.1±0.2°, 21.4±0.2°, 22.4±0.2°, 24.1±0.2°, and 24.5±0.2°.

Differential scanning calorimetry

Solid-state thermal properties of the crystal form II of the potassium salt of the compound of formula (1) were studied by differential scanning calorimetry (DSC).

Determination conditions: Mettler Toledo DSC, Greifensee, Switzerland; and
nitrogen protection, a temperature range of 303.15 K to 473.15 K, and a heating rate of 10K/min or 5K/min.

The crystal form II of the potassium salt of the compound of formula (1) had an endothermic peak in the range of 155°C to 165°C, and the differential scanning calorimetry thermogram thereof was basically as shown in FIG. 17A.

In DSC determination, according to the measurement parameters and heating rate, the actually measured starting temperature and maximum temperature had some variability.

**Thermogravimetric analysis**

Test conditions: purging with nitrogen at 60 ml/min, and collecting data at a heating rate of 10°C/min between room temperature and 300°C.

The crystal form II of the potassium salt of the compound of formula (1) had a weight loss of 8.5% below 200°C, and a TG curve thereof was displayed in FIG. 17B.

**X-ray powder diffraction determination and differential scanning calorimetry determination were carried out on the crystal form of the magnesium salt prepared by the method listed in the method II of Example 8.**

X-ray powder diffraction determination

Test conditions: D/max - 2500 diffractometer (Rigaku, Japan);

$2\theta$ ranging from 2° to 40°, and a scanning speed of 8°/min; and
a voltage of 40 KV, and a current of 100 mA.

The crystal form I of the magnesium salt had characteristic peaks at $2\theta$ positions (°) of 18.8±0.2°, 23.8±0.2°, 26.3±0.2°, and 29.5±0.2° in an X-ray powder diffraction pattern by using Cu-K$\alpha$ radiation; and also had characteristic peaks at positions of 23.0±0.2°, 25.9±0.2°, 31.5±0.2°, 32.0±0.2°, 38.1±0.2°, and 39.0±0.2°.

The X-ray powder diffraction pattern of the crystal form of the magnesium salt of the compound of formula (1) was shown in FIG. 18. The crystal form had peaks at the following diffraction angle $2\theta$ (°) of 18.8±0.2°, 23.0±0.2°, 23.8±0.2°, 25.9±0.2°, 26.3±0.2°, 29.5±0.2°, 31.5±0.2°, 32.0±0.2°, 38.1±0.2°, and 39.0±0.2°.

**Differential scanning calorimetry**

Solid-state thermal properties of the crystal form of the magnesium salt of the compound of formula (1) were

studied by differential scanning calorimetry (DSC).

Determination conditions: Mettler Toledo DSC, Greifensee, Switzerland; and nitrogen protection, a temperature range of 303.15 K to 473.15 K, and a heating rate of 10K/min or 5K/min.

The crystal form of the magnesium salt of the compound of formula (1) had an endothermic peak in the range of 165°C to 180°C, and the differential scanning calorimetry thermogram thereof was basically as shown in FIG. 19.

In DSC determination, according to the measurement parameters and heating rate, the actually measured starting temperature and maximum temperature had some variability.

**X-ray powder diffraction determination was carried out on the crystal form I of the calcium salt prepared by the method listed in the method II of Example 9.**

**X-ray powder diffraction determination**

Test conditions: D/max - 2500 diffractometer (Rigaku, Japan);

$2\theta$ ranging from 2° to 40°, and a scanning speed of 8°/min; and
a voltage of 40 KV, and a current of 100 mA.

The crystal form I of the calcium salt had characteristic peaks at $2\theta$ positions (°) of 15.8±0.2°, 16.1±0.2°, 26.3±0.2°, 28.0±0.2°, 30.2±0.2°, and 31.2±0.2° in an X-ray powder diffraction pattern by using Cu-Kα radiation, and a X-ray powder diffraction pattern thereof was as shown in FIG. 20.

**X-ray powder diffraction determination, differential scanning calorimetry determination and thermogravimetric analysis were carried out on the crystal form II of the calcium salt prepared by the method listed in the method III of Example 9.**

**X-ray powder diffraction determination**

Conditions for X-ray powder diffraction determination:

X-ray reflection parameters: Cu, Kα; entrance slit: 0.6 mm; divergence slit: 8 mm; and scan mode: continuous; and scan range: 3.0-45.0°; sampling step: 0.02°; scanning time per step: 0.3 s; and detector angle: 2.0°.

The crystal form II of the calcium salt had characteristic peaks at $2\theta$ positions (°) of 3.9±0.2°, 6.0±0.2°, 8.1±0.2°, 11.1±0.2°, 16.0±0.2°, 17.4±0.2° in an X-ray powder diffraction pattern, and 19.0±0.2° by using Cu-Kα radiation, and a X-ray powder diffraction pattern thereof was as shown in FIG. 21.

**Differential scanning calorimetry determination**

Solid-state thermal properties of the crystal form II of the calcium salt of the compound of formula (1) were studied by differential scanning calorimetry (DSC).

Determination conditions: purging with nitrogen at 50 ml/min, and collecting data at a heating rate of 10°C/min between 25°C and 320°C.

The crystal form II of the calcium salt of the compound of formula (1) had a small endothermic peak at about 80°C and exothermic peaks at about 200°C and 280°C. Comprehensive experimental observation showed that the calcium salt turned black at 200°C, so the calcium salt melt at 200°C and then further decomposed at 280°C. A differential scanning calorimetry thermogram of the crystal form II of the calcium salt was basically as shown in FIG. 22.

In DSC determination, according to the measurement parameters and heating rate, the actually measured starting temperature and maximum temperature had some variability.

**Thermogravimetric analysis**

Test conditions: purging with nitrogen at 60 ml/min, and collecting data at a heating rate of 10°C/min between room temperature and 300°C.

The crystal form II of the calcium salt of the compound of formula (1) had weight loss below 250°C, and a TG curve thereof was displayed in FIG. 23.

**X-ray powder diffraction determination, differential scanning calorimetry determination and thermogravimetric analysis were carried out on the crystal form I of the sodium salt prepared by the method listed in the method III of Example 6.**

### X-ray powder diffraction determination

Conditions for X-ray powder diffraction determination:

X-ray reflection parameters: Cu, Kα; entrance slit: 0.6 mm; divergence slit: 8 mm; and scan mode: continuous; and scan range: 3.0-45.0°; sampling step: 0.02°; scanning time per step: 0.3 s; and detector angle: 2.0°.

The crystal form I of the sodium salt had characteristic peaks at 2$\theta$ positions (°) of 3.9±0.2°, 4.5±0.2°, 7.8±0.2°, 9.1±0.2°, 11.8±0.2°, 12.7±0.2° in an X-ray powder diffraction pattern by using Cu-Kα radiation; also had characteristic peaks at 8.5±0.2°, 10.5±0.2°, 14.2±0.2°, 16.8±0.2°, and 17.9±0.2°; and also had characteristic peaks at 18.6±0.2°, 20.4±0.2°, 30.5±0.2°, and 34.7±0.2°.

The X-ray powder diffraction pattern of the crystal form I of the sodium salt of the compound of formula (1) was shown in FIG. 24. The crystal form had peaks at the following diffraction angle 2$\theta$ (°) of 3.9±0.2°, 4.5±0.2°, 7.8±0.2°, 8.5±0.2°, 9.1±0.2°, 10.5±0.2°, 11.8±0.2°, 12.7±0.2°, 14.2±0.2°, 16.8±0.2°, 17.9±0.2°, 18.6±0.2°, 20.4±0.2°, 30.5±0.2°, and 34.7±0.2°.

### Differential scanning calorimetry determination

Solid-state thermal properties of the crystal form I of the sodium salt of the compound of formula (1) were studied by differential scanning calorimetry (DSC).

Determination conditions: purging with nitrogen at 50 ml/min, and collecting data at a heating rate of 10°C/min between 25°C and 220°C.

A differential scanning calorimetry thermogram of the crystal form I of the sodium salt of the compound of formula (1) was basically as shown in FIG. 25.

In DSC determination, according to the measurement parameters and heating rate, the actually measured starting temperature and maximum temperature had some variability.

### Thermogravimetric analysis

Test conditions: purging with nitrogen at 60 ml/min, and collecting data at a heating rate of 10°C/min between room temperature and 300°C.

The crystal form I of the sodium salt of the compound of formula (1) had weight loss below 200°C, and a TG curve thereof was displayed in FIG. 26.

The crystal structures of the present invention were not limited to the crystal structures that provide X-ray powder diffraction patterns exactly the same as the X-ray powder diffraction patterns drawn in the drawings disclosed in the present application, and any crystal structure that is basically the same as those disclosed in the drawings is included in the scope of the present invention.

### Example 10 Investigation on the stability of the crystal form A of the compound of formula (1)

### 1. Sample for testing

The crystal form A of the compound of formula (1) prepared according to the method of Example 1.

### 2. Investigation conditions

2.1 Investigation conditions for the crystal form A of the compound of formula (1):

placed at 105°C for 5 days, opened, and sampled for testing on day 5;
placed at 60°C for 10 days, opened/closed, and sampled for testing on day 10;
placed at 25°C and RH 92.5% for 10 days, opened/closed, and sampled for testing on day 10;
placed at 40°C and RH 75% for one month, opened/closed, and sampled for testing on the last day of the month; and
under illumination, employing a closed way (polyethylene bag + composite film bag) to meet illumination requirements, and sampled for testing.

Illumination requirements: ICH Q1B Stability Testing: Photostability Testing of New Drug Substances and Products.

### 3. Determination method

**Determination of related substances:** determined according to 0512 HPLC in General Principle (Volume IV) of Chinese Pharmacopoeia (2015 Version).

**Moisture determination:** determined according to 2 Coulometric Titration in the First Method (Karl Fischer titration) of 0832 Moisture Determination Method in General Principle (Volume IV) of Chinese Pharmacopoeia (2015 Version).

**XRD determination:** determined according to the Second Method-Powder X-ray Diffraction Method of 0451 X-ray Diffraction Method in General Principle (Volume IV) of Chinese Pharmacopoeia (2015 Version).

**DSC determination:** precisely weighing an appropriate amount of sample in a Standard disc, covering the Standard disc, placing the Standard disc in a sample disc position in a DSC furnace, placing a blank disc of the same type in a reference position, and carrying out the following temperature rise program:

1. Equilibrate at 25°C; and
2. Ramp 10°C to 200°C.

### 4. Testing result

Stability data of the crystal form A of the compound of formula (1) was shown in the following table.

**Table 1 Stability investigation result of the crystal form A of the compound of formula (1)**

| Sampl e | Placement condition | Trait | Total related % | Mois ture % | XRD | DSC |
|---|---|---|---|---|---|---|
| Crysta 1 form A | 0 day | Off-white power | 0.18 | 0.53 | As control | 170.33 °C |
| | 105°C, opened, 5 days | Off-white power | 0.30 | 0.54 | Same as day 0 | 170.65 °C |
| | 60°C, opened, 10 days | Off-white power | 0.25 | 0.54 | Same as day 0 | 170.26 °C |
| | 60°C, closed, 10 days | Off-white power | 0.24 | 0.61 | Same as day 0 | 170.34 °C |
| | 25°C, RH 92.5%, opened, 10 days | Off-white power | 0.24 | 0.54 | Same as day 0 | 170.23 °C |
| | 25°C, RH 92.5%, closed, 10 days | Off-white power | 0.24 | 0.54 | Same as day 0 | 170.42 °C |
| | 40°C, RH 75%, opened, 1M | Off-white power | 0.24 | 0.46 | Same as day 0 | 170.32 °C |
| | 40°C, RH 75%, closed, 1M | Off-white power | 0.22 | 0.57 | Same as day 0 | 170.38 °C |
| | Avoid light for 18 days | Off-white power | 0.19 | 0.47 | Same as day 0 | 170.20 °C |

### 5. Testing conclusion

The total related substances of the crystal form A of the compound of formula (1) were slightly increased at a high temperature of 105°C and under the opened/closed conditions of 60°C, 25°C and RH 92.5%, as well as 40°C and RH 75%, and other inspection items such as moisture, XRD, and DSC stability were good. It indicated that the crystal form A of the compound of formula (1) was relatively stable under the above conditions, which was convenient for the preparation, transportation and storage of drugs and more conducive to ensuring the effectiveness and safety of drug use.

**Example 11 Investigation on the stability of the crystal form B of the compound of formula (1)**

**1. Sample for testing**

The crystal form B of the compound of formula (1) prepared according to the method of Example 2.

**2. Investigation conditions**

2.1 Investigation conditions for the crystal form B of the compound of formula (1):

placed at 105°C for 5 days, opened, and sampled for testing on day 5;
placed at 60°C for 10 days, opened/closed, and sampled for testing on day 10;
placed at 25°C and RH 92.5% for 10 days, opened/closed, and sampled for testing on day 10;
placed at 40°C and RH 75% for one month, opened/closed, and sampled for testing on the last day of the month; and
under illumination, employing a closed way (polyethylene bag + composite film bag) to meet illumination requirements, and sampled for testing.

Illumination requirements: ICH Q1B Stability Testing: Photostability Testing of New Drug Substances and Products.

**3. Determination method**

**Determination of related substances:** determined according to 0512 HPLC in General Principle (Volume IV) of Chinese Pharmacopoeia (2015 Version).

**Moisture determination:** determined according to 2 Coulometric Titration in the First Method (Karl Fischer titration) of 0832 Moisture Determination Method in General Principle (Volume IV) of Chinese Pharmacopoeia (2015 Version).

**XRD determination:** determined according to the Second Method-Powder X-ray Diffraction Method of 0451 X-ray Diffraction Method in General Principle (Volume IV) of Chinese Pharmacopoeia (2015 Version).

**DSC determination:** precisely weighing an appropriate amount of sample in a Standard disc, covering the Standard disc, placing the Standard disc in a sample disc position in a DSC furnace, placing a blank disc of the same type in a reference position, and carrying out the following temperature rise program:

1. Equilibrate at 25°C; and
2. Ramp 10°C to 200°C.

**4. Testing result**

Stability data of the crystal form B of the compound of formula (1) was shown in the following table.

**Table 2 Stability investigation result of the crystal form B of the compound of formula (1)**

| Sampl e | Placement condition | Trait | Total relate d % | Moist ure % | XRD | DSC |
|---|---|---|---|---|---|---|
| Crystal form B | 0 day | White powder | 0.08 | 0.17 | As control | 169.28 °C |
| | 105°C, opened, 5 days | White powder | 0.22 | 0.28 | Same as day 0 | 169.53 °C |
| | 60°C, opened, 10 days | White powder | 0.13 | 0.26 | Same as day 0 | 169.27 °C |
| | 60°C, closed, 10 days | White powder | 0.11 | 0.21 | Same as day 0 | 169.35 °C |
| | 25°C, RH 92.5%, opened, 10 days | White powder | 0.10 | 0.26 | Same as day 0 | 169.31 °C |
| | 25°C, RH 92.5%, closed, 10 days | White powder | 0.10 | 0.35 | Same as day 0 | 169.47 °C |
| | 40°C, RH 75%, opened, 1M | White powder | 0.09 | 0.35 | Same as day 0 | 169.43 °C |
| | 40°C, RH 75%, closed, 1M | White powder | 0.08 | 0.39 | Same as day 0 | 169.57 °C |
| | Avoid light for 18 days | White powder | 0.07 | 0.17 | Same as day 0 | 169.15 °C |

**5. Testing conclusion**

The total related substances of the crystal form B of the compound of formula (1) were slightly increased at a high temperature of 105°C and under the opened/closed conditions of 60°C, 25°C and RH 92.5%, and other inspection items such as moisture, XRD, and DSC stability were good. When placed for one month under the condition of 40°C and RH75 %, the related substances, and the moisture, XRD, and DSC stability were all good. It indicated that the crystal form B of the compound of formula (1) was relatively stable under the above conditions, which was convenient for the preparation, transportation and storage of drugs and more conducive to ensuring the effectiveness and safety of drug use.

**Example 12 Investigation on the stability of the crystal form C of the compound of formula (1)**

**1. Sample for testing**

The crystal form C of the compound of formula (1) prepared according to the method of Example 3.

**2. Investigation conditions**

2.1 Investigation conditions for the crystal form C of the compound of formula (1):

placed at 105°C for 5 days, opened, and sampled for testing on day 5;
placed at 60°C for 10 days, opened/closed, and sampled for testing on day 10;
placed at 25°C and RH 92.5% for 10 days, opened/closed, and sampled for testing on day 10;
placed at 40°C and RH 75% for one month, opened/closed, and sampled for testing on the last day of the month; and
under illumination, employing a closed way (polyethylene bag + composite film bag) to meet illumination requirements, and sampled for testing.

Illumination requirements: ICH Q1B Stability Testing: Photostability Testing of New Drug Substances and Products.

### 3. Determination method

**Determination of related substances:** determined according to 0512 HPLC in General Principle (Volume IV) of Chinese Pharmacopoeia (2015 Version).

**Moisture determination:** determined according to 2 Coulometric Titration in the First Method (Karl Fischer titration) of 0832 Moisture Determination Method in General Principle (Volume IV) of Chinese Pharmacopoeia (2015 Version).

**XRD determination:** determined according to the Second Method-Powder X-ray Diffraction Method of 0451 X-ray Diffraction Method in General Principle (Volume IV) of Chinese Pharmacopoeia (2015 Version).

**DSC determination:** precisely weighing an appropriate amount of sample in a Standard disc, covering the Standard disc, placing the Standard disc in a sample disc position in a DSC furnace, placing a blank disc of the same type in a reference position, and carrying out the following temperature rise program:

1. Equilibrate at 25°C; and 2. Ramp 10°C to 200°C.

### 4. Testing result

Stability data of the crystal form C of the compound of formula (1) was shown in the following table.

**Table 3 Stability investigation result of the crystal form C of the compound of formula (1)**

| Sample | Placement condition | Trait | Total related % | Moisture % | XRD | DSC |
|---|---|---|---|---|---|---|
| Crystal form C | 0 day | Off-white power | 1.25 | 0.24 | As control | 172.44 °C |
| | 105°C, opened, 5 days | Off-white power | 1.77 | 0.24 | Same as day 0 | 171.85 °C |
| | 60°C, opened, 10 days | Off-white power | 2.09 | 0.08 | Same as day 0 | 172.21 °C |
| | 60°C, closed, 10 days | Off-white power | 2.20 | 0.08 | Same as day 0 | 172.21 °C |
| | 25°C, RH 92.5%, opened, 10 days | Off-white power | 1.07 | 0.11 | Same as day 0 | 172.35 °C |
| | 25°C, RH 92.5%, closed, 10 days | Off-white power | 1.06 | 0.09 | Same as day 0 | 172.36 °C |
| | 40°C, RH 75%, opened, 1M | Off-white power | 1.31 | 0.13 | Same as day 0 | 172.35 °C |
| | 40°C, RH 75%, closed, 1M | Off-white power | 1.30 | 0.19 | Same as day 0 | 172.36 °C |
| | Avoid light for 18 days | Off-white power | 1.25 | 0.13 | Same as day 0 | 172.42 °C |

### 5. Testing conclusion

The total related substances of the crystal form C of the compound of formula (1) were slightly increased at a high temperature of 105°C for 5 days and 60°C for 10 days; and the related substances, content, moisture, and XRD stability were good under the opened/closed conditions of 25°C and RH 92.5% for 10 days as well as 40°C and RH 75% for one month.

## Example 13 Investigation on the stability of the crystal form D of the compound of formula (1)

### 1. Sample for testing

The crystal form D of the compound of formula (1) prepared according to the method of Example 4.

## 2. Investigation conditions

2.1 Investigation conditions for the crystal form D of the compound of formula (1):

placed at 105°C for 5 days, opened, and sampled for testing on day 5;
placed at 60°C for 10 days, opened/closed, and sampled for testing on day 10;
placed at 25°C and RH 92.5% for 10 days, opened/closed, and sampled for testing on day 10;
placed at 40°C and RH 75% for one month, opened/closed, and sampled for testing on the last day of the month; and
under illumination, employing a closed way (polyethylene bag + composite film bag) to meet illumination requirements, and sampled for testing.

Illumination requirements: ICH Q1B Stability Testing: Photostability Testing of New Drug Substances and Products.

## 3. Determination method

**Determination of related substances:** determined according to 0512 HPLC in General Principle (Volume IV) of Chinese Pharmacopoeia (2015 Version).

**Moisture determination:** determined according to 2 Coulometric Titration in the First Method (Karl Fischer titration) of 0832 Moisture Determination Method in General Principle (Volume IV) of Chinese Pharmacopoeia (2015 Version).

**XRD determination:** determined according to the Second Method-Powder X-ray Diffraction Method of 0451 X-ray Diffraction Method in General Principle (Volume IV) of Chinese Pharmacopoeia (2015 Version).

**DSC determination:** precisely weighing an appropriate amount of sample in a Standard disc, covering the Standard disc, placing the Standard disc in a sample disc position in a DSC furnace, placing a blank disc of the same type in a reference position, and carrying out the following temperature rise program:

1. Equilibrate at 25°C; and
2. Ramp 10°C to 200°C.

## 4. Testing result

Stability data of the crystal form D of the compound of formula (1) was shown in the following table.

**Table 4 Stability investigation result of the crystal form D of the compound of formula (1)**

| Sampl e | Placement condition | Trait | Total related % | Moistur e % | XRD | DSC |
|---|---|---|---|---|---|---|
| Crysta 1 form D | 0 day | Off-white power | 0.37 | 0.066 | As control | 167.79 °C |
| | 105°C, opened, 5 days | Off-white power | 0.51 | 0.045 | Same as day 0 | 167.19 °C |
| | 60°C, opened, 10 days | Off-white power | 0.41 | 0.079 | Same as day 0 | 167.41 °C |
| | 60°C, closed, 10 days | Off-white power | 0.40 | 0.089 | Same as day 0 | 167.43 °C |
| | 25°C, RH 92.5%, opened, 10 days | Off-white power | 0.38 | 0.048 | Same as day 0 | 167.51 °C |
| | 25°C, RH 92.5%, closed, 10 days | Off-white power | 0.38 | 0.092 | Same as day 0 | 167.46 °C |
| | 40°C, RH 75%, opened, 1M | Off-white power | 0.39 | 0.12 | Same as day 0 | 167.75 °C |
| | 40°C, RH 75%, closed, 1M | Off-white power | 0.38 | 0.065 | Same as day 0 | 167.77 °C |
| | Avoid light for 18 days | Off-white power | 0.38 | 0.048 | Same as day 0 | 167.49 °C |

**5. Testing conclusion**

The total related substances of the crystal form D of the compound of formula (1) were slightly increased at a high temperature of 105°C; and other inspection items such as moisture content, XRD, and DSC stability were good. The total related substances, the moisture, XRD, and DSC stability were all well under the opened/closed conditions of 40°C and RH 75% for one month as well as 60°C, 25°C and RH 92.5% for 10 days. It indicated that the crystal form D of the compound of formula (1) was relatively stable under the above conditions, which was convenient for the preparation, transportation and storage of drugs and more conducive to ensuring the effectiveness and safety of drug use.

**Example 14 Investigation on the stability of the crystal form II of the potassium salt** of the **compound** of formula **(1)**

1. **Sample for testing**

The crystal form II of the potassium salt of the compound of formula (1) prepared according to the method III of Example 5.

**2. Investigation conditions**

2.1 Investigation conditions for the crystal form II of the potassium salt of the compound of formula (1):

placed at 60°C for 10 days, packaged with a low-density polyethylene bag, and sampled for testing on day 10;
placed at 25°C and RH 92.5% for 10 days, packaged with a low-density polyethylene bag, and sampled for testing on day 10; and
placed at 40°C and RH 75% for one month, packaged with a low-density polyethylene bag, and sampled for testing on the last day of the month.

**3. Determination method**

**Determination of related substances:** determined according to 0512 HPLC in General Principle (Volume IV) of Chinese Pharmacopoeia (2015 Version).

**4. Testing result**

Stability data of the crystal form II of the potassium salt of the compound of formula (1) was shown in the following table.

**Table 5 Stability investigation result of the crystal form II of the potassium salt of the compound of formula (1)**

| Placement condition | Total related (%) | Trait | RRT 0.39 | RRT 0.57 | RRT 0.66 | RRT 1.21 |
|---|---|---|---|---|---|---|
| 0 day | 0.09 | Off-white power | 0.01 | / | 0.01 | 0.01 |
| 60°C, 10 days | 0.08 | Off-white power | 0.01 | 0.01 | 0.01 | 0.01 |
| RH 92.5%, 10 days | 0.10 | Off-white power | 0.01 | 0.01 | 0.01 | 0.02 |
| 40°C, 1M | 0.10 | Off-white power | 0.01 | 0.02 | 0.01 | 0.01 |

**5. Testing conclusion**

Under the conditions of 60°C and RH 92.5% for 10 days, and the conditions of 40°C and RH 75% for one month, the properties and related substances of the crystal form II of the potassium salt of the compound of formula (1) were basically the same as those of day 0, which showed that the crystal form II of the potassium salt of the compound of formula (1) was relatively stable under the above conditions, which was convenient for the preparation, transportation and storage of drugs and more conducive to ensuring the effectiveness and safety of drug use.

**Example 15 Investigation on the hygroscopicity of the crystal form II of the calcium salt of the compound of formula (1)**

**1. Sample for testing:** the crystal form II of the calcium salt of the compound of formula (1) prepared according to the method III of Example 9.
**2. Investigation conditions:** placed under RH 80% for one day, opened.
**3. Testing result**

The crystal form II of the calcium salt of the compound of formula (1) increased weight by 1.38% after being left at RH 80% for one day, and was slightly hygroscopic.

**Example 16 Comparative testing - Study on the stability of the amorphous form of the compound of formula (1)**

**1. Sample for testing**

The amorphous form of the compound of formula (1) prepared according to the method I of the Preparation Example.

**2. Investigation conditions**

2.1 Investigation conditions for the amorphous form of the compound of formula (1):

placed at 60°C for 10 days, opened/closed, and sampled for testing on day 10;
placed at 25°C and RH 92.5% for 10 days, opened/closed, and sampled for testing on day 10;
placed at 40°C and RH 75% for one month, opened/closed, and sampled for testing on the last day of the month; and under illumination, employing a closed way (polyethylene bag + composite film bag) to meet illumination requirements, and sampled for testing.

Illumination requirements: ICH Q1B Stability Testing: Photostability Testing of New Drug Substances and Products.

### 3. Determination method

**Determination of related substances:** determined according to 0512 HPLC in General Principle (Volume IV) of Chinese Pharmacopoeia (2015 Version).

**Moisture determination:** determined according to 2 Coulometric Titration in the First Method (Karl Fischer titration) of 0832 Moisture Determination Method in General Principle (Volume IV) of Chinese Pharmacopoeia (2015 Version).

### 4. Testing result

Stability data of the amorphous form of the compound of formula (1) was shown in the following table.

**Table 6 Stability investigation result of the amorphous form of the compound of formula (1)**

| Batch | Placement condition | Trait | Total related % | Moisture % |
|---|---|---|---|---|
| Amorphou s form | 0 day | Off-white power | 0.21 | 0.70 |
| | 60°C, opened, for 10 days | Off-white power | 4.30 | 0.56 |
| | 60°C, closed, for 10 days | Off-white power | 3.92 | 0.73 |
| | 25°C, RH 92.5%, opened, for 10 days | Off-white power | 0.38 | 3.1 |
| | 25°C, RH 92.5%, closed, for 10 days | Off-white power | 0.35 | 2.8 |
| | 40°C, RH 75%, opened, 1M | Off-white power | 2.86 | 1.9 |
| | 40°C, RH 75%, closed, 1M | Off-white power | 2.98 | 2.1 |
| | Avoid light for 18 days | Off-white power | 0.42 | 1.26 |

### 5. Testing conclusion

The total related substances of the amorphous form of the compound of formula (1) was obviously increased and the moisture stability was good after being placed for 10 days under the opened/closed high temperature condition of 60°C; the total related substances were slightly increased after being placed for 10 days under the opened/closed conditions of 25°C and RH 92.5%, and the moisture was obviously increased; the total related substances and the moisture were both obviously increased after being placed for one month under the opened/closed conditions of 40°C and RH 75%, indicating that the amorphous form of the compound of formula (1) was less stable than that of the crystal form A, the crystal form B, the crystal form C and the crystal form D under the above conditions.

### Experimental Example 1: In vitro KHK kinase inhibitory activity test of the compound of formula (1)

Sample for testing: compound of formula (1), the chemical name and preparation method of which were referred to the Preparation Example.

Control drug: PF-06835919, prepared by referring to the method in reference (US15/381,295).

Reagents:

| Reagent | Vendor | Cat No. |
|---|---|---|
| HEPES | Life Technologies | 15630-080 |
| ADP-GloTM Kinase Assay kit | Promega | V9102 |
| D-Fructose | Sigma | F2543 |
| KHK-C | Origene | TP323488 |
| Brij 35 detergent | Merck | 203728 |
| ATP | Promega | V915B |

(continued)

| Reagent | Vendor | Cat No. |
|---|---|---|
| $MgCl_2$ | Sigma | M1028 |
| KCl | Sigma | P9541 |
| DMSO | MP | 196055 |

Consumables:

| Consumables | Vendor | Cat No. |
|---|---|---|
| Topseal A | PerkinElmer | E5341 |
| 96 Well Plates | Nunc | 249944 |
| 384-Well Polypropylene microplate | Labcyte | P-05525 |
| Optiplate-384 | Perkin Elmer | 6007290 |

Instruments:

| Instrument | Vendor | Cat No. |
|---|---|---|
| Echo 550 Liquid Handler | Labcyte | Echo 550 |
| Plate reader | Perkin Elmer | Envision 2104 |
| Centrifuge | Eppendorf | 5810R |
| Multi-channel pipettes | Eppendorf/Sartorius | / |

1. Compound dilution

1) DMSO was used to prepare the compound of the present invention and a control drug to 10 mM, which was used as a testing stock solution.
2) The compound stock solution of the present invention was diluted 10-fold to 1 mM, and then the compound of the present invention was diluted in gradient 3-fold to 11 concentrations, with the highest concentration of 1 mM. The control drug stock solution was diluted 100-fold to 0.1 mM, and then the control drug was diluted in gradient 3-fold to 11 concentrations, with the highest concentration of 0.1 mM.
3) 0.1 μL of the diluted compound of the present invention and 0.1 μL of the diluted control drug were transferred by using Echo550 to a 384-well plate respectively, with two repeated wells set for each concentration, and centrifuged at 1,000 rpm for 1 min.

2. Enzyme reaction testing

1) 5 μL of KHK-C kinase working fluid was added to a 384-well plate, centrifuged at 1,000 rpm for 1 min, and then incubated at a room temperature of 25°C for 15 min.
2) 5 μL of substrate working fluid was added to the 384-well plate, a kinase reaction was started, then the substrate working fluid was centrifuged at 1,000 rpm for 1 min, and then incubated at a room temperature of 25°C for 60 min.
3) The KHK-C kinase reaction was performed at final concentrations of 1 nM KHK-C, 100 μM ATP, 200 μM D-Fructose, 50 mM HEPES, 10 mM $MgCl_2$, and 0.01% Brij35, and a final concentration of DMSO was 1%.

4) The final concentrations of the tested compounds were 10,000 nM, 3,333.33 nM, 1,111.11 nM, 370.37 nM, 123.457 nM, 41.15 nM, 13.71 nM, 4.572 nM, 1.524 nM, 0.508 nM, and 0.169 nM.
5) The final concentrations of the control drugs were 1,000 nM, 333.33 nM, 111.11 nM, 37.037 nM, 12.346 nM, 4.115 nM, 1.371 nM, 0.4572 nM, 0.1524 nM, 0.0508 nM, and 0.0169 nM.

3. Reaction termination and detection

1) 10 μL of ADP Glo reagent was added, centrifuged at 1,000 rpm for 1 min, and then incubated at a room temperature of 25°C for 40 min.
2) 20 μL of kinase detection reagent was added, centrifuged at 1,000 rpm for 1 min, and then incubated at a room temperature of 25°C for 40 min.
3) After the reaction was completed, fluorescence value LUM was read on Envision.

4. Data analysis

Inhibition (% inh) was calculated by using the following equation:

$$\text{inhibition}(\%) = 100\% \times \frac{Lum_{HC} - Lum_{CPD}}{Lum_{HC} - Lum_{LC}}$$

wherein, $Lum_{HC}$ represented: luminous signal intensity of High control (DMSO with the same volume as the compound to be tested was added to the reaction system);
$Lum_{LC}$ represented: luminous signal intensity of Low control (control drug 1 μM);
$Lum_{cpd}$ represented: luminous signal intensity of the sample compound for testing; and
$IC_{50}$ was calculated by curve fitting with software.

Testing result:

Table 7 Inhibitory activity of the compound of formula (1) of the present invention on KHK-C

| Compound | $IC_{50}$ (nM) |
|---|---|
| PF-06835919 | 9.5 |
| Compound of formula (1) | 0.82 |

It could be seen from the above testing result that the compound of formula (1) of the present invention could effectively inhibit the activity of KHK-C kinase and was an effective inhibitor of KHK-C kinase.

**Experimental Example 2: In vitro cell inhibitory activity test of the compound of formula (1)**

Sample for testing: compound of formula (1), the chemical name and preparation method of which were referred to the Preparation Example.
Control drug: PF-06835919, prepared by referring to the method in reference (US15/381,295).

Reagents:

| Reagent | Vendor | Cat No. |
|---|---|---|
| MEM medium | Gibco | 10370-021 |
| HepG2 hepatoma cell line | ATCC | HB-8065 |

(continued)

| Reagent | Vendor | Cat No. |
|---|---|---|
| Sodium pyruvate | Gibco | 11360-070 |
| Glutamine | Gibco | 35050-061 |
| Fetal bovine serum | Gibco | 10091-148 |
| (FBS) | | |
| DPBS buffer | Gibco | 14200-075 |
| 0.25% pancreatin (containing EDTA) | Gibco | 25200-072 |
| DMSO | Sigma | D4540 |
| Penicillin-streptomycin | Gibco | 10378016 |
| Ammonium acetate | TEDIA | AS0139-028 |
| D-fructose | sigma | F3510-100G |

Instruments:

| Instrument | Vendor | Cat No. |
|---|---|---|
| $CO_2$ incubator | SANYO | MCO-15A4 |
| Biosafety cabinet | Shanghai Lishen Technology Instrument Co., Ltd. | 1200A2 |
| Refrigerated centrifuge | Eppendorf | Centrifuge 5840R |
| Microscope | Nikon | TS100-F |

Testing method:

1. Cell culture and inoculation

1) HepG2 cells in logarithmic growth phase were collected and counted by using a platelet counter. Cell viability was detected by trypan blue exclusion method to ensure that the cell viability was above 90%.
2) A concentration of the HepG2 cells was adjusted, and 81 μL of FBS-free basal medium (starvation treated) cell suspension was added to each 96-well plate with a testing well cell inoculation quantity of $5\times10^4$.
3) The HepG2 cells in the 96-well plates were cultured overnight at 37°C, 5% $CO_2$, and 95% humidity.

2. Dosing

1) Before dosing, 9 μL of FBS was added into testing wells of the 96-well plate, and the starvation treatment was stopped.
2) A 10-fold compound solution was prepared, with the highest concentration of 100 μM, 8 concentrations and 3-fold gradient dilution. 10 μL of drug solution was added to each well of the 96-well plate inoculated with the cells, and three repeated wells were set for each drug concentration. 10 μL of 1% DMSO solution was added to the solvent control group, and three repeated wells were set.
3) The cells in the 96-well plates added with the drug were incubated at 37°C, 5% $CO_2$, and 95% humidity for 30 min.
4) A 11-fold D-Fructose solution was prepared, with a concentration of 220 mM. 10 μL of D-Fructose was added to each well of the 96-well plate inoculated with the HepG2 cells, and three repeated wells were set. 10 μL of 11% DPBS solution was added to the solvent control group, and three repeated wells were set.

5) The cells in the 96-well plates added with the D-fructose were cultured overnight at 37°C, 5% $CO_2$, and 95% humidity for 3h.

3. Sample treatment

The culture medium in the 96-well plate was removed, the cells in the well plate were washed with cold DPBS (200 μl/ well) for three times, then 30 μL of cold 10 mM ammonium acetate (pH 7.4) was added into each well to lyse the cells (hypotonic lysis), and after 5 min of ice lysis, the cells were fully pipetted, and the cell lysate was taken for LC-MS analysis.

4. Data analysis

Data were analyzed by GraphPad Prism 5.0 software, a dose-effect curve was obtained by using nonlinear S-curve regression, and the EC50 value was calculated.

5. Testing result:

Table 8 Inhibitory effect of the compound of formula (1) on D-Fructose-induced fructose-1-phosphate in HepG2 cells

| Compound | EC50 (nM) |
|---|---|
| PF-06835919 | 169.3 |
| Compound of formula (1) | 21.9 |

The KHK-C kinase in the liver had the highest expression quantity, and meanwhile, the KHK-C kinase could specifically metabolize fructose to generate fructose-1-phosphate. The above testing result showed that the compound of formula (1) of the present invention could effectively inhibit D-Fructose from inducing HepG2 cells to generate fructose-1-phosphate, and was an effective KHK-C inhibitor.

## Claims

1. A crystal form of a compound of formula (1), a pharmaceutically acceptable salt of the compound of formula (1), and a crystal form of the pharmaceutically acceptable salt, **characterized in that**:

a crystal form A of the compound of formula (1) has characteristic peaks at 2$\theta$ positions of 8.0±0.2°, 9.4±0.2°, 10.5±0.2°, 12.7±0.2°, 19.5±0.2°, and 21.2±0.2° in an X-ray powder diffraction pattern by using Cu-Kα radiation; preferably, also has characteristic peaks at positions of 10.2±0.2°, 13.6±0.2°, 14.2±0.2°, 15.8±0.2°, 17.2±0.2°, and 25.3±0.2°; further preferably, also has characteristic peaks at positions of 6.9±0.2°, 15.1±0.2°, 16.6±0.2°, 18.9±0.2°, 22.0±0.2°, and 23.1±0.2°; and more preferably, the X-ray powder diffraction pattern of the crystal form A of the compound of formula (1) is substantially as shown in FIG. 1;
a crystal form B of the compound of formula (1) has characteristic peaks at 2$\theta$ positions of 6.4±0.2°, 10.0±0.2°, 12.7±0.2°, 15.5±0.2°, 17.6±0.2°, and 22.0±0.2° in an X-ray powder diffraction pattern by using Cu-Kα radiation; preferably, also has characteristic peaks at positions of 8.3±0.2°, 16.5±0.2°, 17.7±0.2°, and 20.6±0.2°; more preferably, the X-ray powder diffraction pattern of the crystal form B of the compound of formula (1) is substantially as shown in FIG. 4;
a crystal form C of the compound of formula (1) has characteristic peaks at 2$\theta$ positions of 8.5±0.2°, 9.9±0.2°, 10.7±0.2°, and 18.5±0.2° in an X-ray powder diffraction pattern by using Cu-Kα radiation; preferably, also has characteristic peaks at positions of 13.0±0.2°, 13.4±0.2°, 13.8±0.2°, and 16.8±0.2°; further preferably, also has characteristic peaks at positions of 19.2±0.2°, 20.7±0.2°, 21.1±0.2°, and 22.1±0.2°; and more preferably, the X-ray powder diffraction pattern of the crystal form C of the compound of formula (1) is substantially as shown in FIG. 7;
a crystal form D of the compound of formula (1) has characteristic peaks at 2$\theta$ positions of 6.4±0.2°, 10.0±0.2°, 12.7±0.2°, 17.7±0.2°, 18.6±0.2°, and 25.5±0.2° in an X-ray powder diffraction pattern by using Cu-Kα radiation; preferably, also has characteristic peaks at positions of 14.9±0.2°, 15.6±0.2°, and 20.6±0.2°; further preferably, also has characteristic peaks at positions of 8.3±0.2°, 16.5±0.2°, 19.1±0.2°, and 20.2±0.2°; and more preferably, the X-ray powder diffraction pattern of the crystal form D of the compound of formula (1) is substantially as shown in FIG. 10;

the pharmaceutically acceptable salt of the compound of formula (1) is selected from a base addition salt formed by the compound of formula (1) with an inorganic base or an organic base; preferably, the base addition salt is selected from a potassium salt, a sodium salt, a lithium salt, a magnesium salt or a calcium salt;

preferably, a crystal form I of the potassium salt has characteristic peaks at 2$\theta$ positions of 4.8±0.2°, 5.9±0.2°, 7.1±0.2°, 8.3±0.2°, 11.8±0.2°, and 15.5±0.2° in an X-ray powder diffraction pattern by using Cu-K$\alpha$ radiation; preferably, also has characteristic peaks at positions of 14.6±0.2°, 16.6±0.2°, 17.9±0.2°, 18.8±0.2°, 19.6±0.2°, and 21.5±0.2°; further preferably, also has characteristic peaks at positions of 13.0±0.2°, 14.2±0.2°, 23.3±0.2°, and 25.0±0.2°; and more preferably, the X-ray powder diffraction pattern of the crystal form I of the potassium salt is substantially as shown in FIG. 13;

preferably, a crystal form II of the potassium salt has characteristic peaks at 2$\theta$ positions of 4.7±0.2°, 6.5±0.2°, 7.9±0.2°, 10.3±0.2°, 11.0±0.2°, and 12.1±0.2° in an X-ray powder diffraction pattern by using Cu-K$\alpha$ radiation; preferably, also has characteristic peaks at positions of 5.1±0.2°, 13.0±0.2°, 20.1±0.2°, 21.4±0.2°, 22.4±0.2°, and 24.1±0.2°; further preferably, also has characteristic peaks at positions of 8.3±0.2°, 17.6±0.2°, 19.5±0.2°, 20.6±0.2°, 21.1±0.2°, and 24.5±0.2°; and more preferably, the X-ray powder diffraction pattern of the crystal form II of the potassium salt is substantially as shown in FIG. 16;

preferably, a crystal form of the magnesium salt has characteristic peaks at 2$\theta$ positions of 18.8±0.2°, 23.8±0.2°, 26.3±0.2°, and 29.5±0.2° in an X-ray powder diffraction pattern by using Cu-K$\alpha$ radiation; preferably, also has characteristic peaks at positions of 23.0±0.2°, 25.9±0.2°, 31.5±0.2°, 32.0±0.2°, 38.1±0.2°, and 39.0±0.2°; and more preferably, the X-ray powder diffraction pattern of the crystal form of the magnesium salt is substantially as shown in FIG. 18;

preferably, a crystal form I of the calcium salt has characteristic peaks at 2$\theta$ positions of 15.8±0.2°, 16.1±0.2°, 26.3±0.2°, 28.0±0.2°, 30.2±0.2°, and 31.2±0.2° in an X-ray powder diffraction pattern by using Cu-K$\alpha$ radiation; and preferably, the X-ray powder diffraction pattern of the crystal form I of the calcium salt is substantially as shown in FIG. 20;

preferably, a crystal form II of the calcium salt has characteristic peaks at 2$\theta$ positions of 3.9±0.2°, 6.0±0.2°, 8.1±0.2°, 11.1±0.2°, 16.0±0.2°, 17.4±0.2°, and 19.0±0.2° in an X-ray powder diffraction pattern by using Cu-K$\alpha$ radiation; and preferably, the X-ray powder diffraction pattern of the crystal form II of the calcium salt is substantially as shown in FIG. 21; and

preferably, a crystal form I of the sodium salt has characteristic peaks at 2$\theta$ positions of 3.9±0.2°, 4.5±0.2°, 7.8±0.2°, 9.1±0.2°, 11.8±0.2°, and 12.7±0.2° in an X-ray powder diffraction pattern by using Cu-K$\alpha$ radiation; preferably, also has characteristic peaks at positions of 8.5±0.2°, 10.5±0.2°, 14.2±0.2°, 16.8±0.2°, and 17.9±0.2°; further preferably, also has characteristic peaks at positions of 18.6±0.2°, 20.4±0.2°, 30.5±0.2°, and 34.7±0.2°; and more preferably, the X-ray powder diffraction pattern of the crystal form I of the sodium salt is substantially as shown in FIG. 24;

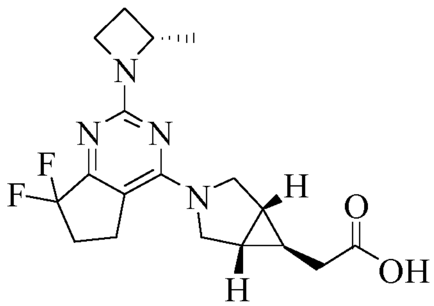
formula (I).

**2.** The crystal form, the pharmaceutically acceptable salt, and the crystal form of the pharmaceutically acceptable salt of claim 1, **characterized in that**:

the crystal form A has an endothermic peak in the range of 167°C to 173°C in a differential scanning calorimetry thermogram, preferably has an endothermic peak at 170°C±2°C; and further preferably, the differential scanning calorimetry thermogram of the crystal form A is substantially as shown in FIG. 2;

the crystal form B has an endothermic peak in the range of 167°C to 173°C in a differential scanning calorimetry thermogram, preferably has an endothermic peak at 169°C±2°C; and further preferably, the differential scanning calorimetry thermogram of the crystal form B is substantially as shown in FIG. 5;

the crystal form C has an endothermic peak in the range of 170°C to 175°C in a differential scanning calorimetry thermogram, preferably has an endothermic peak at 172°C±2°C; and further preferably, the differential scanning calorimetry thermogram of the crystal form C is substantially as shown in FIG. 8;
the crystal form D has an endothermic peak in the range of 165°C to 170°C in a differential scanning calorimetry thermogram, preferably has an endothermic peak at 167°C±2°C; and further preferably, the differential scanning calorimetry thermogram of the crystal form D is substantially as shown in FIG. 11;
the crystal form I of the potassium salt has an endothermic peak in the range of 174°C to 180°C in a differential scanning calorimetry thermogram, preferably has an endothermic peak at 176°C±2°C; and further preferably, the differential scanning calorimetry thermogram of the crystal form I is substantially as shown in FIG. 14;
the crystal form II of the potassium salt has an endothermic peak in the range of 155°C to 165°C in a differential scanning calorimetry thermogram, preferably has an endothermic peak at 160°C±2°C; and further preferably, the differential scanning calorimetry thermogram of the crystal form II is substantially as shown in FIG. 17A; and
the crystal form of the magnesium salt has an endothermic peak in the range of 165°C to 180°C in a differential scanning calorimetry thermogram, preferably has an endothermic peak at 170°C±2°C; and further preferably, the differential scanning calorimetry thermogram of the crystal form of the magnesium salt is substantially as shown in FIG. 19.

**3.** The crystal form, the pharmaceutically acceptable salt, and the crystal form of the pharmaceutically acceptable salt of claim 1 or 2, **characterized in that**:

the crystal form A has no weight loss below 200°C as determined by thermogravimetric analysis; preferably, a thermogravimetric analysis chart of the crystal form A is substantially as shown in FIG. 3;
the crystal form B has no weight loss below 200°C as determined by thermogravimetric analysis; preferably, a thermogravimetric analysis chart of the crystal form B is substantially as shown in FIG. 6;
the crystal form C has no weight loss below 200°C as determined by thermogravimetric analysis; preferably, a thermogravimetric analysis chart of the crystal form C is substantially as shown in FIG. 9; and
the crystal form D has no weight loss below 200°C as determined by thermogravimetric analysis; preferably, a thermogravimetric analysis chart of the crystal form D is substantially as shown in FIG. 12.

**4.** Preparation methods for the crystal form, the pharmaceutically acceptable salt, and the crystal form of the pharmaceutically acceptable salt of any one of claims 1-3, **characterized in that**:

the preparation method for the crystal form A comprises: mixing the compound of formula (1) with an organic solvent I, heating the mixture to a first temperature, cooling the temperature to be below 35°C, filtering, and drying the mixture to obtain the crystal form A of the compound of formula (1);
the preparation method for the crystal form B comprises: mixing the compound of formula (1) with an organic solvent II, heating the mixture to a second temperature, cooling the temperature to 10°C-30°C, filtering, and drying the mixture to obtain the crystal form B of the compound of formula (1);
the preparation method for the crystal form C comprises: mixing the compound of formula (1) with an organic solvent III, heating the mixture to a third temperature, cooling for 10h-25h, filtering, drying, and then transferring the mixture to an oven of 100°C-140°C for 1-5h to obtain the crystal form C of the compound of formula (1);
the preparation method for the crystal form D comprises: mixing the compound of formula (1) with an organic solvent IV, adding water after dissolving, filtering the mixture to obtain a solid, adding an organic solvent V, heating and refluxing the mixture for 10-25h, filtering, and drying the mixture to obtain the crystal form D of the compound of formula (1); and
the preparation method for the pharmaceutically acceptable salt of compound of formula (1) comprises: subjecting the compound of formula (1) and an inorganic base to a salt-forming reaction in a reaction solvent A.

**5.** The preparation method of claim 4, **characterized in that** the first temperature is selected from 40°C-95°C, preferably 65°C-90°C;

the second temperature is selected from 40°C-80°C, preferably 60°C-70°C; and
the third temperature is selected from 40°C-80°C, preferably 50°C-65°C.

**6.** The preparation method according to claim 4 or 5, **characterized in that**:

the organic solvent I is a first ester solvent; preferably, the first ester solvent is a fatty ester solvent; preferably, the fatty ester solvent is selected from one or a combination of two or more solvents of methyl formate, ethyl

formate, propyl formate, isopropyl formate, methyl acetate, ethyl acetate, n-propyl acetate, isopropyl acetate, methyl propionate, ethyl propionate, propyl propionate, isopropyl propionate, butyl acetate, and isobutyl acetate; preferably, the fatty ester solvent is selected from one or a combination of two or more solvents of ethyl formate, methyl acetate, ethyl acetate, isopropyl acetate, n-propyl acetate, butyl acetate, and isobutyl acetate;

the organic solvent II and the organic solvent IV are each independently selected from a first alcohol solvent; preferably, the first alcohol solvents are each independently selected from one or a combination of two or more solvents of fatty alcohol solvents, alicyclic alcohol solvents, and aromatic alcohol solvents;

preferably, the fatty alcohol solvents are each independently selected from ethanol, propanol, isopropanol, n-butanol, isobutanol, tert-butanol, sec-butanol, n-pentanol, n-hexanol, ethylene glycol, propylene glycol, or glycerol; preferably, the fatty alcohol solvents are selected from ethanol, n-propanol, isopropanol, isobutanol, and n-pentanol;

preferably, the alicyclic alcohol solvents are each independently selected from cyclopentanol, cyclopentanemethanol, cyclohexanol, cyclohexanemethanol, or cyclohexyl ethanol;

preferably, the aromatic alcohol solvents are each independently selected from phemethylol, phenyl ethanol, or phenylpropanol;

the organic solvent III is methanol; and

the organic solvent V is an ether solvent; preferably, the ether solvent is selected from ether, isopropyl ether, methyl tert-butyl ether, tetrahydrofuran, and 1,4-dioxane.

**7.** The preparation methods of claim 4, **characterized in that**:

the reaction solvent A is selected from one or a combination of two or more solvents of a second alcohol solvent, a ketone solvent, a nitrile solvent or an ester solvent;

preferably, the second alcohol solvent is selected from methanol, ethanol, propanol, isopropanol, n-butanol, isobutanol, tert-butanol, sec-butanol, n-pentanol, n-hexanol, ethylene glycol, propylene glycol, or glycerol; preferably, the second alcohol solvent is selected from methanol, ethanol, n-propanol, isopropanol, isobutanol, or n-pentanol;

preferably, the ketone solvent is selected from acetone;

preferably, the nitrile solvent is selected from acetonitrile;

preferably, the second ester solvent is selected from one or a combination of two or more solvents of methyl formate, ethyl formate, propyl formate, isopropyl formate, methyl acetate, ethyl acetate, n-propyl acetate, isopropyl acetate, methyl propionate, ethyl propionate, propyl propionate, isopropyl propionate, butyl acetate, and isobutyl acetate; and

the inorganic base is selected from potassium carbonate, sodium carbonate, magnesium carbonate, calcium carbonate, potassium bicarbonate, sodium bicarbonate, magnesium bicarbonate, calcium bicarbonate, potassium hydroxide, sodium hydroxide, lithium hydroxide, magnesium hydroxide, or calcium hydroxide.

**8.** A pharmaceutical formulation, **characterized by** comprising the crystal form A, the crystal form B, the crystal form C and the crystal form D, and the pharmaceutically acceptable salt of the compound of formula (1) and the crystal form of the pharmaceutically acceptable salt of any one of claims 1-3, and one or more pharmaceutically acceptable carriers and/or diluents, wherein the pharmaceutical formulation is any clinically or pharmaceutically acceptable dosage form.

**9.** A pharmaceutical composition, **characterized by** comprising the crystal form and the pharmaceutically acceptable salt of the compound of formula (1) and the crystal form of the pharmaceutically acceptable salt of any one of claims 1-3, and one or more second therapeutically active agents; optionally, the pharmaceutical composition further comprising one or more pharmaceutically acceptable carriers and/or diluents.

**10.** Use of the crystal form and the pharmaceutically acceptable salt of the compound of formula (1) and the crystal form of the pharmaceutically acceptable salt of any one of claims 1-3, and the pharmaceutical composition of claim 8 in preparation of a medicament for treatment and/or prevention of a KHK-mediated disease or related disease.

**11.** The use of claim 10, **characterized in that** the KHK-mediated disease and related disease are selected from endocrine disorders, urinary diseases, metabolic diseases, nonalcoholic steatohepatitis, cirrhosis, fatty liver, hepatitis, liver failure, hereditary fructose intolerance, nonalcoholic fatty liver disease, hepatobiliary diseases, fibrotic diseases, cardiovascular and cerebrovascular diseases, inflammatory-immune diseases, central nervous system diseases, gastrointestinal diseases, and hyperproliferative diseases such as cancer.

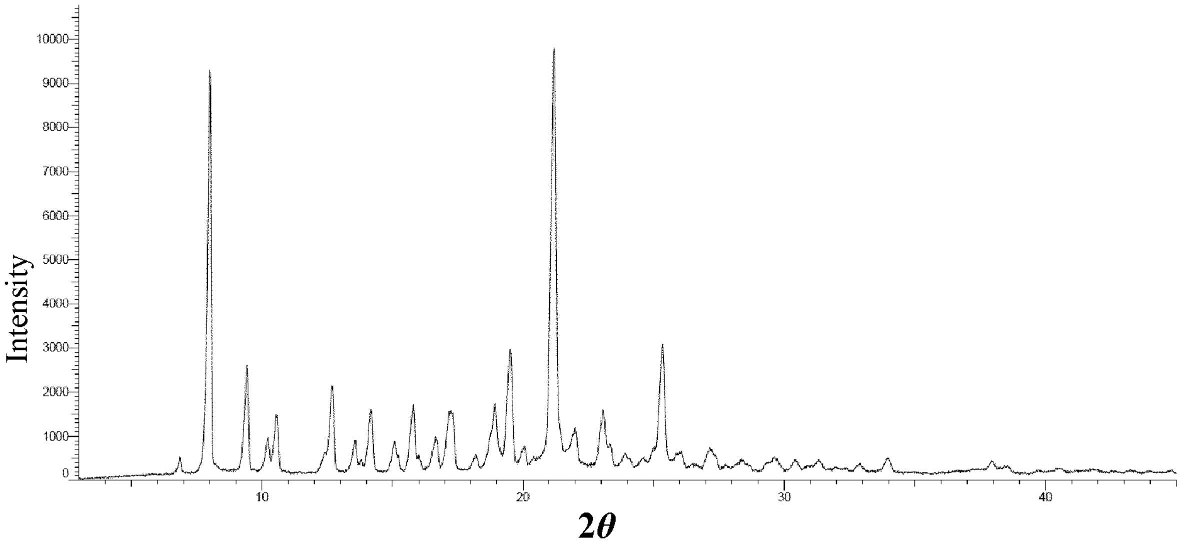
Intensity
10000
9000
8000
7000
6000
5000
4000
3000
2000
1000
0
10
20
30
40
2θ

FIG. 1

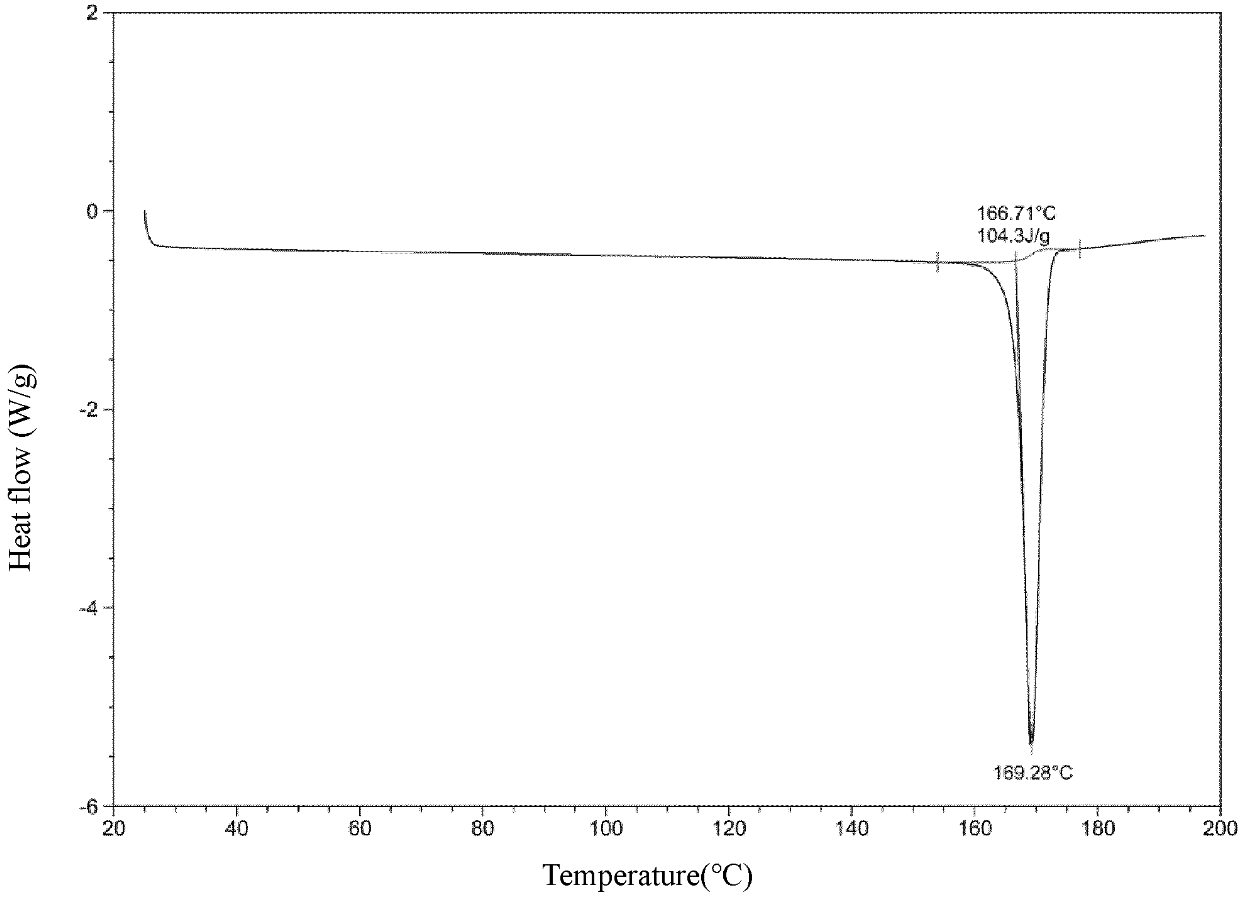
166.71°C
104.3J/g
169.28°C
Heat flow (W/g)
2
0
-2
-4
-6
20
40
60
80
100
120
140
160
180
200
Temperature(°C)

FIG. 2

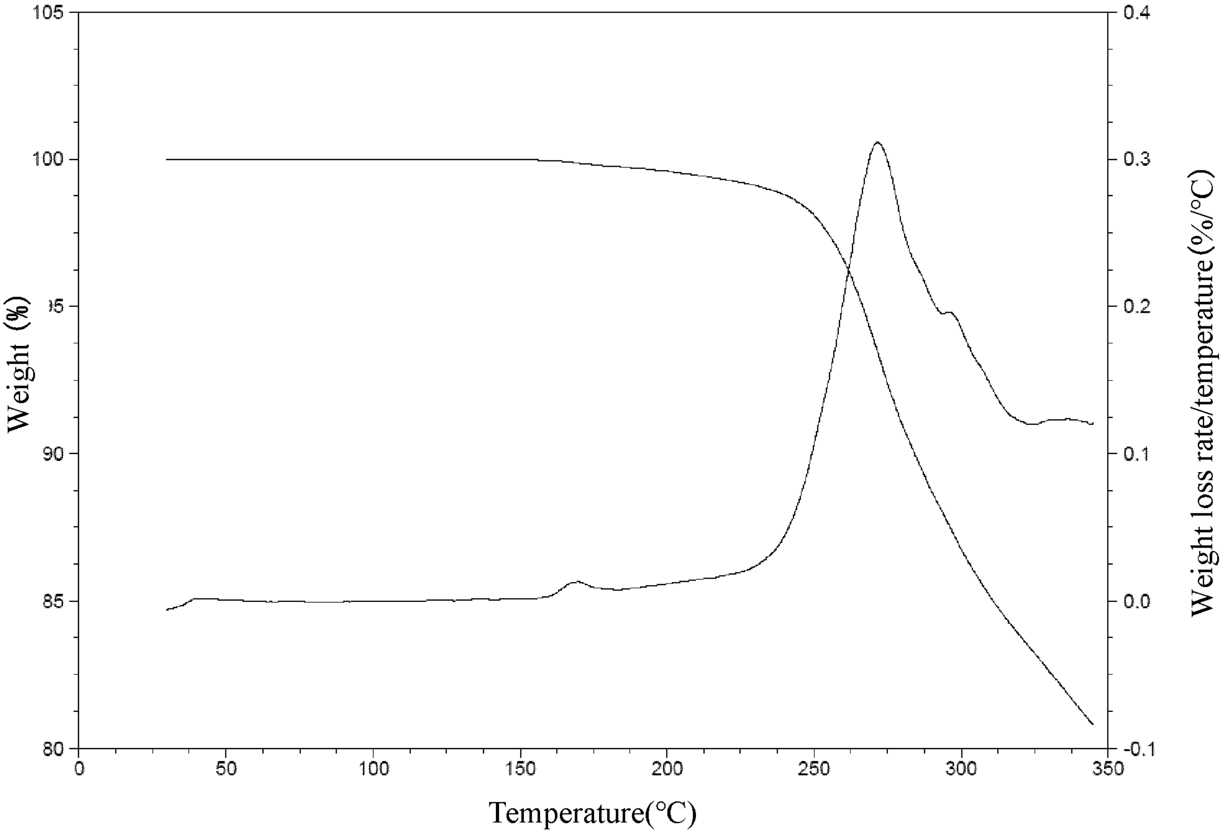
105
100
95
90
85
80
0.4
0.3
0.2
0.1
0.0
-0.1
0
50
100
150
200
250
300
350
Weight (%)
Weight loss rate/temperature (%/°C)
Temperature(°C)

FIG. 3

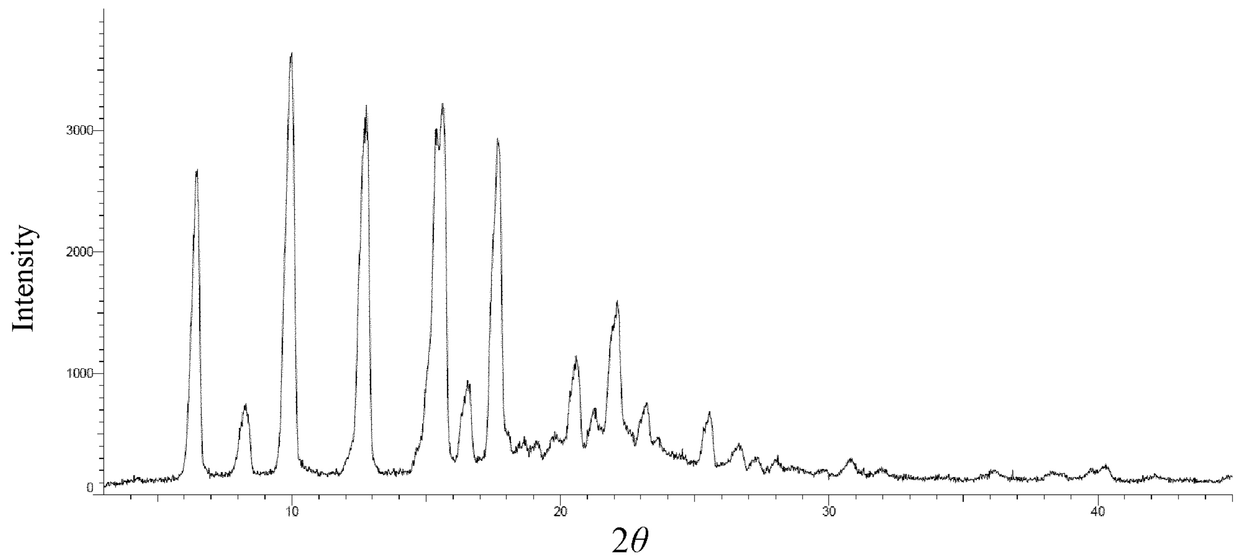
3000
2000
1000
0
10
20
30
40
Intensity
2θ

FIG. 4

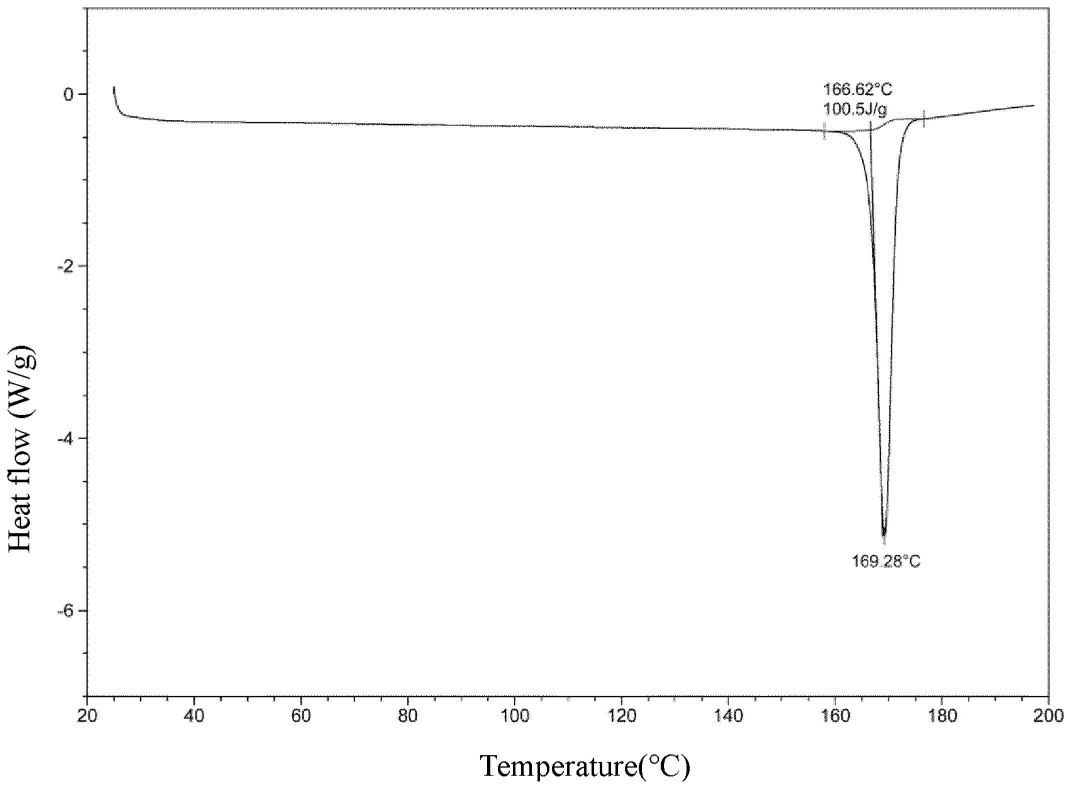
166.62°C
100.5J/g
169.28°C
Heat flow (W/g)
0
-2
-4
-6
20
40
60
80
100
120
140
160
180
200
Temperature(°C)

FIG. 5

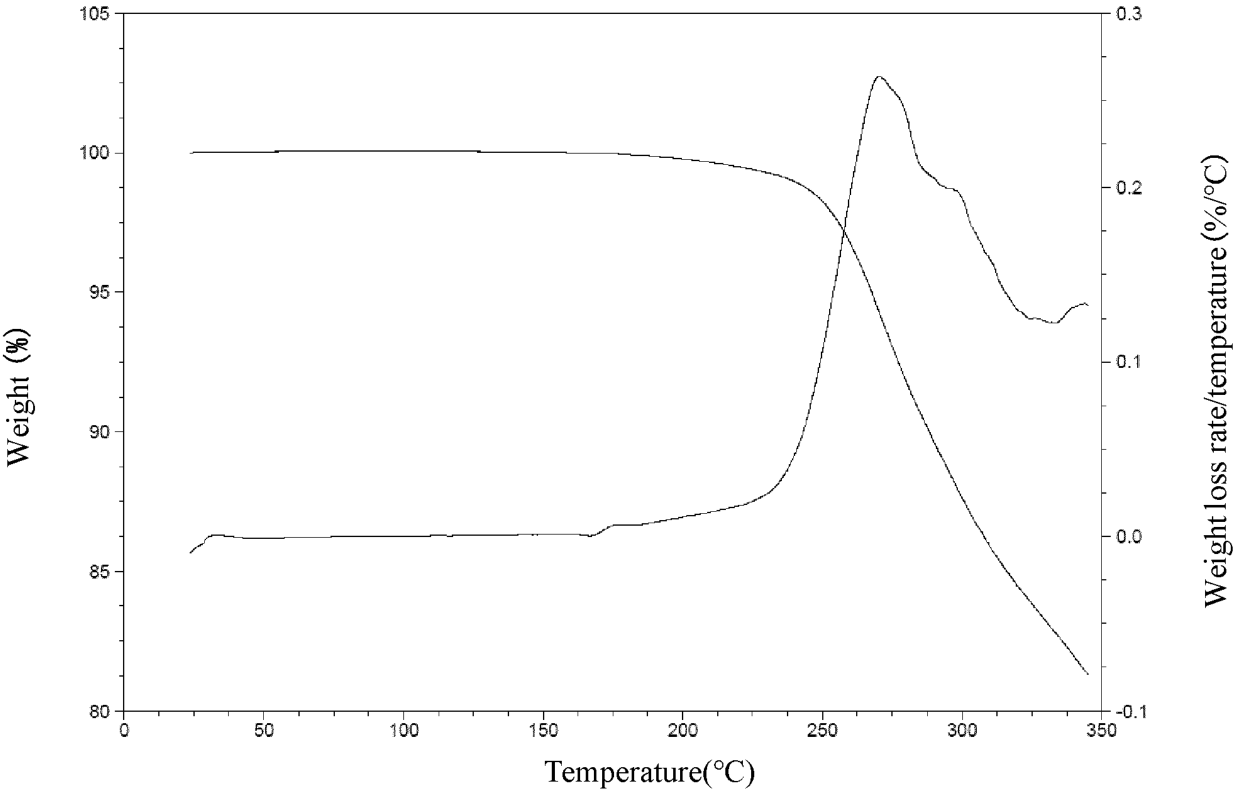
Weight (%)
105
100
95
90
85
80
Weight loss rate/temperature (%/°C)
0.3
0.2
0.1
0.0
-0.1
0
50
100
150
200
250
300
350
Temperature(°C)

FIG. 6

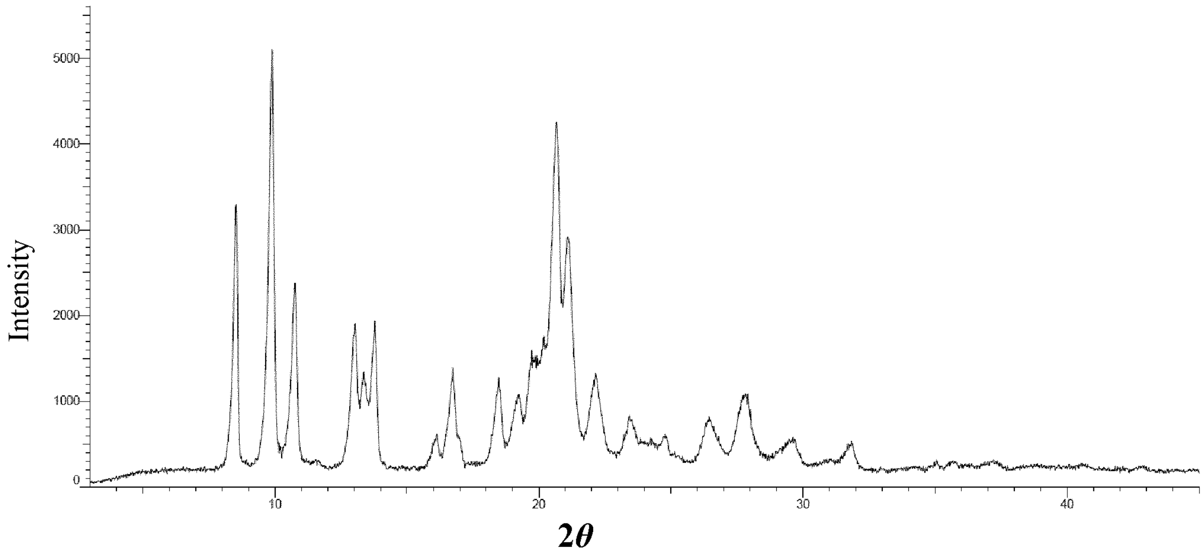
Intensity
5000
4000
3000
2000
1000
0
10
20
30
40
2θ

FIG. 7

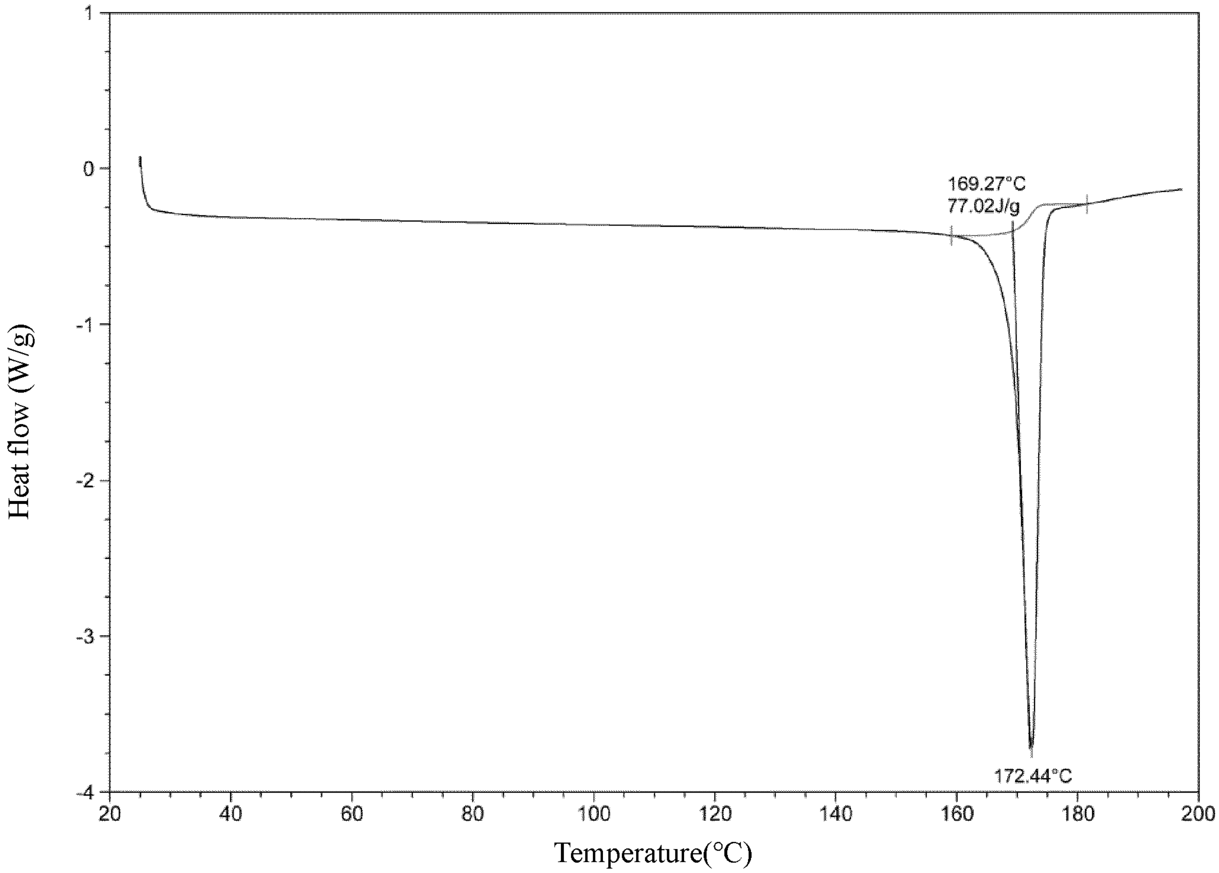
Heat flow (W/g)
1
0
-1
-2
-3
-4
20
40
60
80
100
120
140
160
180
200
Temperature(°C)
169.27°C
77.02J/g
172.44°C

FIG. 8

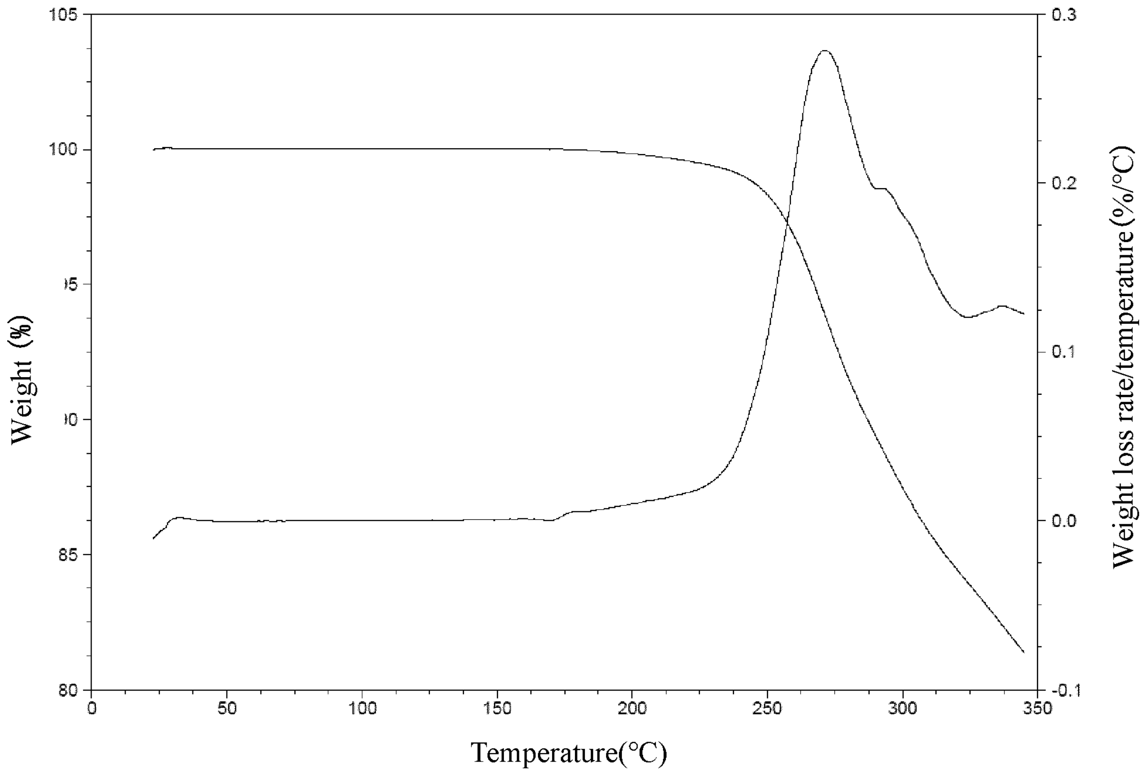
Weight (%)
Weight loss rate/temperature(%/°C)
Temperature(°C)

FIG. 9

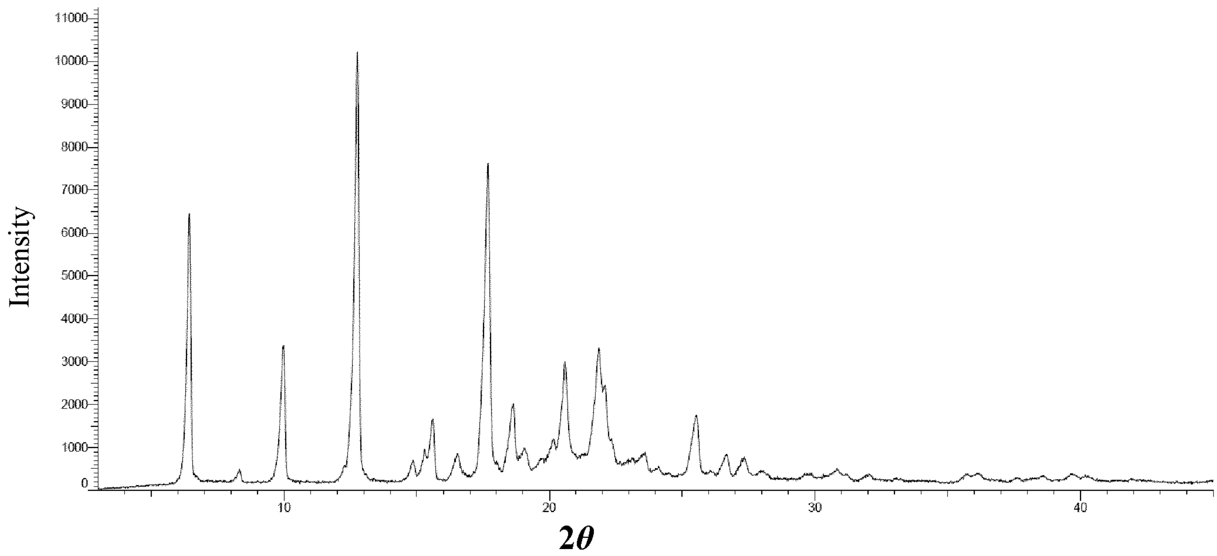
Intensity
2θ

FIG. 10

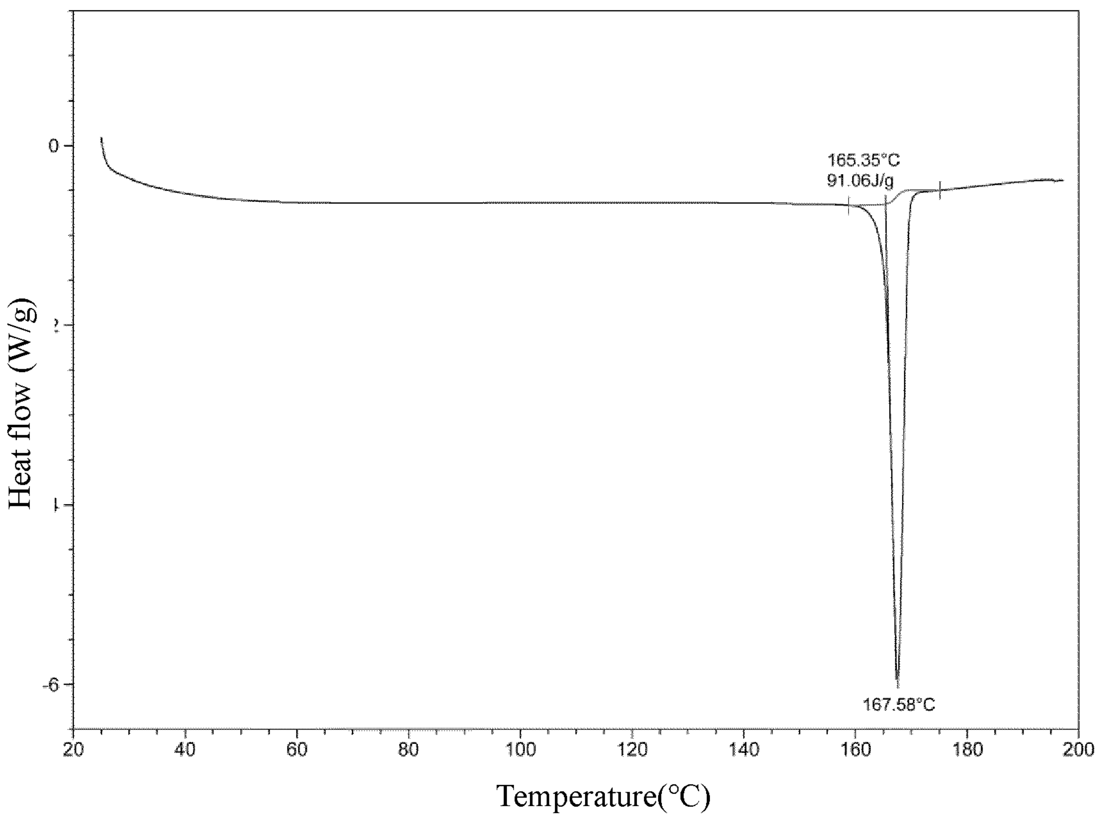
165.35°C
91.06J/g
167.58°C
0
-6
20
40
60
80
100
120
140
160
180
200
Heat flow (W/g)
Temperature(°C)

FIG. 11

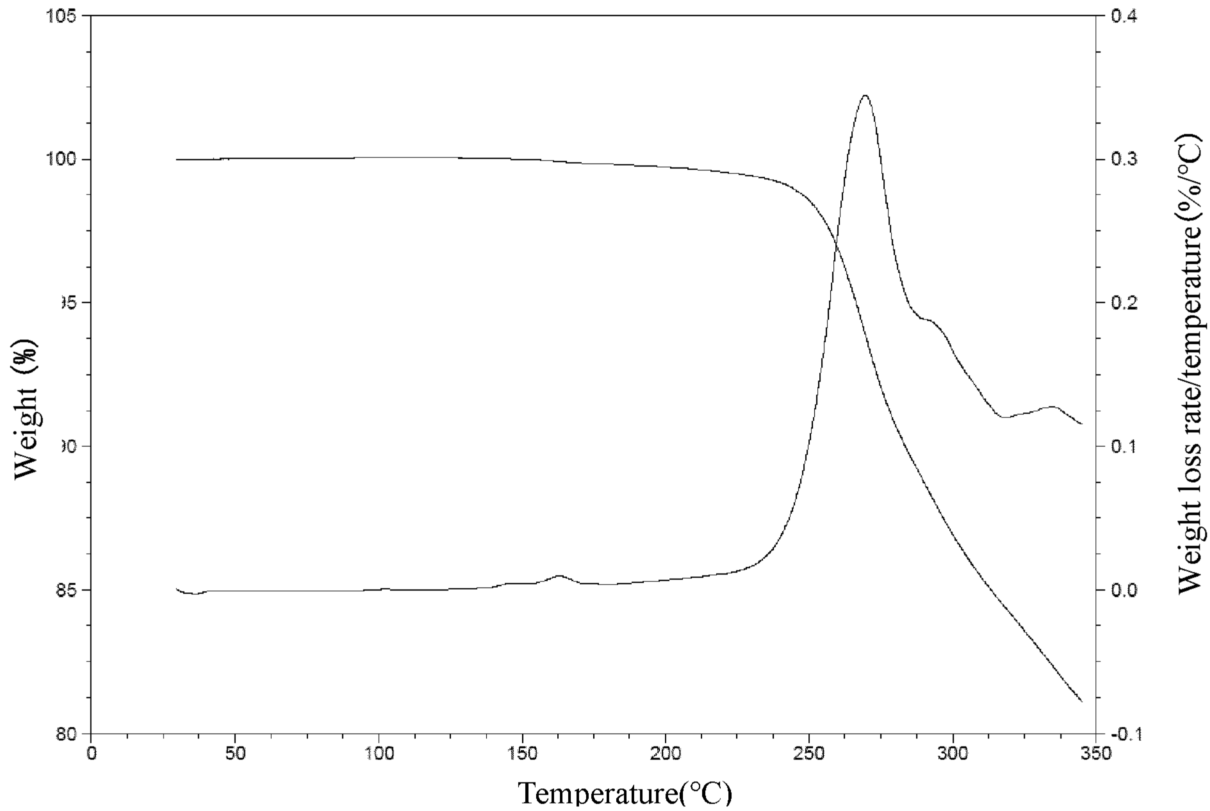
105
100
85
80
0.4
0.3
0.2
0.1
0.0
-0.1
0
50
100
150
200
250
300
350
Weight (%)
Weight loss rate/temperature (%/°C)
Temperature(°C)

FIG. 12

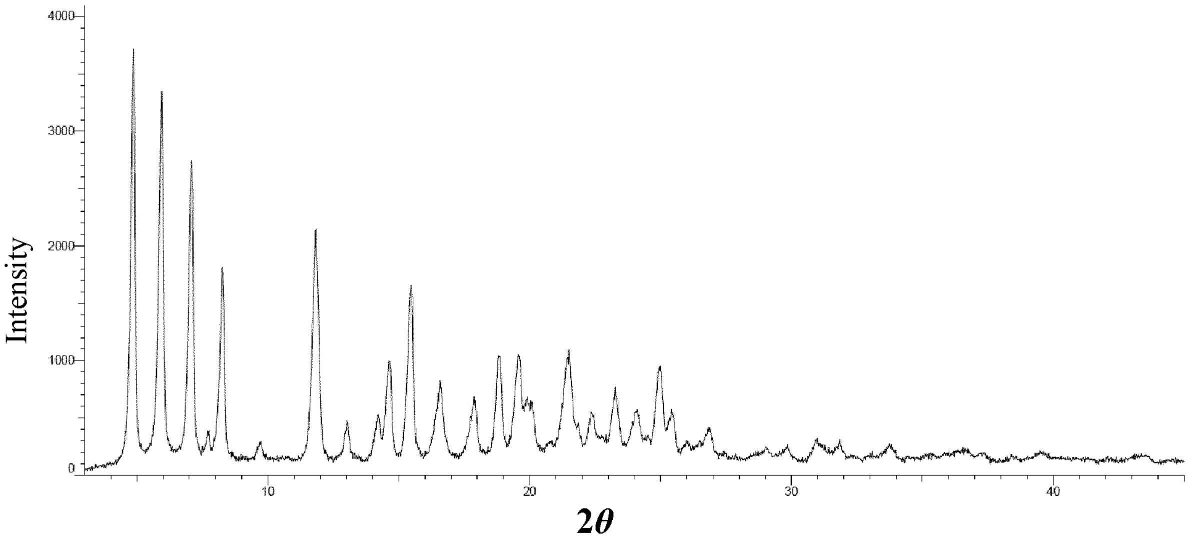
Intensity
2θ

FIG. 13

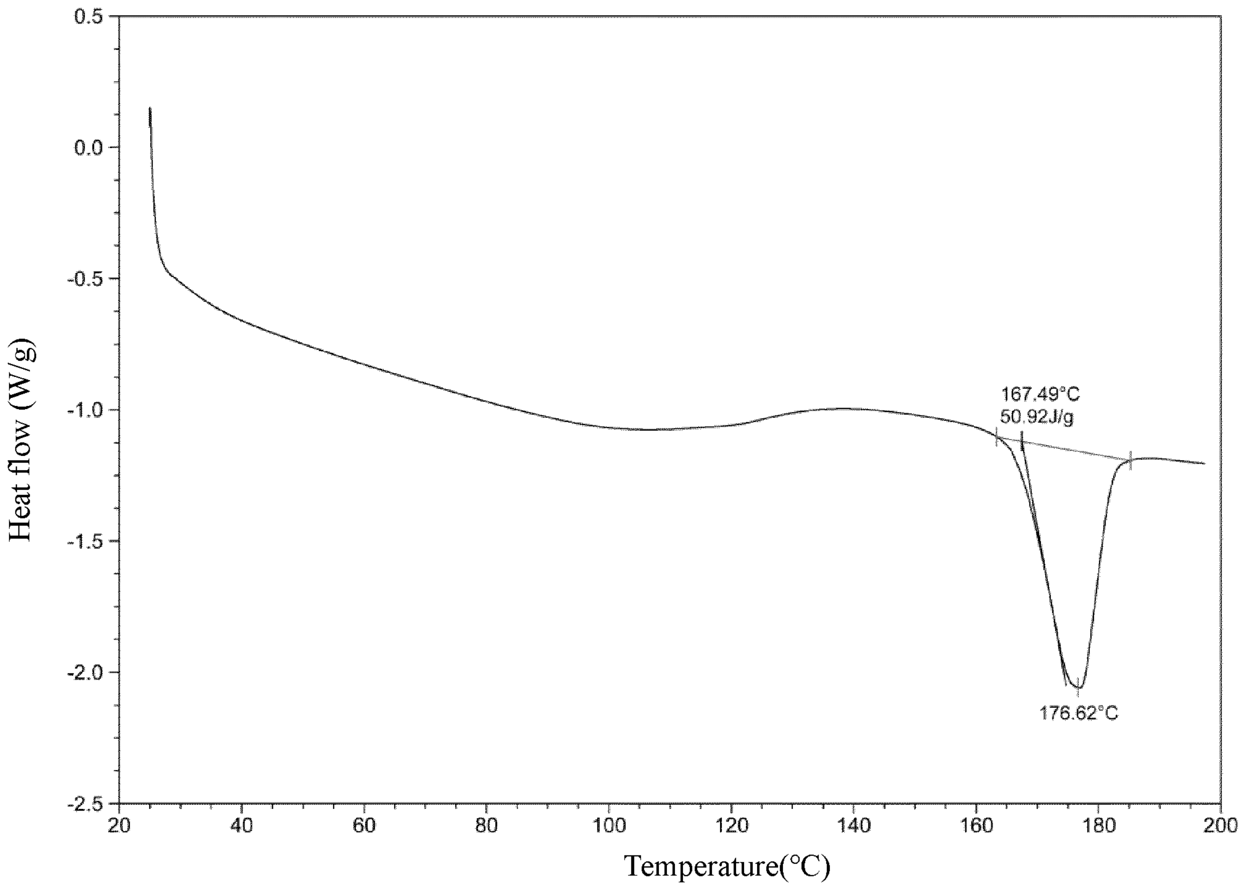
Heat flow (W/g)
Temperature(°C)
167.49°C
50.92J/g
176.62°C

FIG. 14

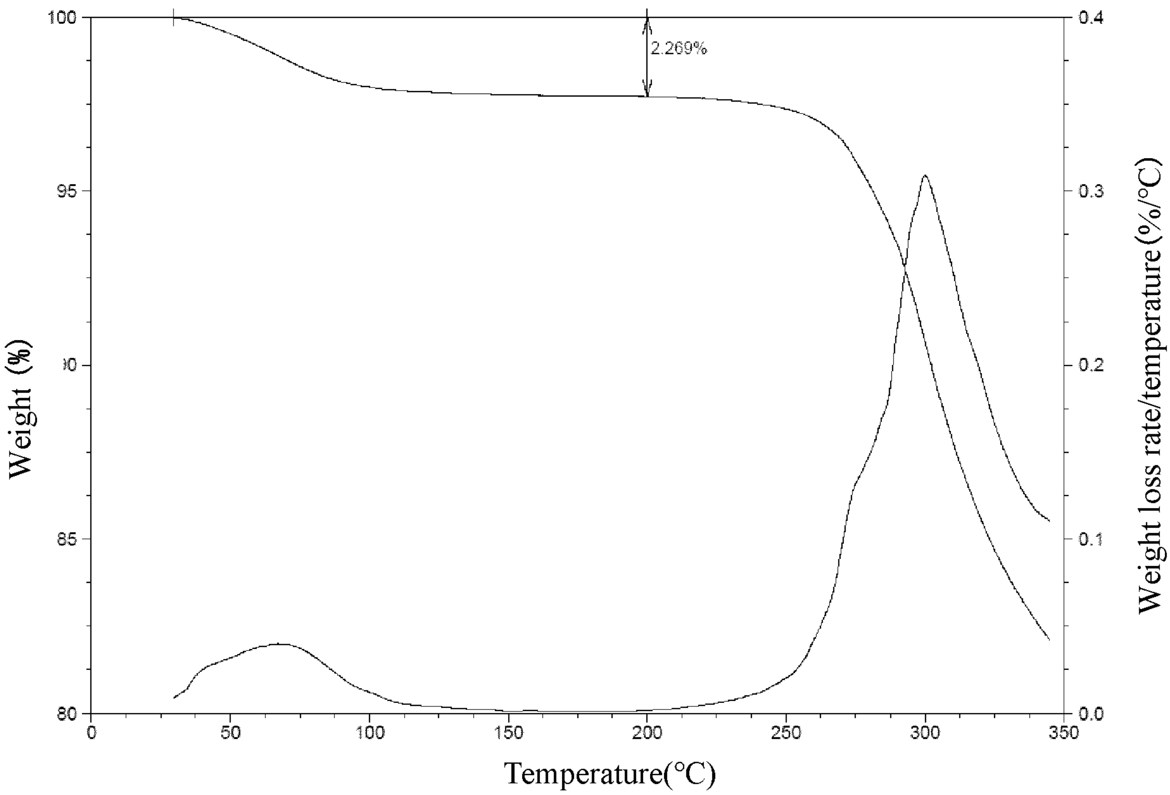
100
95
90
85
80
0.4
0.3
0.2
0.1
0.0
0
50
100
150
200
250
300
350
2.269%
Weight (%)
Weight loss rate/temperature(%/°C)
Temperature(°C)

FIG. 15

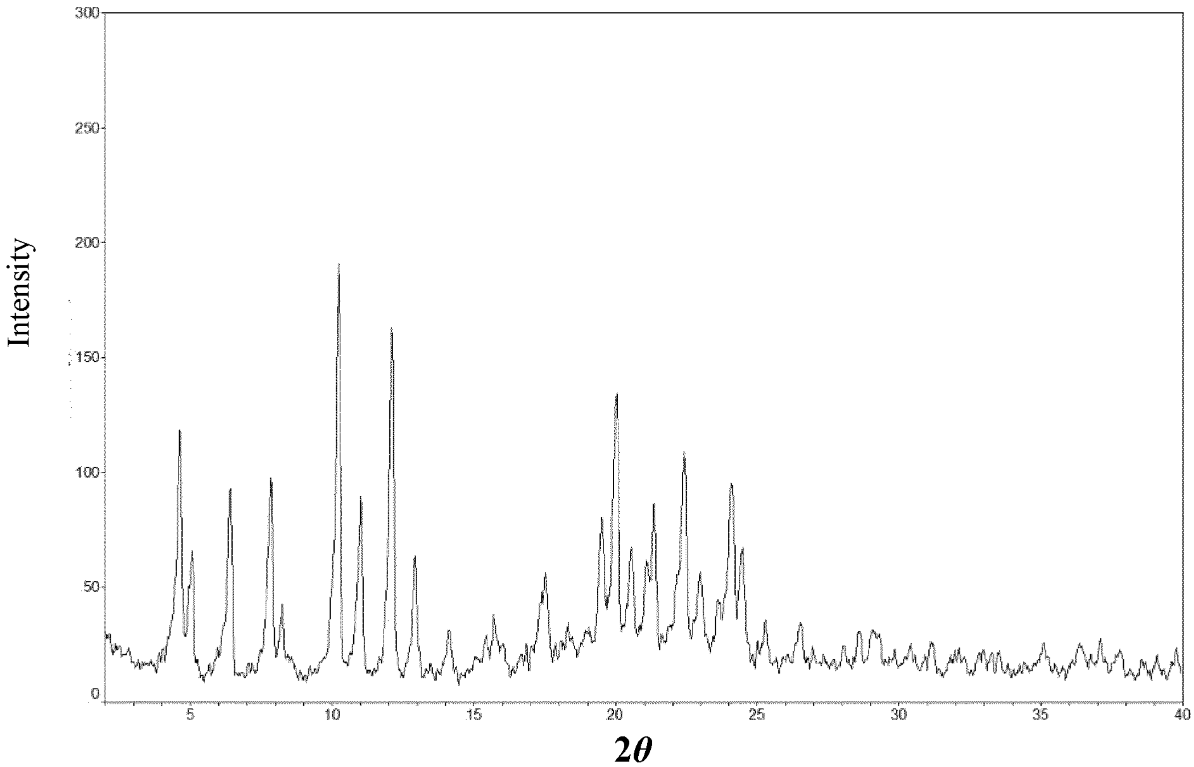
300
250
200
150
100
50
0
5
10
15
20
25
30
35
40
Intensity
2θ

FIG. 16

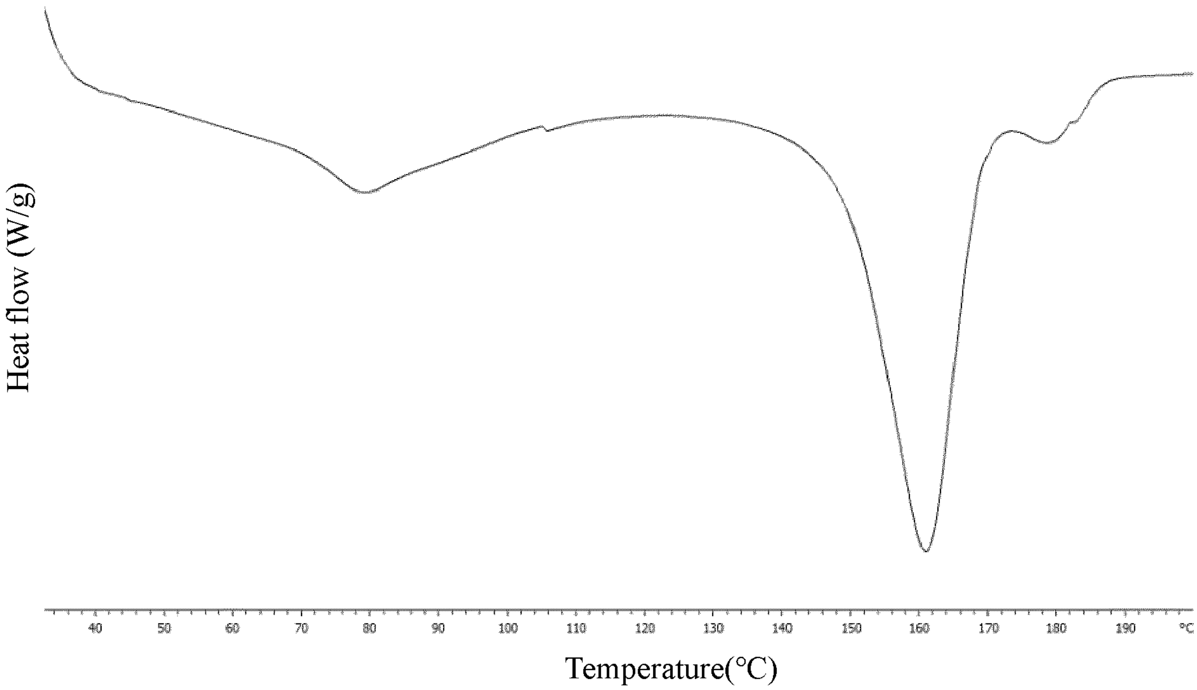


FIG. 17A

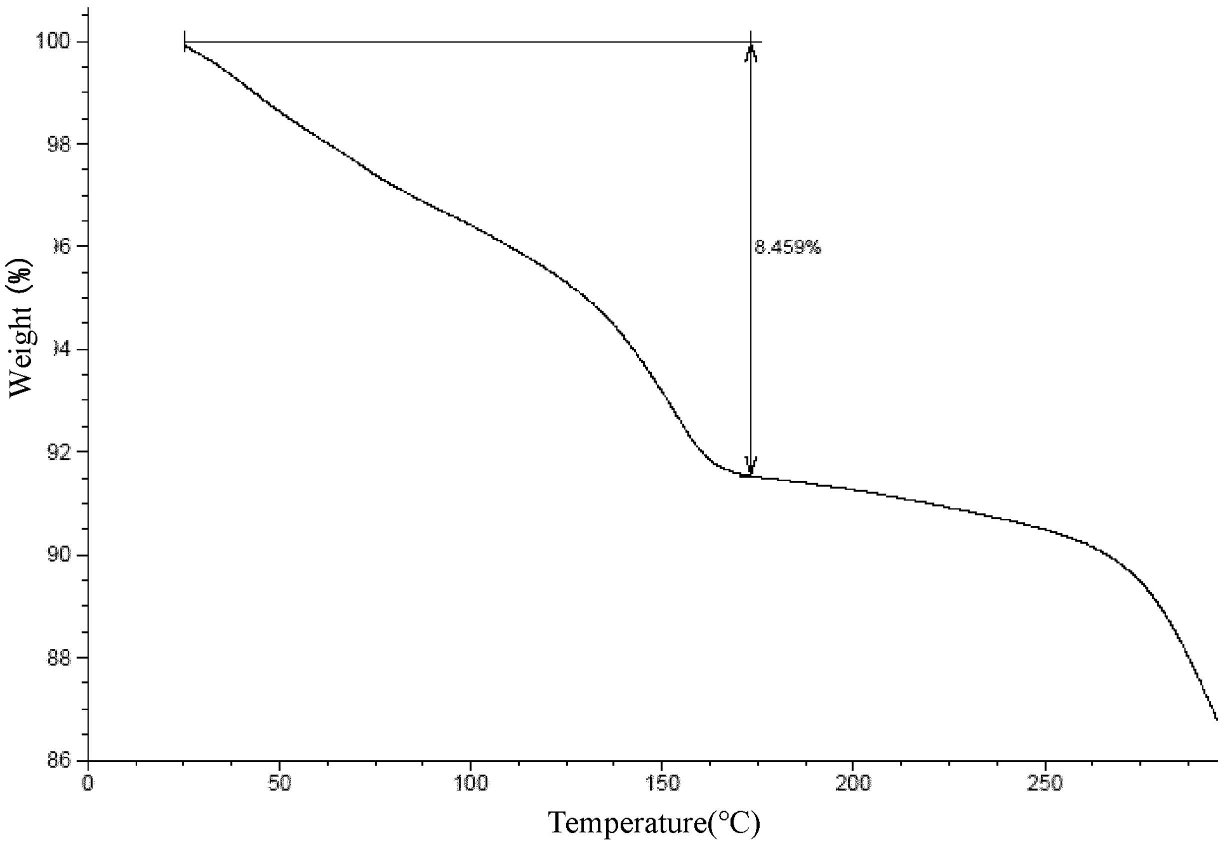


FIG. 17B

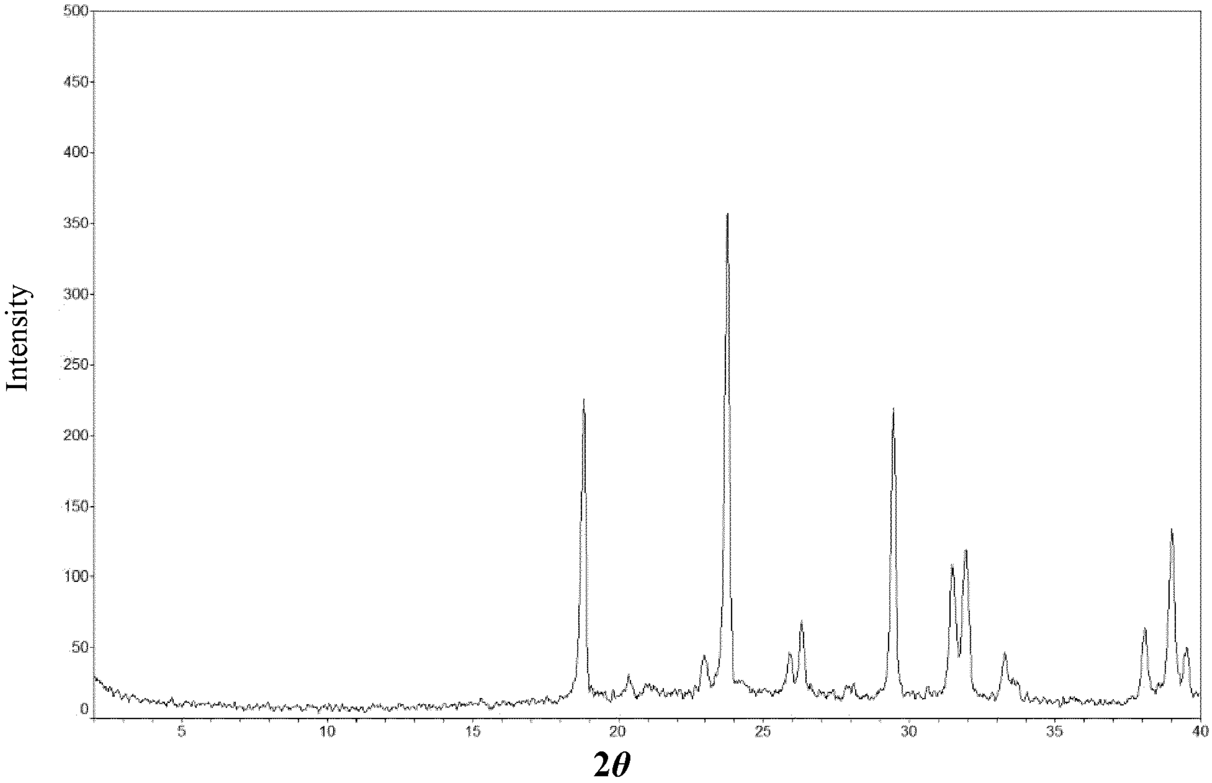
500
450
400
350
300
250
200
150
100
50
0
Intensity
5
10
15
20
25
30
35
40
2θ

FIG. 18

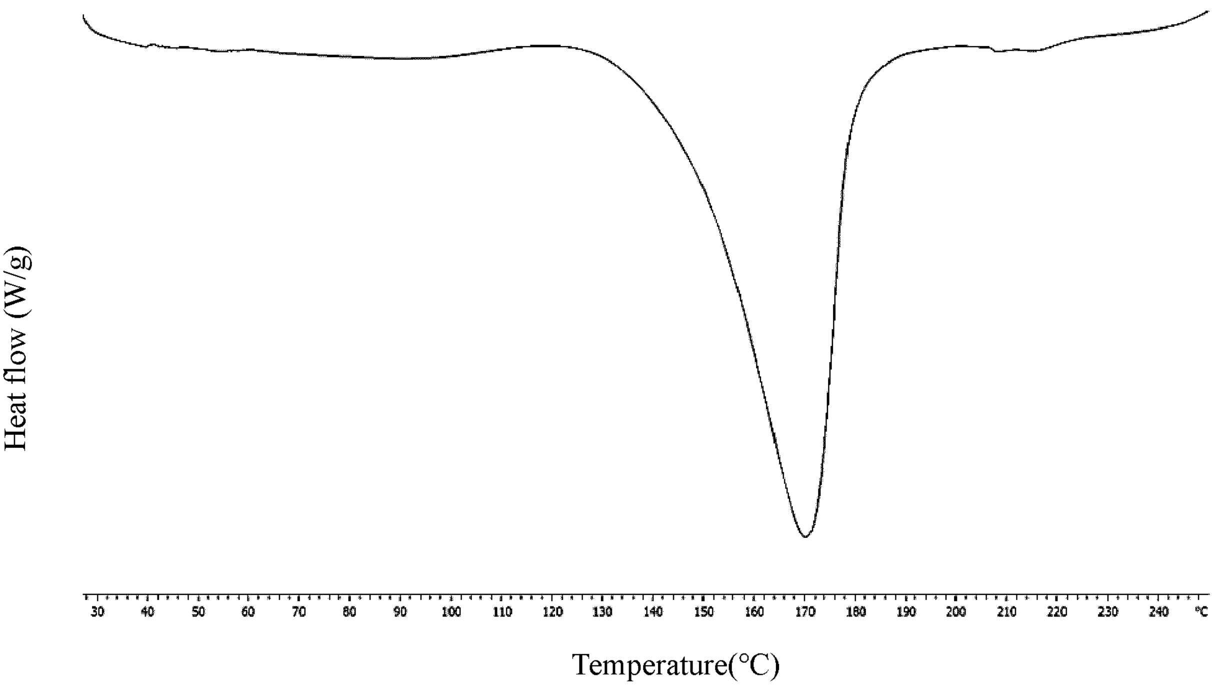
Heat flow (W/g)
30
40
50
60
70
80
90
100
110
120
130
140
150
160
170
180
190
200
210
220
230
240
°C
Temperature(°C)

FIG. 19

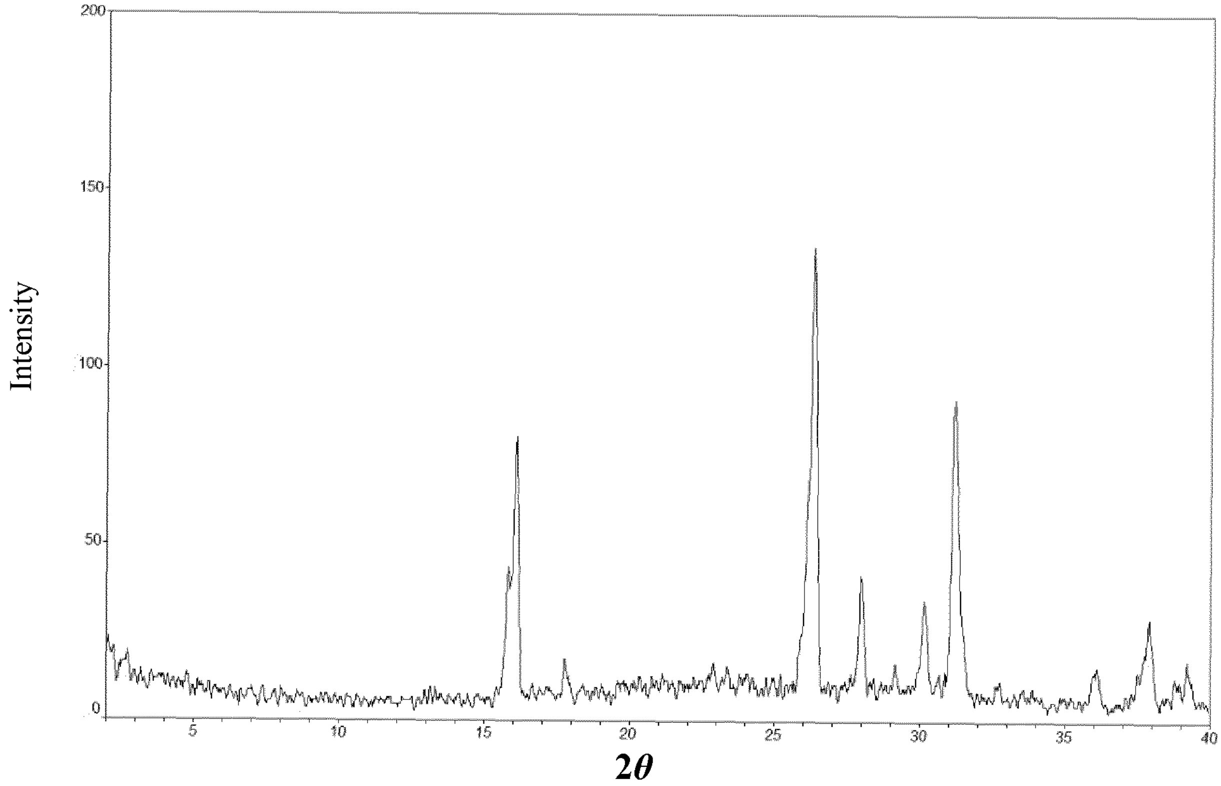
Intensity
200
150
100
50
0
5
10
15
20
25
30
35
40
2θ

FIG. 20

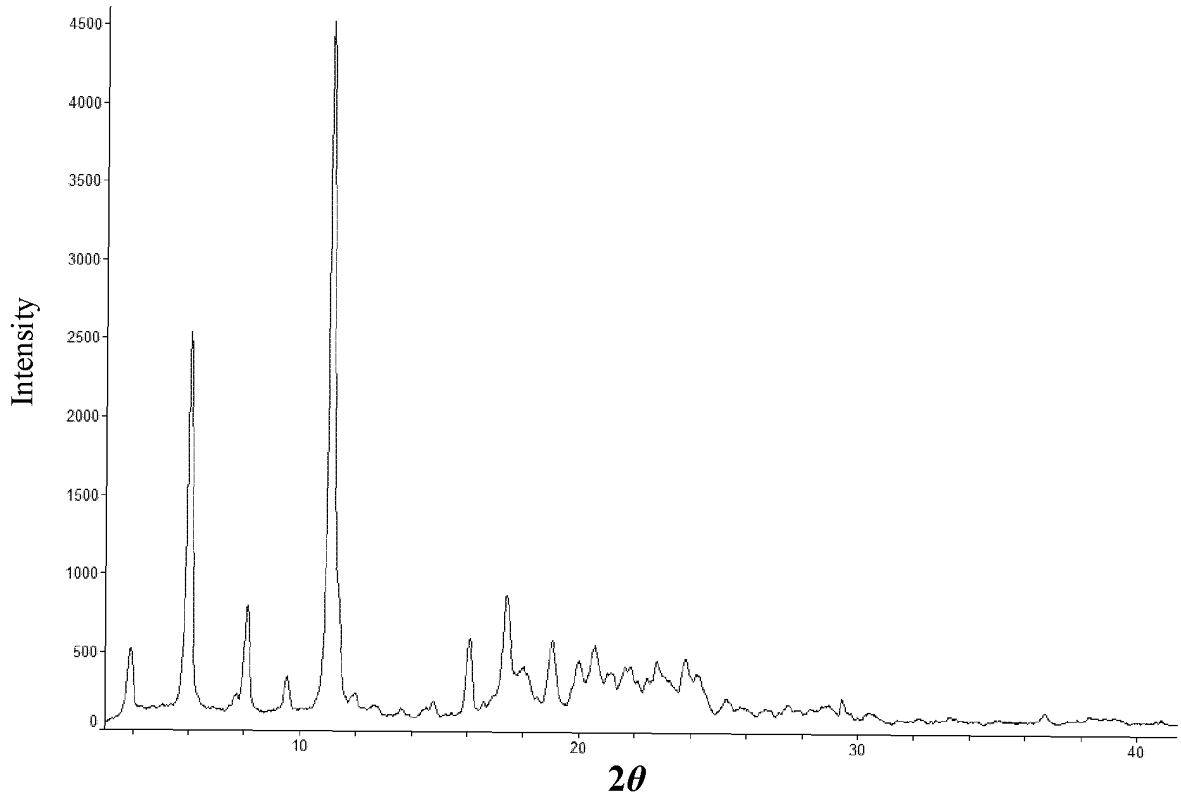
Intensity
4500
4000
3500
3000
2500
2000
1500
1000
500
0
10
20
30
40
2θ

FIG. 21

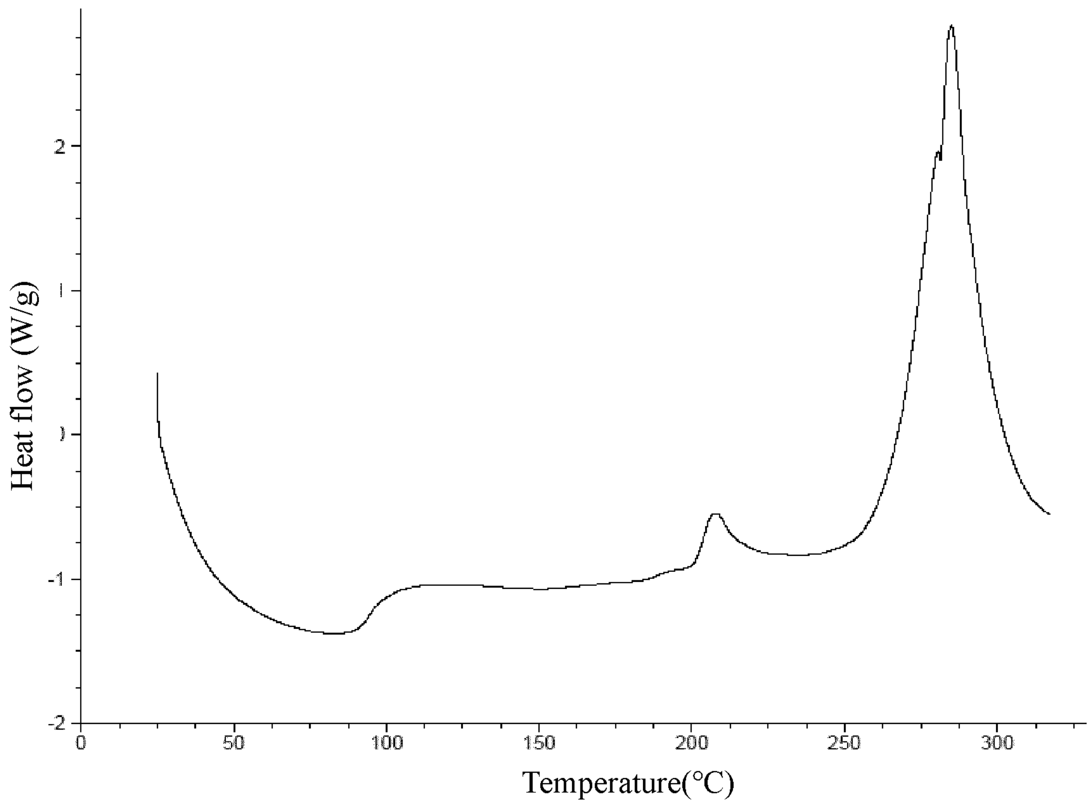
Heat flow (W/g)
Temperature(°C)
2
1
-1
-2
0
50
100
150
200
250
300

FIG. 22

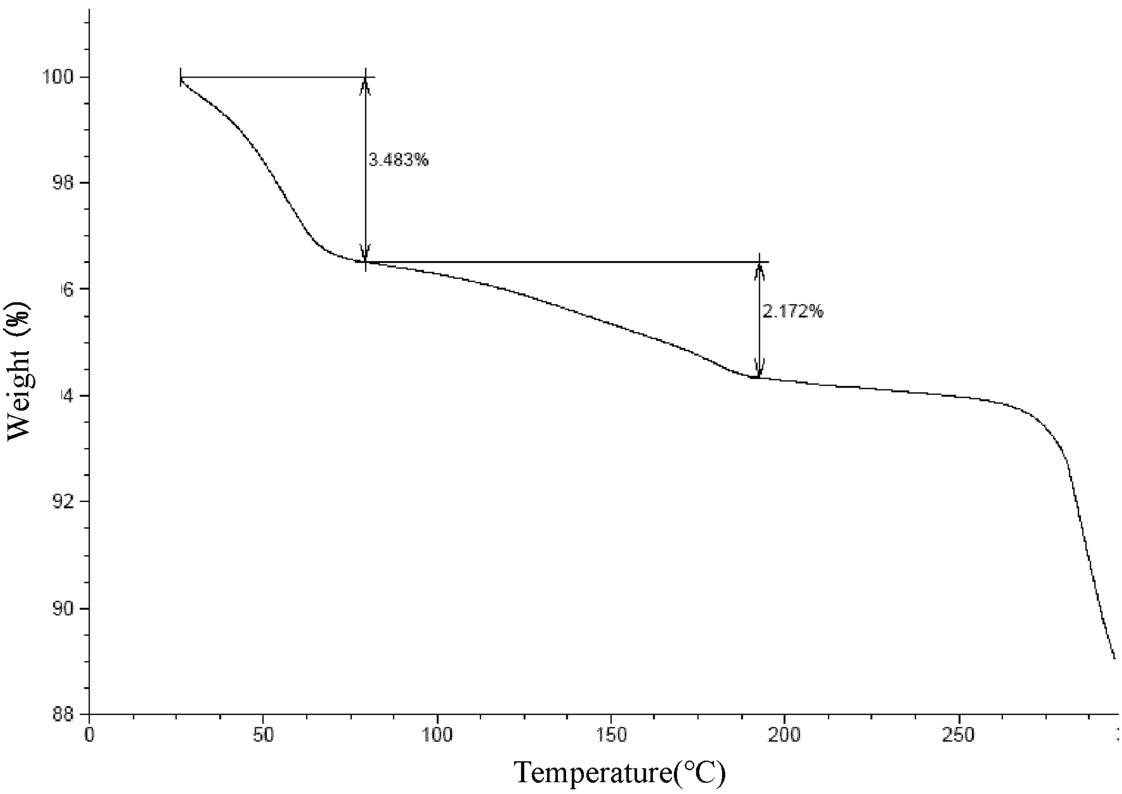
Weight (%)
Temperature(°C)
3.483%
2.172%
100
98
92
90
88
0
50
100
150
200
250

FIG. 23

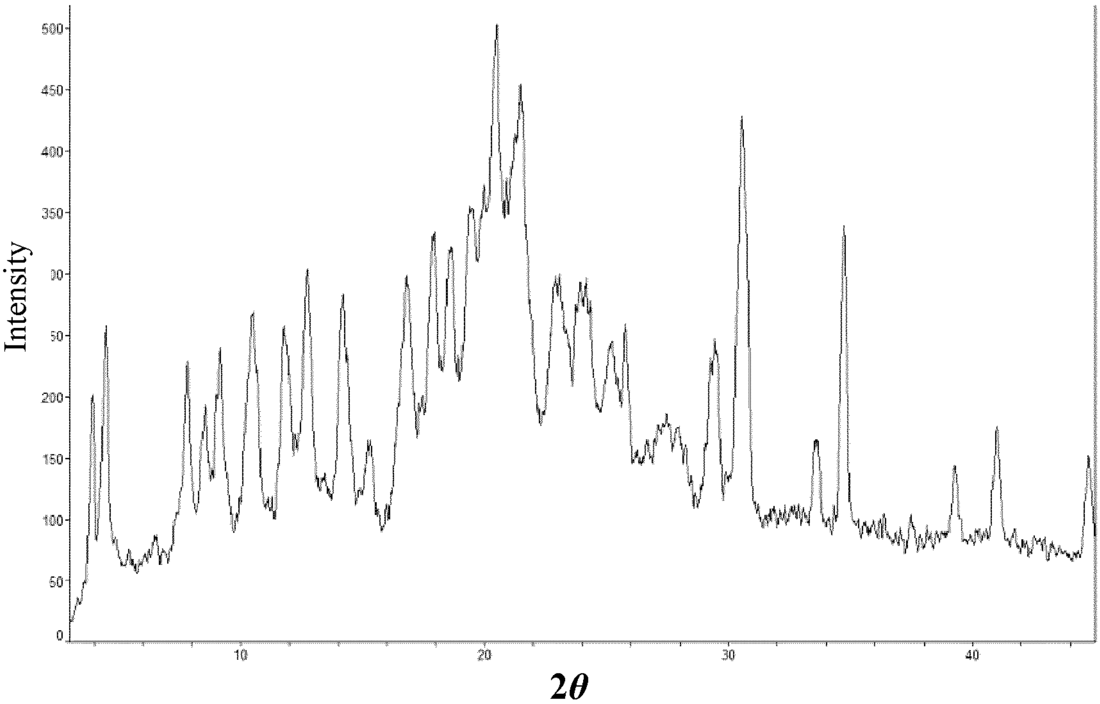
Intensity
2θ

FIG. 24

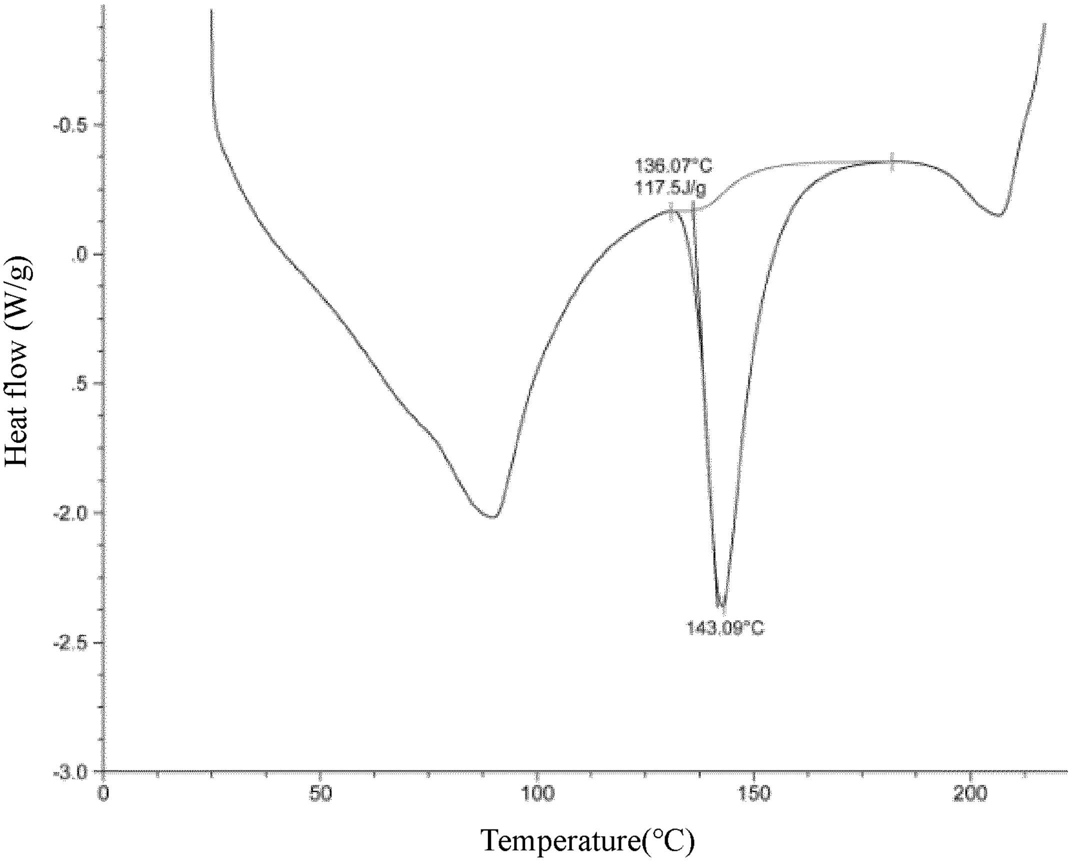
136.07°C
117.5J/g
143.09°C
Heat flow (W/g)
Temperature(°C)

FIG. 25

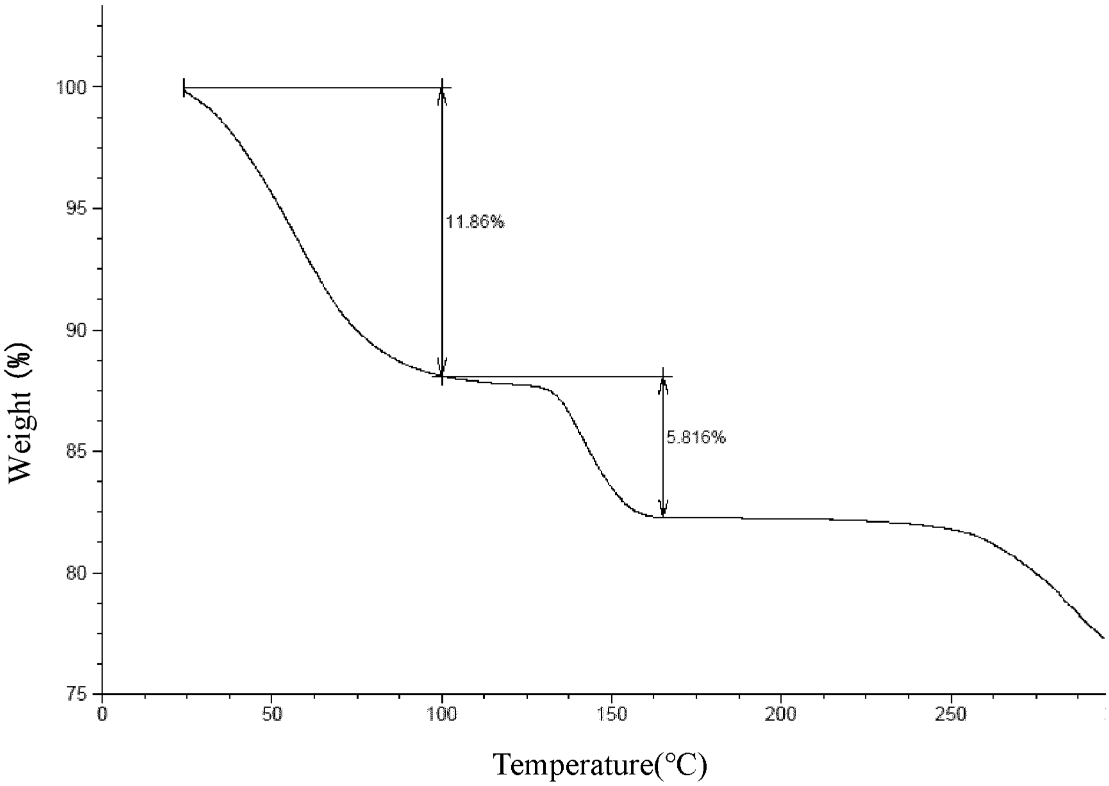

100
95
90
85
80
75
Weight (%)
0
50
100
150
200
250
Temperature(°C)
11.86%
5.816%


FIG. 26

**INTERNATIONAL SEARCH REPORT**

International application No. **PCT/CN2021/108515**

**A. CLASSIFICATION OF SUBJECT MATTER**

C07D 403/14(2006.01)i; A61K 31/517(2006.01)i; A61P 5/00(2006.01)i; A61P 3/00(2006.01)i; A61P 13/00(2006.01)i; A61P 1/16(2006.01)i; A61P 9/00(2006.01)i; A61P 25/00(2006.01)i; A61P 25/04(2006.01)i; A61P 35/00(2006.01)i; A61P 29/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, CNTXT, VEN, WOTXT, EPTXT, USTXT, CNKI, 万方, caplus, marpat, registry: 轩竹, 己酮糖激酶, 果糖激酶, KHK, 抑制, 晶型, 结晶, ketohexokinase, inhibit+, cryst+

**C. DOCUMENTS CONSIDERED TO BE RELEVANT**

| Category* | Citation of document, with indication, where appropriate, of the relevant passages | Relevant to claim No. |
|---|---|---|
| PX | WO 2020156445 A1 (KBP BIOSCIENCES CO., LTD.) 06 August 2020 (2020-08-06) claims 1-11, description pages 18-19, 25-35 | 1-11 |
| A | CN 108473469 A (PFIZER INC.) 31 August 2018 (2018-08-31) entire document, in particular claims 1-24, description, paragraphs [0164]-[0176] | 1-11 |
| A | CN 102939283 A (JANSSEN PHARMACEUTICA NV.) 20 February 2013 (2013-02-20) entire document | 1-11 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 August 2021** | **27 September 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/108515**

| Patent document cited in search report | Publication date (day/month/year) | Patent family member(s) | Publication date (day/month/year) |
|---|---|---|---|
| WO 2020156445 A1 | 06 August 2020 | AU 2020214064 A1 | 05 August 2021 |
| | | CA 3127130 A1 | 06 August 2020 |
| CN 108473469 A | 31 August 2018 | PE 20181289 A1 | 07 August 2018 |
| | | BR 112018012047 A2 | 04 December 2018 |
| | | UY 37051 A | 31 July 2017 |
| | | AU 2016380920 B2 | 31 October 2019 |
| | | RU 2696269 C1 | 01 August 2019 |
| | | GE P20207147 B | 10 September 2020 |
| | | CU 20180046 A7 | 05 September 2018 |
| | | US 10174007 B2 | 08 January 2019 |
| | | WO 2017115205 A1 | 06 July 2017 |
| | | SV 2018005709 A | 10 September 2018 |
| | | TW 201726659 A | 01 August 2017 |
| | | IL 260330 D0 | 30 August 2018 |
| | | PH 12018501375 A1 | 11 February 2019 |
| | | EC SP18048517 A | 31 July 2018 |
| | | CL 2018001667 A1 | 03 August 2018 |
| | | ZA 201803449 B | 27 February 2019 |
| | | HK 1259073 A1 | 22 November 2019 |
| | | JP 6503515 B2 | 17 April 2019 |
| | | US 10988463 B2 | 27 April 2021 |
| | | EP 3397631 A1 | 07 November 2018 |
| | | KR 20180083427 A | 20 July 2018 |
| | | KR 102073048 B1 | 04 February 2020 |
| | | CA 2952466 A1 | 29 June 2017 |
| | | US 2018037575 A1 | 08 February 2018 |
| | | CO 2018006714 A2 | 10 July 2018 |
| | | SG CN 11201804363 U A | 30 July 2018 |
| | | US 2020377482 A1 | 03 December 2020 |
| | | US 9809579 B2 | 07 November 2017 |
| | | TN 2018000198 A1 | 04 October 2019 |
| | | UA 121271 C2 | 27 April 2020 |
| | | US 10787438 B2 | 29 September 2020 |
| | | JP 2019500383 A | 10 January 2019 |
| | | TW I653232 B | 11 March 2019 |
| | | AU 2016380920 A1 | 14 June 2018 |
| | | EP 3397631 B1 | 07 April 2021 |
| | | US 2019106412 A1 | 11 April 2019 |
| | | NI 201800072 A | 22 November 2018 |
| | | US 2020148669 A1 | 14 May 2020 |
| | | CN 108473469 B | 03 November 2020 |
| | | MX 2018007755 A | 09 November 2018 |
| | | US 2017183328 A1 | 29 June 2017 |
| CN 102939283 A | 20 February 2013 | US 9771375 B2 | 26 September 2017 |
| | | AU 2011242711 A1 | 08 November 2012 |
| | | US 2011263559 A1 | 27 October 2011 |
| | | AU 2011242711 C1 | 21 April 2016 |
| | | CN 102939283 B | 03 June 2015 |
| | | EP 2560962 B1 | 20 May 2015 |

Form PCT/ISA/210 (patent family annex) (January 2015)

**INTERNATIONAL SEARCH REPORT**
**Information on patent family members**

International application No.
**PCT/CN2021/108515**

| Patent document cited in search report | Publication date (day/month/year) | Patent family member(s) | | | Publication date (day/month/year) |
|---|---|---|---|---|---|
| | | JP | 2013525370 | A | 20 June 2013 |
| | | ES | 2545811 | T3 | 16 September 2015 |
| | | US | 8822447 | B2 | 02 September 2014 |
| | | EP | 2560962 | A1 | 27 February 2013 |
| | | AU | 2011242711 | B2 | 12 November 2015 |
| | | WO | 2011133750 | A1 | 27 October 2011 |
| | | US | 2014336170 | A1 | 13 November 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 15381295 B


### Non-patent literature cited in the description


- **FRIEDMAN SL et al.** *Nat Med,* 2018, vol. 24, 908-22
- **BRAY GA.** Soft drink consumption and obesity: it is all about fructose. *Current opinion in lipidology,* 2010, vol. 21 (1), 51-7
- *The American journal of clinical nutrition,* 2002, vol. 76 (5), 911-22
- *The American journal of clinical nutrition,* 2007, vol. 86 (4), 899-906
- **SHI HONGBIN et al.** Study on association between fructose and nonalcoholic fatty liver disease. *Medical Recapitulate,* 2017, vol. 23 (9), 1685-1689
- **GEORGE MAREK ; 1 VARINDERPAL PANNU ; 1 PRASHANTH SHANMUGHAM ; 1 BRIANNA PANCIONE ; 1 DOMINIC MASCIA ; 1 SEAN CROSSON ; 1 TAKUJI ISHIMOTO ; YURI Y. SAUTIN1.** Adiponectin Resistance and Proinflammatory Changes in the Visceral Adipose Tissue Induced by Fructose Consumption via Ketohexokinase-Dependent Pathway. *Diabetes,* 2015, vol. 64, 508-518
- **THOMAS JENSEN et al.** Fructose and Sugar: A Major Mediator of Nonalcoholic Fatty Liver Disease. *J Hepatol.,* May 2018, vol. 68 (5), 1063-1075
- **MINGULE A et al.** Endogenous fructose production and metabolism in the liver contributes to the development of metabolic syndrome. *Nat Commun.,* 2013, vol. 4, 2434
- **LANASPA, M.A et al.** *Nature Comm.,* 2013, vol. 4, 2434
- **ALI M et al.** *J. Med. Genet.,* May 1998, vol. 35 (5), 353-365
- *HFI-INFO Discussion Board,* 14 December 2015, http: //hfiinfo.proboards.com
- General Principle. *Chinese Pharmacopoeia,* 2015, vol. IV
- **KARL FISCHER.** Moisture Determination Method in General Principle. *Chinese Pharmacopoeia,* 2015, vol. IV
- Diffraction Method in General Principle. *Chinese Pharmacopoeia,* 2015, vol. IV